(19) Europäisches Patentamt
European Patent Office
Office européen des brevets

(11) EP 4 000 691 A1

(12) EUROPEAN PATENT APPLICATION

(43) Date of publication:
**25.05.2022 Bulletin 2022/21**

(21) Application number: **20207769.9**

(22) Date of filing: **16.11.2020**

(51) International Patent Classification (IPC):
***A61P 35/00*** (2006.01) ***C07C 21/17*** (2006.01)
***C07D 263/32*** (2006.01) ***C07D 498/22*** (2006.01)
***A61K 31/424*** (2006.01)

(52) Cooperative Patent Classification (CPC):
**C07D 498/22; A61P 35/00; C07C 43/315; C07D 263/32**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Otto-von-Guericke-Universität Magdeburg**
**39106 Magdeburg (DE)**

(72) Inventors:
- **Schinzer, Dieter**
**38108 Braunschweig (DE)**
- **Spieß, Oliver**
**21029 Hamburg (DE)**
- **Lizzadro, Luca**
**39108 Magdeburg (DE)**

# (54) DISORAZOLES AND THEIR ANALOGUES AND METHODS FOR THEIR PRODUCTION

(57) The present invention describes disorazoles and their analogues as described in formula III

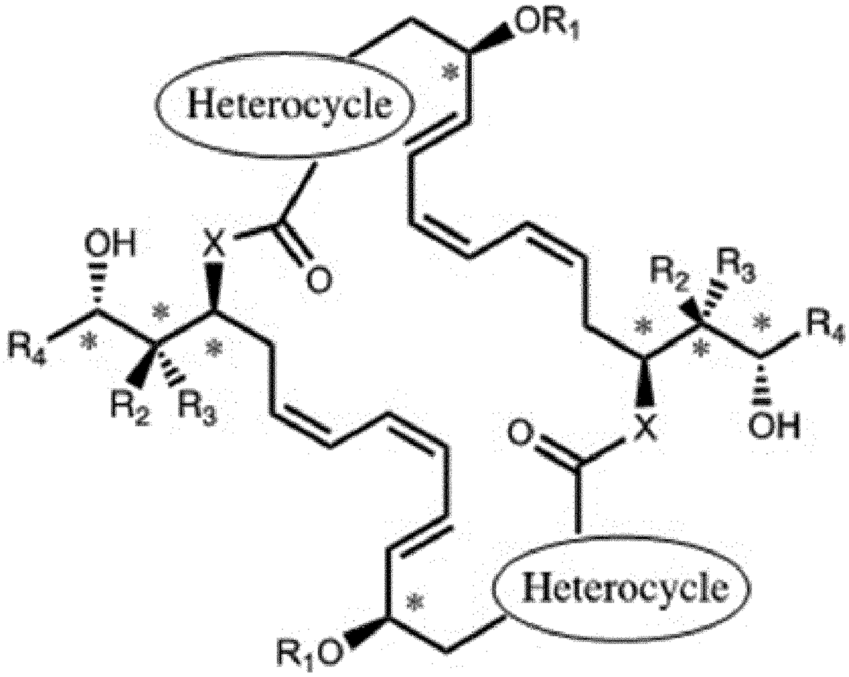

III

wherein the variables are defined in the description, or pharmaceutically acceptable salts thereof.

Described is also a novel synthetic pathway for the production of disorazoles and their analogues, using two precursors twice in coupling reactions and a final cyclisation for a compound of formula III.

The compounds of the present invention are useful as anti-cancer drugs.

EP 4 000 691 A1

## Description

### Technical Field

The present invention relates to disorazoles and their analogues and methods for their production. These compounds can be used as medicaments, in particular for the treatment of various tumors.

### Background Art

Disorazoles are potent anti-cancer drugs and are natural substances. It is known that natural substances of the group consisting of the disorazoles are isolated from the bacterium of the strain *Sorangium cellulosum.*

Synthetic methods for the preparation of disorazoles are described, for example, in WO 2018/237178 A1 or Wipf P. et al., J. Am. Chem. Soc. 2004, 126, 47, 15346-15347.

Therefore, it is an object of the present invention to provide a production method resulting in a high yield of disorazoles and their analogues, especially disorazole C1.

### Summary of Invention

In some aspects, the present invention discloses new disorazoles and their analogues and new synthetic methods for the up-scaled production of disorazoles and their analogues.

One aspect is, that the disorazoles and their analogues may be produced by using two main substructures, also named as precursors, that can be produced in large scales. These two main substructures are used to produce an open-chained precursor for the final cyclisation of disorazoles and their analogues.

One preferred embodiment of the present invention is a compound of the formula III

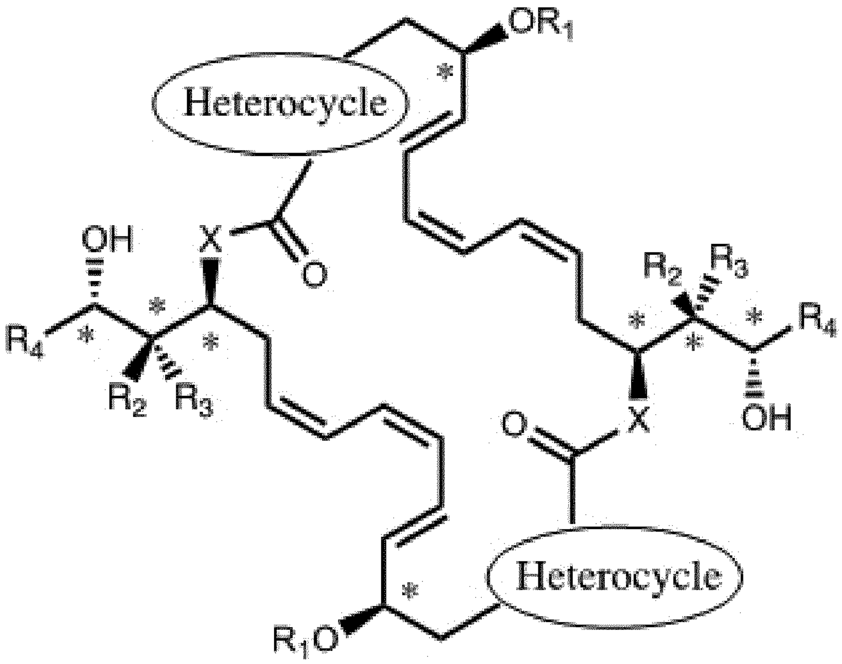

III

wherein:

- X is O, S, or NR;
- R is H, or alkyl$_{(C\leq8)}$;
- $R_1$ is H, alkyl$_{(C\leq8)}$, or cycloalkyl$_{(C\leq8)}$;
- $R_2$ and $R_3$ are each independently H, alkyl$_{(C\leq8)}$, $(CH_2)_n$ (n = 1-5);
- R4 is independently H, alkyl$_{(C\leq8)}$, cycloalkyl$_{(C\leq8)}$, alkenyl$_{(C\leq8)}$, alkinyl$_{(C\leq8)}$, allyl, propargyl, phenyl, or benzyl;
- Heterocycle is independently oxazole, thiazol, imidazole, furyl, pyrrolyl, thiophenyl, pyridyl, or phenyl;
- * indicate the stereoisomeric centers of the compound;

as well as the individual stereoisomers of this compound and/or a pharmaceutically acceptable salt thereof.

Another aspect of the present invention is a method for the production of a compound of formula III

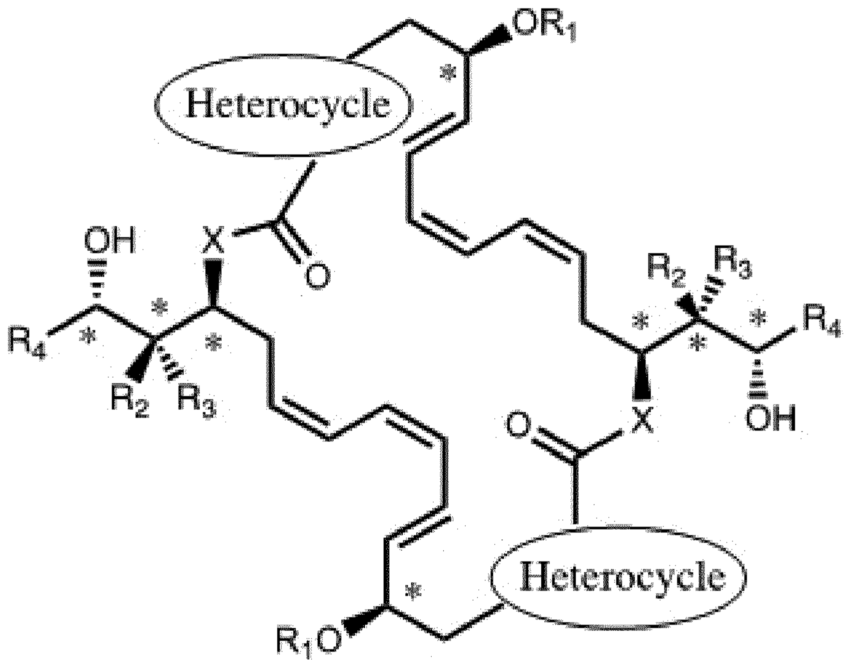

(III)

wherein:

X is O, S, or NR;
R is H, or $alkyl_{(C\leq 8)}$;
$R_1$ is H, $alkyl_{(C\leq 8)}$, or $cycloalkyl_{(C\leq 8)}$;
$R_2$ and $R_3$ are each independently H, $alkyl_{(C\leq 8)}$, $(CH_2)_n$ (n = 1-5);
$R_4$ is independently H, $alkyl_{(C\leq 8)}$, $cycloalkyl_{(C\leq 8)}$, $alkenyl_{(C\leq 8)}$, $alkinyl_{(C\leq 8)}$, allyl, propargyl, phenyl, or benzyl;
Heterocycle is independently oxazole, thiazol, imidazole, furyl, pyrrolyl, thiophenyl, pyridyl, or phenyl;
* indicate the stereoisomeric centers of the compound;

comprising the following steps:

(a) reacting a compound of formula I

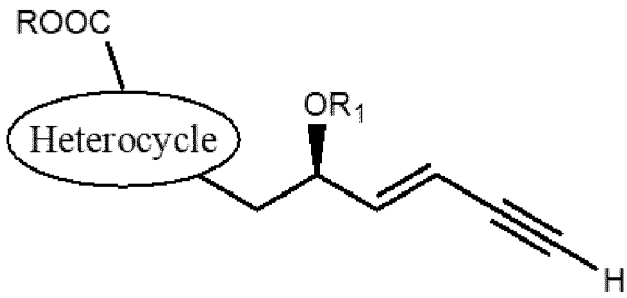

(I)

wherein R and $R_1$ is as defined above;
with a compound of formula II

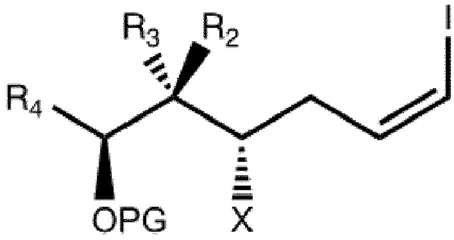

(II)

wherein X is O, S, or NR as defined above and wherein O, S, or NR comprise further a protective group PG; wherein $R_2$, $R_3$, and $R_4$ are defined as above; and wherein PG is independently H, or alkyl$_{(C\leq 8)}$; or a protective group selected independently from MOM, MEM, THP, TMS, TES, TIPS, TBS, TBDPS,

(b) reacting the product obtained in step (a) with a compound of formula I, wherein the coupling of the obtained product and compound of formula I is performed via the carboxyl ester of compound I and the X group of the obtained product;

(c) reacting the product obtained in step (b) with a compound of formula II in the same way as in step (a);

(d) cyclizing the product obtained from step (c) obtaining the product of formula III.

The main feature of this aspect is that two precursors as disclosed, as compounds of formula I and of formula II, are used to produce an open chained precursor of disorazols and their analogues. This open chained precursor is then cyclized and the final products as described as compounds of formula III, namely disorazoles and their analogues, are produced in a high yield and in a great purity.

Another aspect of the present invention is a method for the production of a compound of formula III according to claim 1

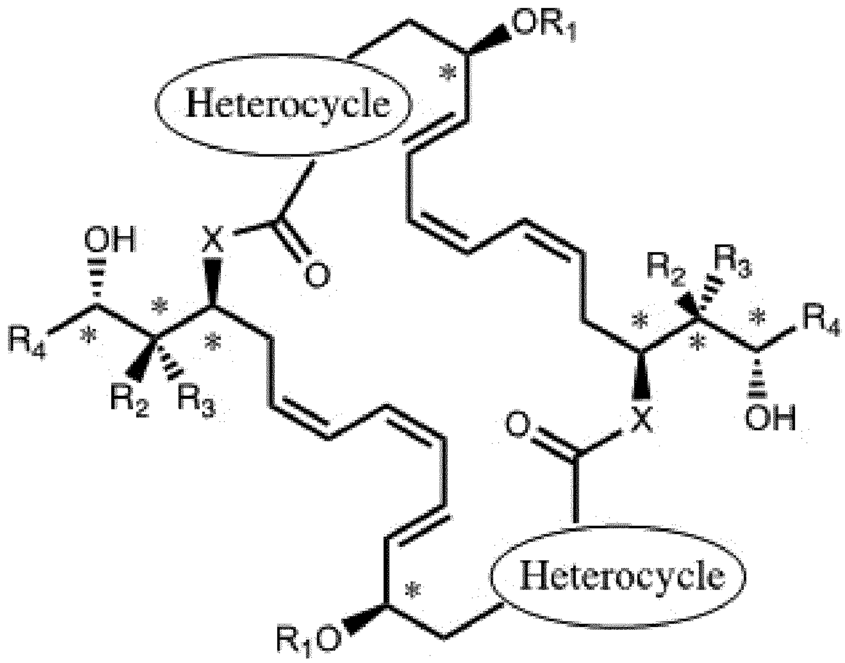

(III)

wherein:

X is O, S, or NR;
R is H, or alkyl$_{(C\leq 8)}$;
$R_1$ is H, alkyl$_{(C\leq 8)}$, or cycloalkyl$_{(C\leq 8)}$;
$R_2$ and $R_3$ are each independently H, alkyl$_{(C\leq 8)}$, $(CH_2)_n$ (n = 1-5);

$R_4$ is independently H, alkyl$_{(C\leq 8)}$, cycloalkyl$_{(C\leq 8)}$, alkenyl$_{(C\leq 8)}$, alkinyl$_{(C\leq 8)}$, allyl, propargyl, phenyl, or benzyl;
Heterocycle is independently oxazole, thiazol, imidazole, furyl, pyrrolyl, thiophenyl, pyridyl, or phenyl;
* indicate the stereoisomeric centers of the compound;

comprising the following steps:

(a) reacting a compound of formula I

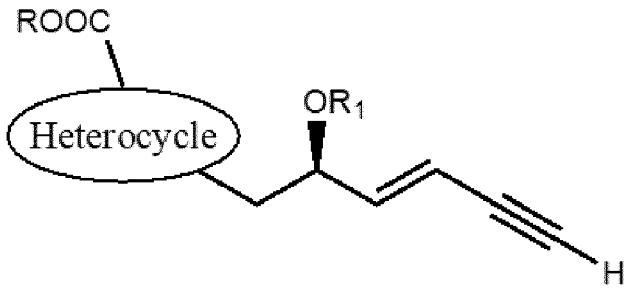

(I)

wherein R and $R_1$ is as defined above;
with a compound of formula II

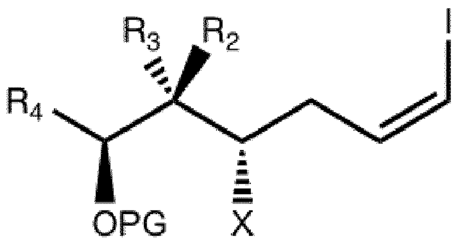

(II)

wherein X is O, S, or NR as defined above and wherein O, S, or NR comprise further a protective group PG; wherein $R_2$, $R_3$, and $R_4$ are defined as above; and wherein PG is independently H, or alkyl$_{(C\leq 8)}$; or a protective group selected independently from MOM, MEM, THP, TMS, TES, TIPS, TBS, TBDPS,

(b) reacting the product of step (a) in two different steps, namely step (b1) and step (b2),
wherein in step (b1) the compound obtained in step (a) is modified, in a way that PG from X is removed to obtain a compound wherein X is OH, SH, or $NH_2$,
and wherein in step (b2) the compound obtained in step (a) is modified, in a way that residue R of the carboxy group is removed and replaced with H;

(c) reacting the product obtained in step (b1) with the product obtained in step (b2);

(d) cyclizing the product obtained from step (c) obtaining the product of formula III.

An especially preferred embodiment of the present invention is a method for the production of disorazole-C1 of formula 37

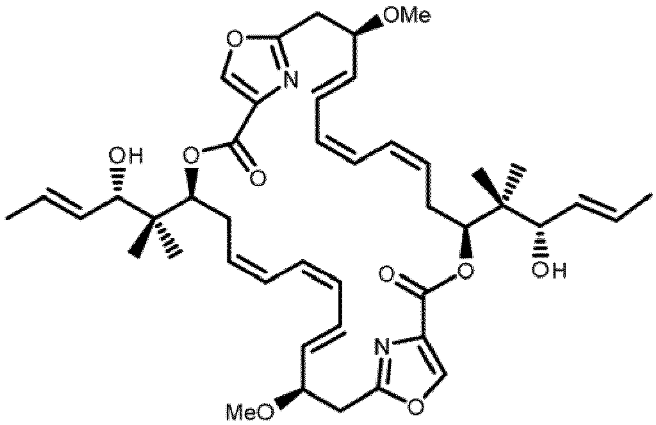

37

comprising the following steps:

(a) reacting a compound of formula 30a

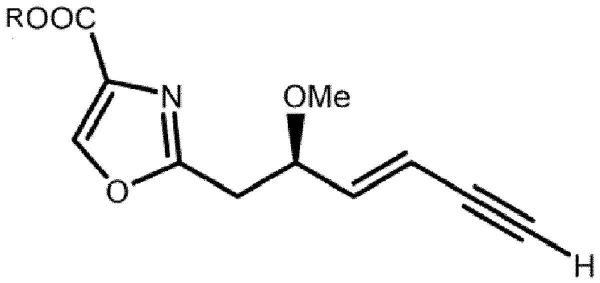

30a

wherein R is methyl or hydrogen
with a compound of formula 18

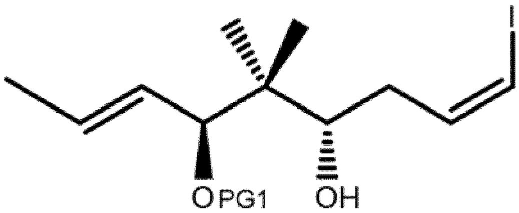

18

wherein PG1 is MOM
obtaining a compound of formula 31

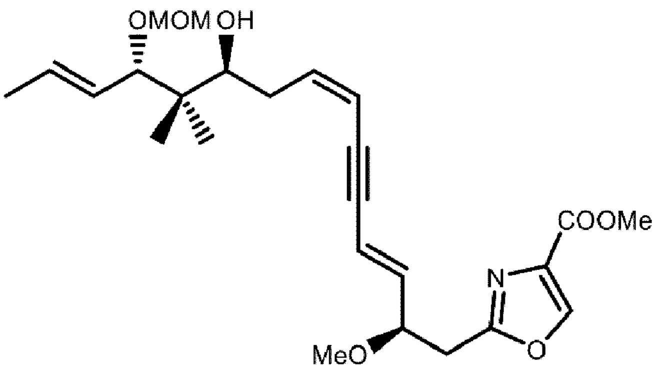

31;

(b) reacting the compound of formula 31 with a compound of formula 30a, obtaining a compound of formula 32

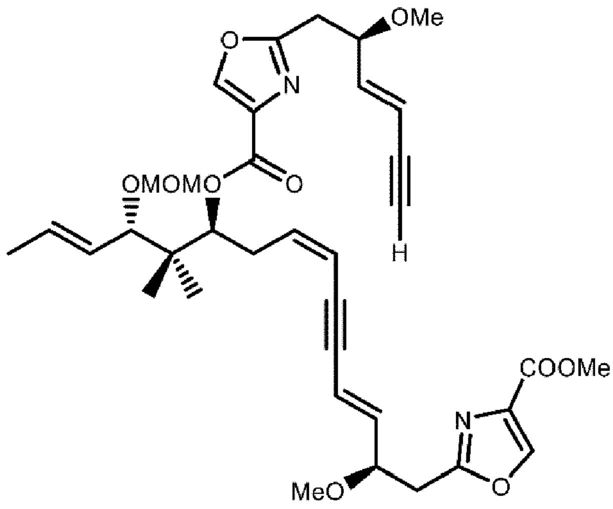

32

(c) reacting the compound of formula 32 with a compound of formula 18, obtaining a compound of formula 33

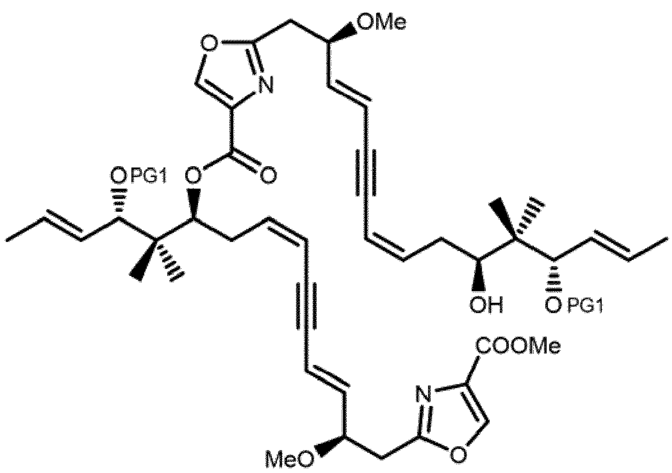

33

(d) reacting compound of formula 33 into disorazole-C1 of formula 37.

Especially preferred herein is the method, wherein step (a) is a coupling reaction combining compounds of formula 18 and of formula 30a, wherein R is methyl.

Especially preferred herein is also the method, wherein step (b) is an esterification.

Further especially preferred herein is the method, wherein step (c) is a coupling reaction combining compounds of formula 31 and of formula 30a, wherein R is hydrogen.

Preferred herein is also the method, wherein step (d) comprises the following steps:

(d1) saponification of methyl ester from compound of formula 33 to a compound of formula 34

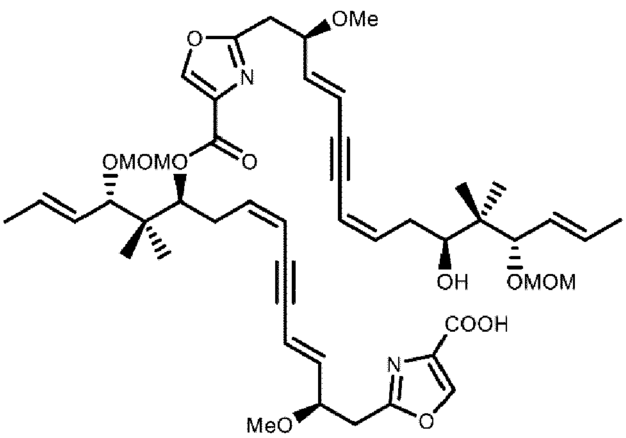

34;

(d2) macrolactonisation of open-chained compound 34 into compound of formula 35

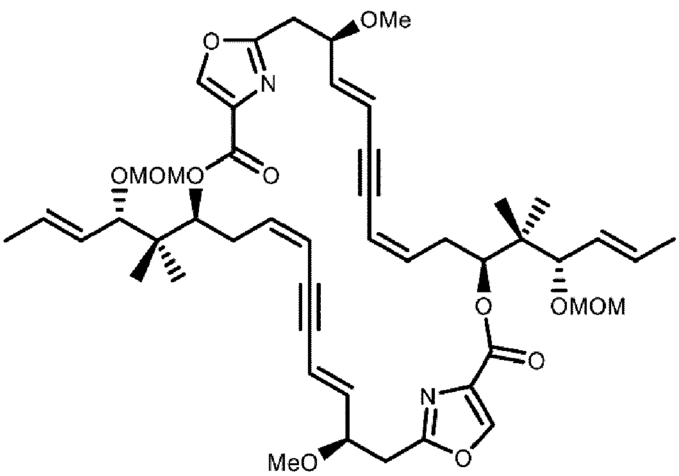

35;

(d3) reduction of the triple bonds of compound of formula 35 forming compound of formula 36

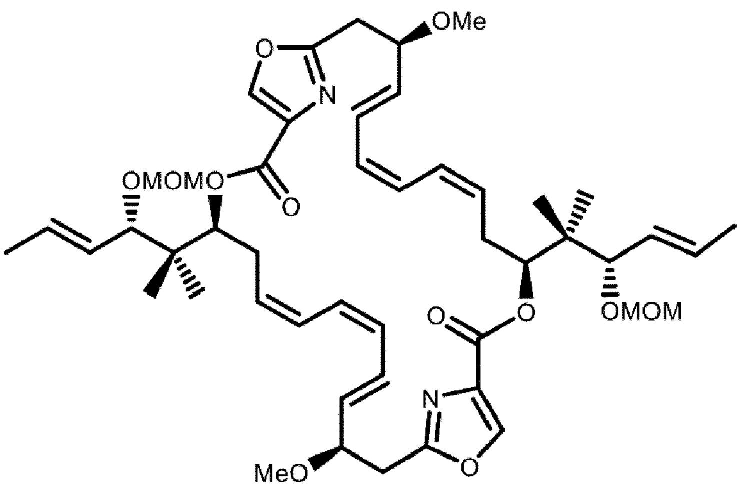

36;

and

(d4) removing the protection groups to obtain compound of formula 37.

Another especially preferred embodiment of the present invention is a method for the production of disorazole-C1 of formula 37

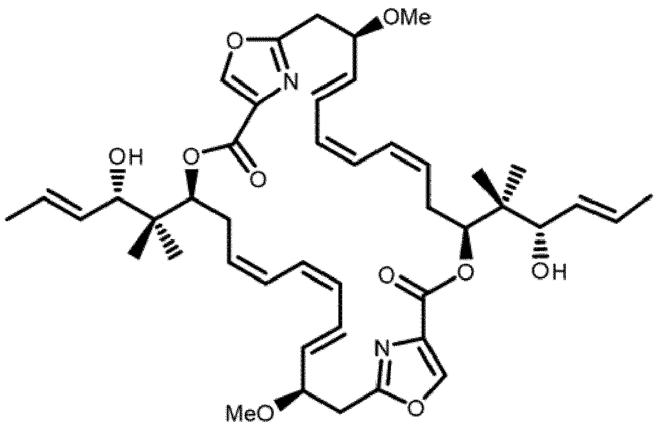

37

comprising the following steps:

(a) reacting a compound of formula 30a

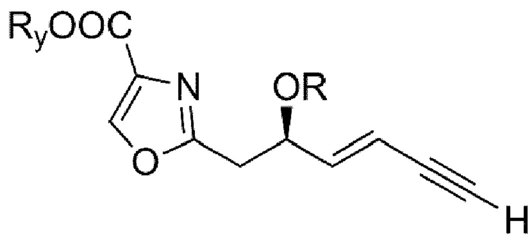

30a

wherein R and $R_y$ are methyl;
with a compound of formula 17a

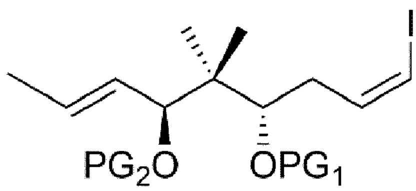

17a

wherein $PG_1$ is methyl and $PG_2$ is MOM;
obtaining a compound of formula 40

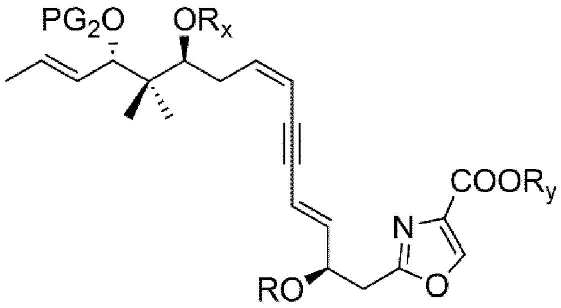

40

wherein $R_x$ is $PG_1$ and $R_y$ is methyl as defined above;

(b) reacting the compound of formula 40 partially in a step (b1) into a compound of formula 41

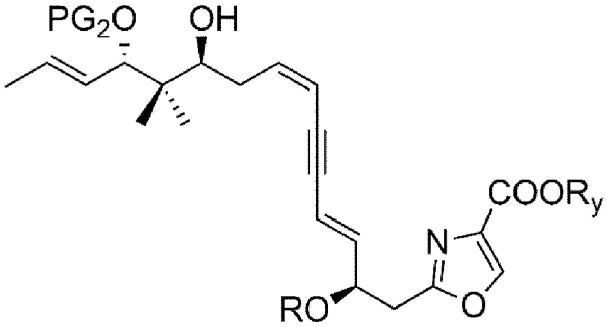

41

and partially in a step (b2) into a compound of formula 42

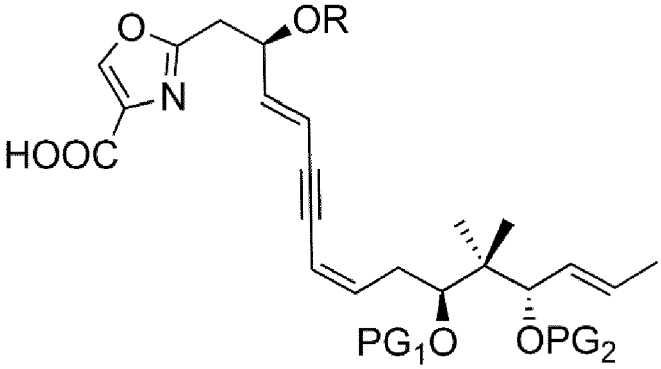

42

(c) reacting the compound of formula 41 with the compound of formula 42, obtaining a compound of formula 43

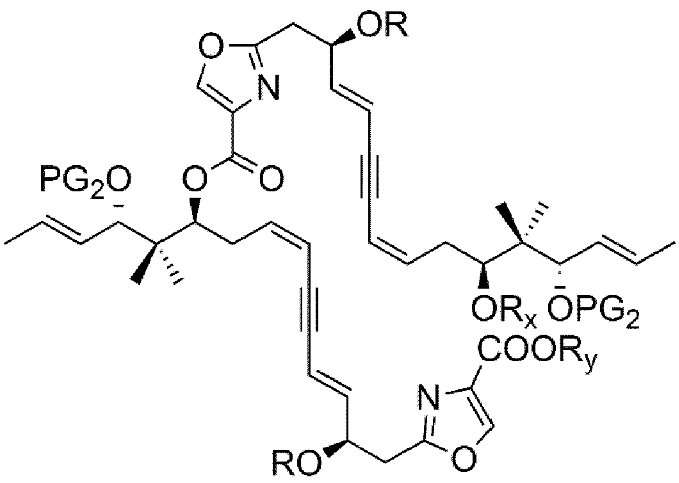

43

(d) removing $R_x$ and $R_y$ and replacing with hydrogen, obtaining a compound of formula 44

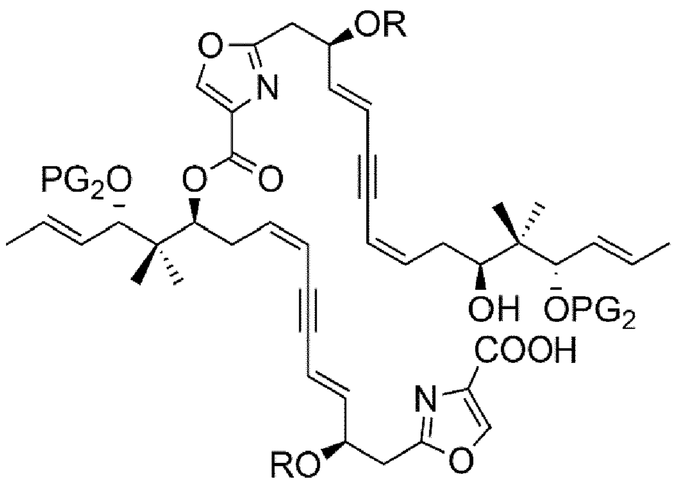

44

(e) reacting compound of formula 44 into disorazole-C1 of formula 37 as described above, wherein the starting compound is the compound of formula 44 instead of formula 34.

A great advantage of this embodiment is that the yield of the reaction and the purity of the products is much higher. The reason is, that the complete reaction is more effective, as the coupled compounds 41 and 42 from precursors 17a and 30a differ in the protective groups. The different compounds 41 and 42 can easily be produced from the same precursors 17a and 30a. The further details are described below in reaction scheme 6.

A further preferred embodiment of the present invention is an intermediate product, for the production according to the present invention, according to formula 30a

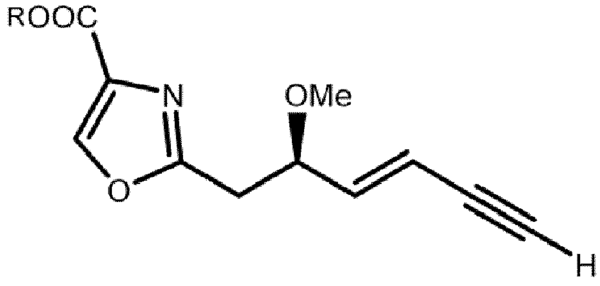

30a

wherein R is methyl or hydrogen.

Another preferred embodiment of the present invention is an intermediate product, for the production according to the present invention, according to formula 18

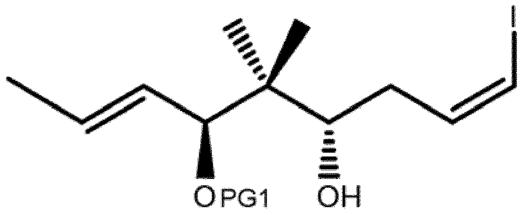

18

wherein PG1 is MOM.

Another preferred embodiment of the present invention is an intermediate product, for the production according to the present invention, according to formula 33

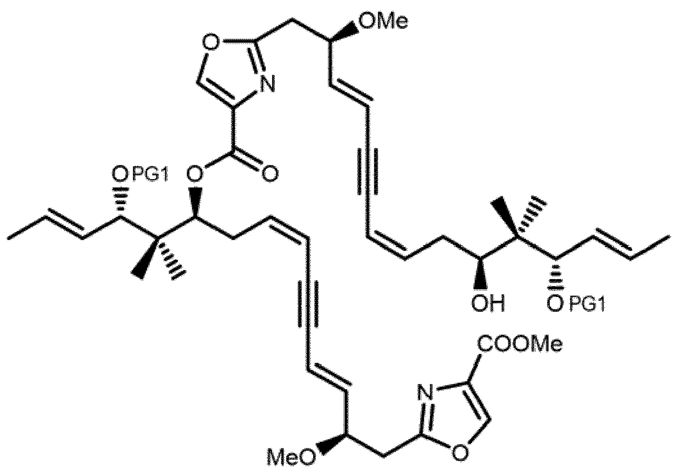

33

wherein PG1 is MOM.

Another preferred embodiment of the present invention is an intermediate product, for the production according to the present invention, according to formula I

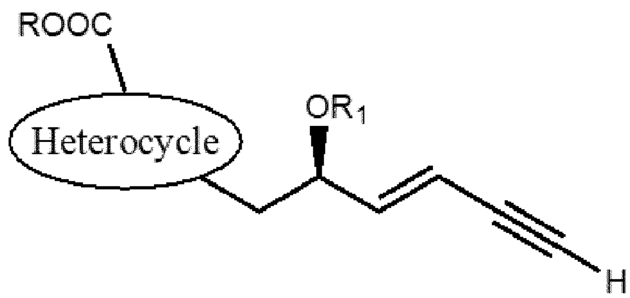

(I)

wherein R and $R_1$ is as defined above.

Another preferred embodiment of the present invention is an intermediate product, for the production according to the present invention, according to formula II

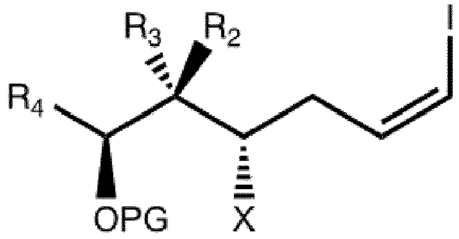

(II)

wherein X is O, S, or NR as defined above and wherein O, S, or NR comprise further a protective group PG; wherein $R_2$, $R_3$, and $R_4$ are defined as above; and wherein PG is independently H, or $alkyl_{(C\leq8)}$; or a protective group selected independently from MOM, MEM, THP, TMS, TES, TIPS, TBS, TBDPS.

A great advantage of the present invention is that only two precursors are needed to synthesize disorazoles and their analogues, namely compounds of formula I, formula II, formula 18, formula 30a, formula 29, or formula 30.

Another great advantage of the present invention is to combine also compound of formula 17a with compound of formula 30a with different protective groups or substituents as described below in scheme 6.

Another great advantage is that the precursors used can be selected according to their stereospecific properties. The compounds of formula I comprises one asymmetric carbon atom.

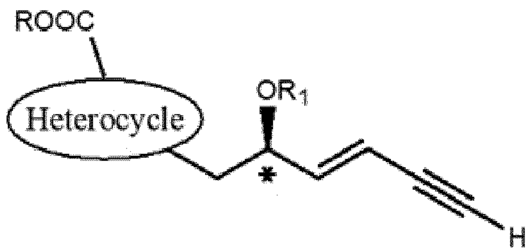

I

The compounds of formula II comprise up to three asymmetric carbon atoms.

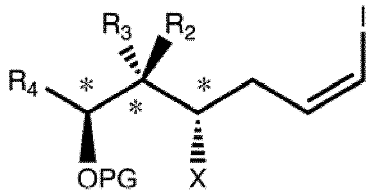

II

For the person skilled in the art it is there therefore clear that the asymmetric centres in the final disorazoles can be produced easily by selecting the respective precursors as indicated above. The final product of formula III contains up to eight asymmetric centres and all possible combinations of the diastereomers can be produced easily. This option is one of the greatest advantages of the present invention.

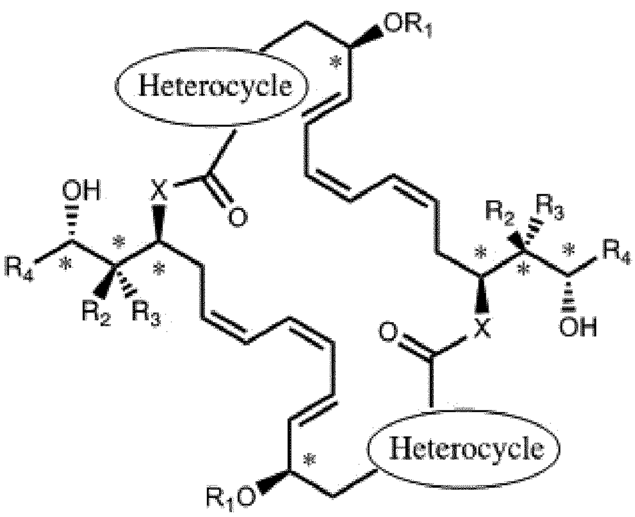

III

One preferred embodiment of the present invention is the production of disorazole C1. A high yield in the production of the final product is also a great advantage.

A further object of the present invention is a pharmaceutical preparation for the treatment of cancer diseases, comprising at least one compound according to the invention and other acceptable excipients, adjuvants and /or additives.

It is known in the state of the art how to produce pharmaceutical preparations, especially when using disorazols as known in the art.

**Description of Embodiments**

The process for the production of disorazole and their analogues according to the invention will now be described in great detail. The following reaction schemes describe the synthesis of disorazoles and their analogues in general. One preferred embodiment of the synthesis in described in the examples as given below in greater details. These examples are one preferred synthetic pathway for the production of disorazole C1.

One compound for the production of disorazole is (S)-HYTRA ((S)-(-)-2-Hydroxy-1,2,2-triphenylethylacetate), which is a protective group. The synthetic pathway is described in Scheme 1.

## Scheme 1:

### Synthesis of (S)-HYTRA

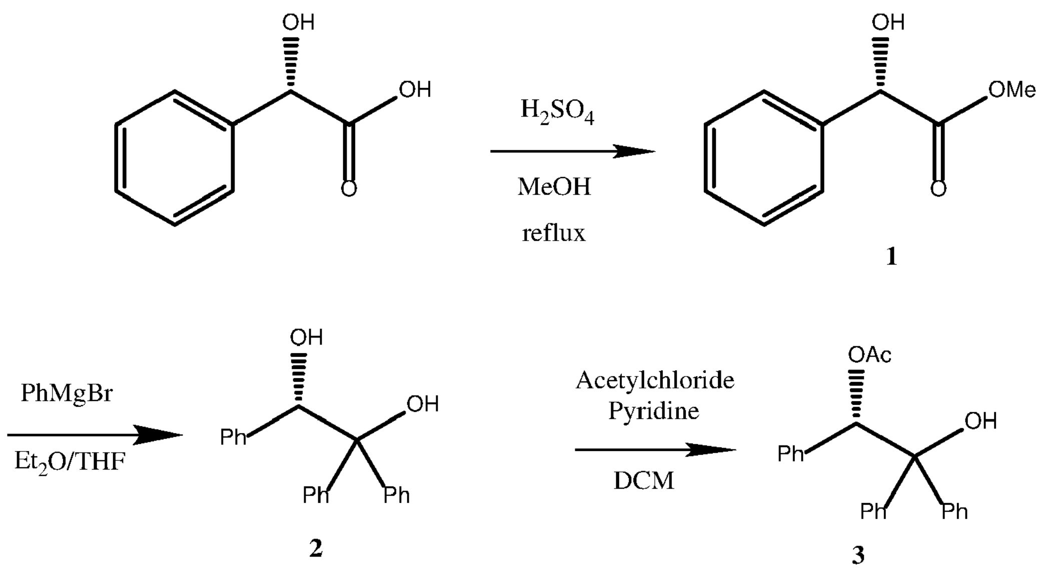


1

2

3

In order to produce the important precursor of formula 18, which is described as lateral chain, a first fragment

is synthesized as described in Scheme 2.

## Scheme 2:

## Synthesis of the first fragment for the lateral chain

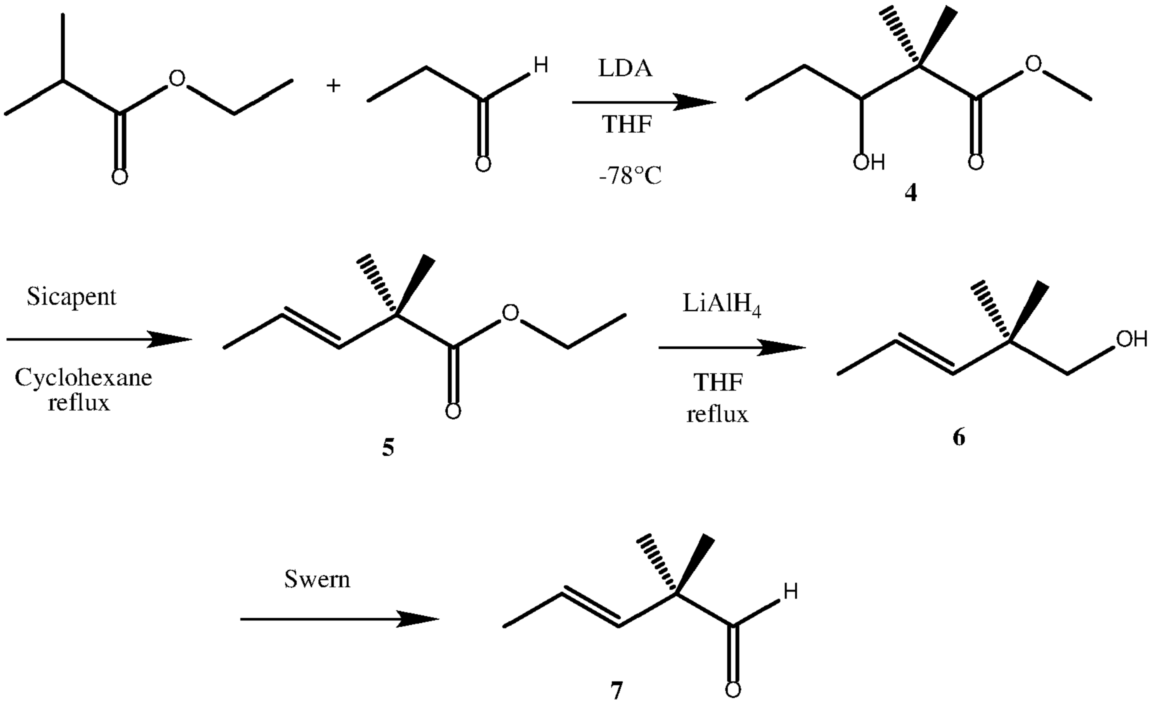


4

5

6

7

Using (S)-HYTRA of formula 3 and the aldehyde of formula 7 (as shown in Scheme 2) the synthesis of precursor of formula 18 is described in Scheme 3. Aldehyde 7 is reacted with compound of formula 3, then reacted with lithium aluminium hydride. An addition of protection groups, preferably with TES, is performed in the next step. The next step is an by ozonolysis, followed by a chiral allylation (preferably with Leighton reagent). In the next step the addition of a protection group for the new hydroxy group is performed. A double bond isomerisation is performed (Grubbs reaction), followed by a Swern oxidation. Furthermore, a Wittig reaction is performed and a Z-vinyl iodide is obtained which is finally selectively deprotected. Now, precursor of formula 18 is prepared, as shown in Scheme 3.

## Scheme 3:

### Synthesis of the lateral chain

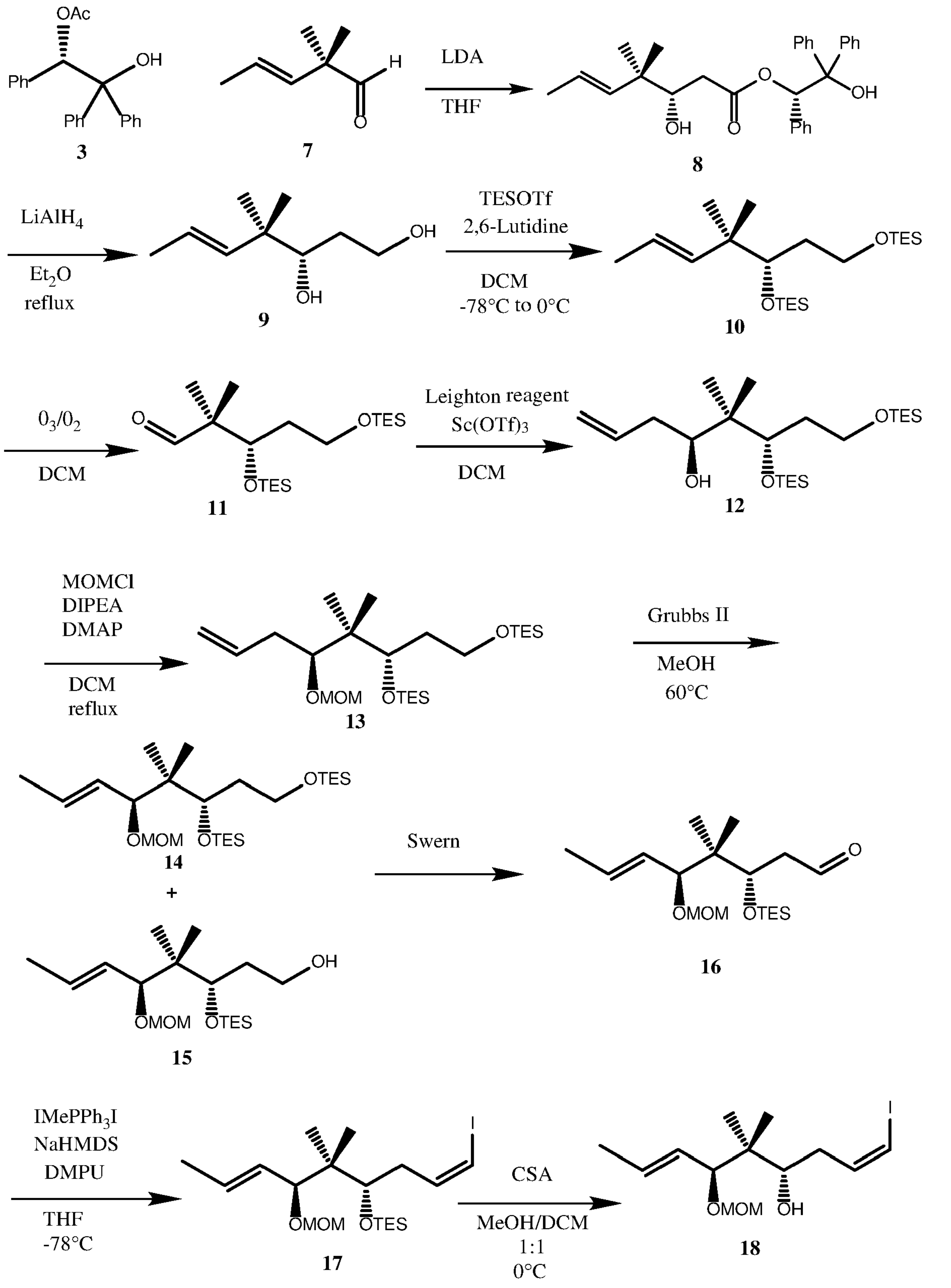


3 7 8 9 10 11 12 13 14 + 15 16 17 18

In the following Scheme 4 the synthesis of the second precursor for the synthetic method for the disorazole

according to the present invention is described. The second precursor, named as oxazole fragment, is synthesized starting with β-hydroxy ester of formula 19. The first step is an O-methylation reaction, followed by ozonolysis. The next step is performed as a Wittig reaction, followed by a hydrolysation of the ester obtaining product of formula 25. In the next step, L-serin methyl ester is reacted with compound of formula 25 to form the respective amide. In the following step, a cyclisation of the amide according to formula 26 is performed and this results to an oxazoline derivative. In the next step, the oxazoline is oxidized in order to obtain the oxazole derivative according to formula 28. The protective group in the compound of formula 28 is then removed and compound of formula 29 is obtained. This compound will be used for the final reaction as one of the starting materials indicated as compound of Formula 30a. The compound of formula 30a is a combination of compounds of formula 29 and 30, which differ from each other in the ester function of the carboxyl group. Finally, compound of formula 29 is saponificated to remove the methyl group from the ester group in the compound of formula 29. Now is final product of formula 30 is obtained.

According to the invention, compounds of formula 29 and formula 30 will be used in combination with the compound of formula 18 for the final reaction steps to obtain the final product disorazole of formula 37.

## Scheme 4:

## Synthesis of the oxazole fragment

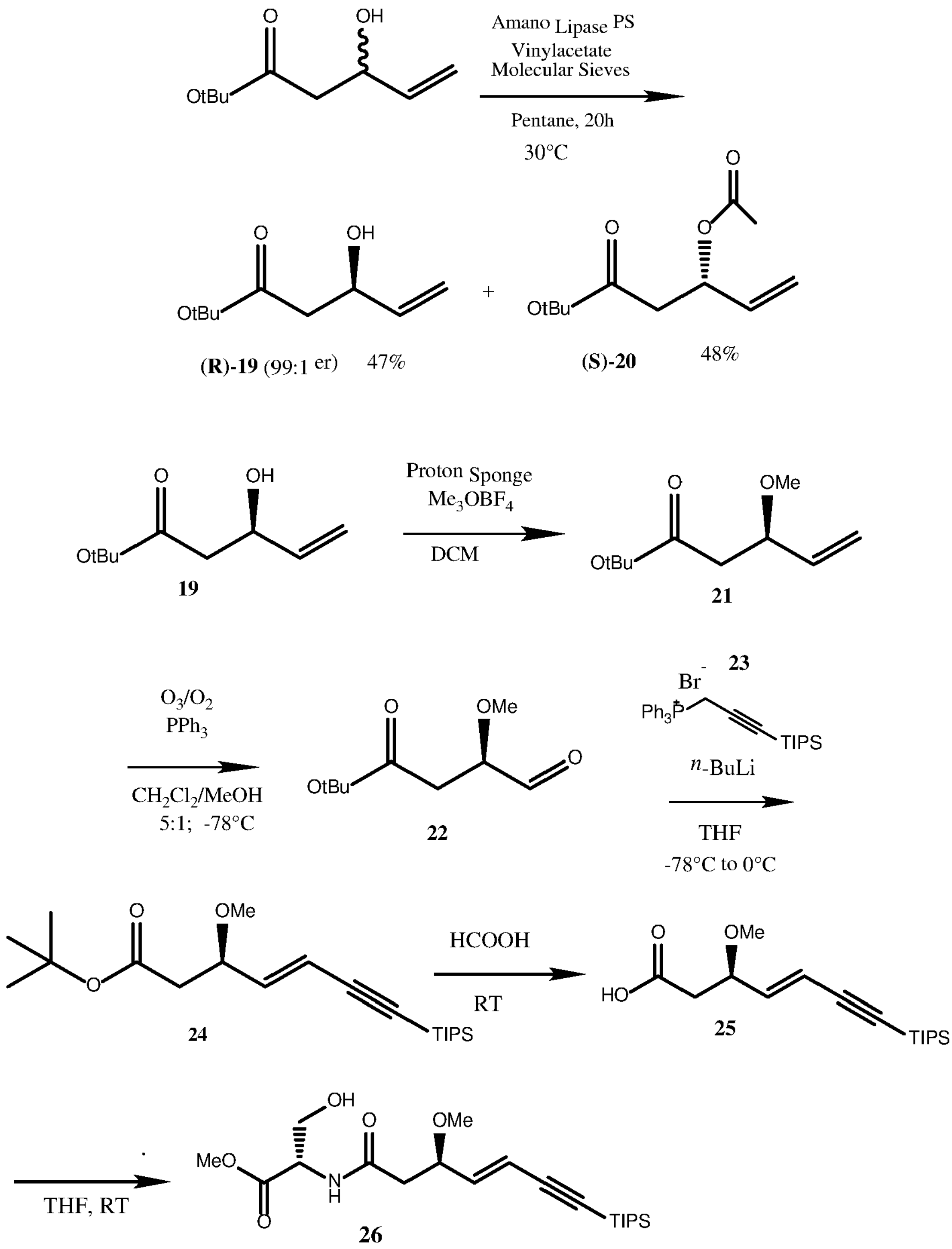


(R)-19 (99:1 er) 47%

(S)-20 48%

19

21

22

23

24

25

26

**Scheme 4 (continued):**

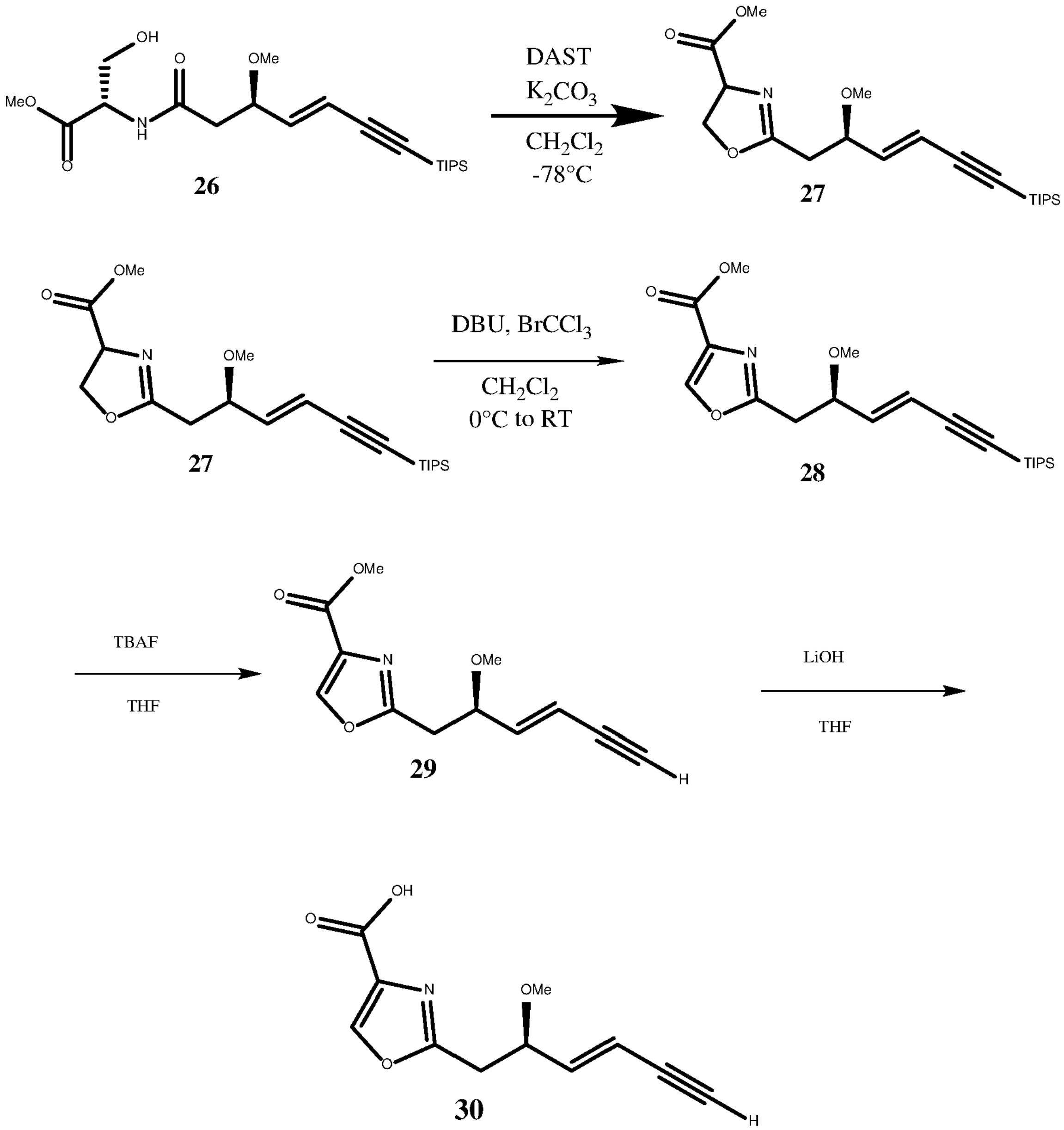


26 27 27 28 29 30

Now, the two precursors, namely the compounds of formula 30 and formula 29 are obtained and will be used in the final strategy for the production of disorazole named as compound of formula 37.

Now, in Scheme 5 the final strategy is described in detail.

## Scheme 5:

### Final strategy

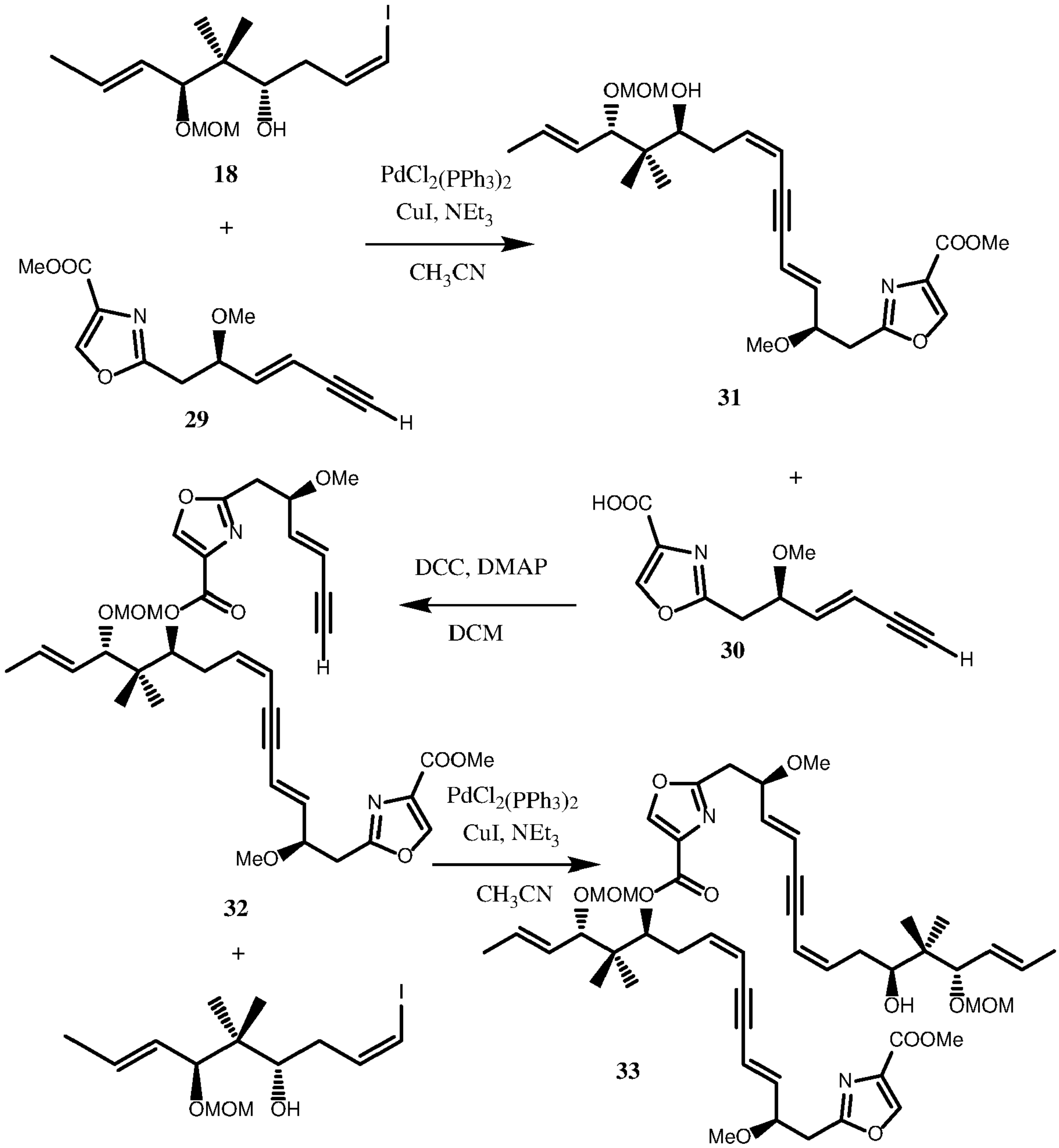


18

+

29

31

+

30

32

+

33

## Scheme 5 (continued):

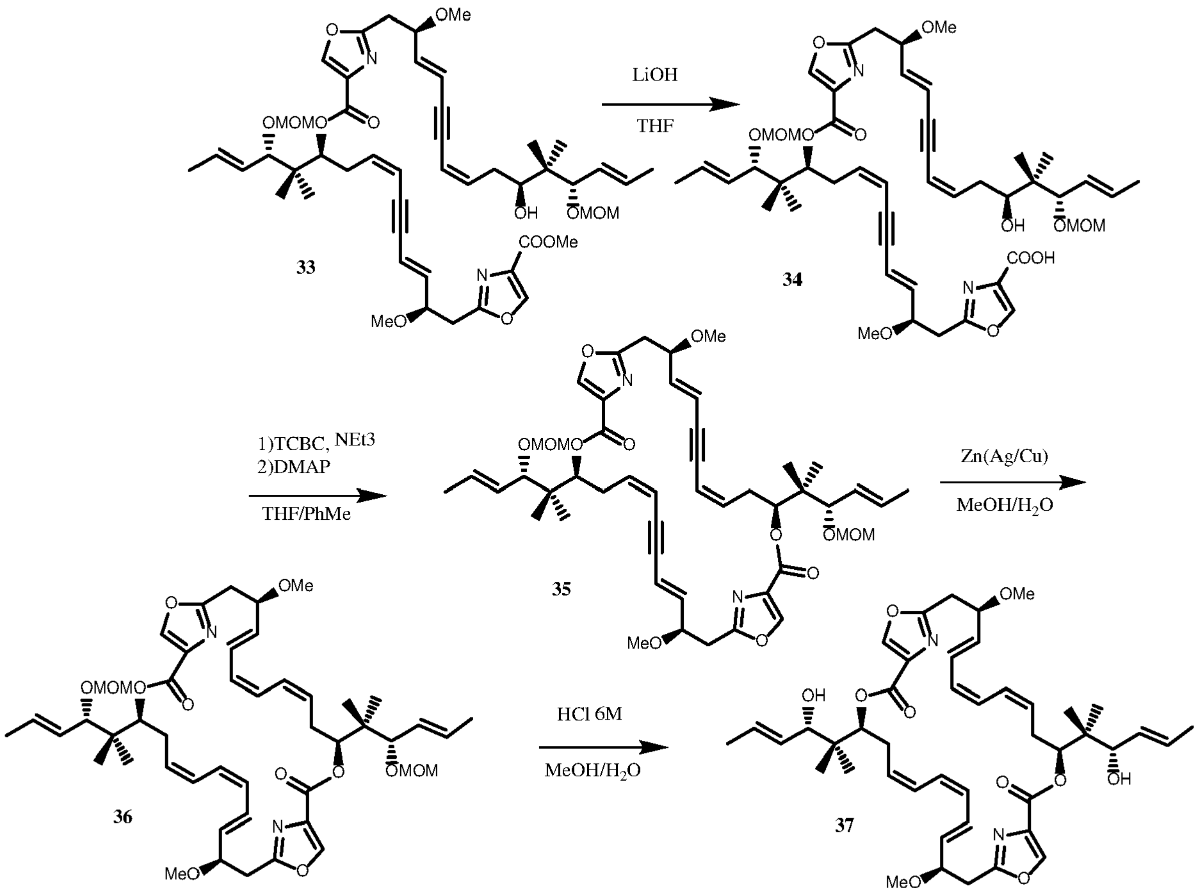


33 34 35 36 37

The total synthesis of disorazole of the formula 37 is performed in the following steps.

The compounds of formula 18 and formula 29 are coupled. Especially preferred is the use of a Sonogashira reaction. Compound of formula 31 is produced and an esterification with compound of formula 30 is performed in the next step, wherein compound of formula 32 is obtained. In the next step, compound of formula 32 and compound of formula 18 are coupled, especially preferred also with a Sonogashira reaction. The reaction produces the open-chained compound of formula 33.

The next steps of the reactions according to Scheme 5 perform the cyclisation of compound of formula 33, forming the final product, disorazole of formula 37.

In the first step, a saponification of the methyl ester in compound of formula 33 is performed and compound of formula 34 is obtained. With compound of formula 34 a macrolactonisation is performed, wherein the Yamaguchi reaction is preferred. With the obtained compound of formula 35 a hydrogenation of the triple bonds is performed and compound of formula 36 is obtained. In the final step, the protective groups are removed and the final product according to the invention, disorazole C1 of formula 37 is obtained.

Now in Scheme 6 a further advantageous strategy for the production of disorazoles according to the present invention is described in detail. The precursor 17a, comprising protective groups $PG_1$ and $PG_2$ is described already in the complete description. This precursor is also named as general formula II. The same applies to precursor 30a, which is also described herein as compound of formula I. In reaction Scheme 6 the carboxyl group is methylated. All reactive groups are protected so that the step a), as indicated in Scheme 6, is performed as a Sonogashira reaction, as described in Scheme 5. In contrast to Scheme 5, the product 40 is now reacted in two different steps in order to obtain the products 41 and 42. The products 41 and 42 differ from each other only in one reactive group. In step b), as indicated in Scheme 6, which is a simple demethylation step, Rx, the methyl group is removed and changed into hydrogen, so that a free hydroxyl group is present in the compound of formula 41. In step c), as indicated in Scheme 6, compound of formula 40 is demethylated in a simple saponification step and results in a free carboxylic group as indicated in the compound of formula 42. Now in step d), as indicated in Scheme 6, a Yamaguchi reaction is performed as a lactonization of compounds of formula 41 and 42 into compound of formula 43. In the final steps e) and f), as indicated in Scheme 6, the protective groups are removed as already shown in steps b) and c), as indicated in Scheme 6. The final cyclization reaction to obtain the disorazole according to the present invention is performed, for example, in the same way as shown in Scheme

5 , as indicated above.

## Scheme 6:

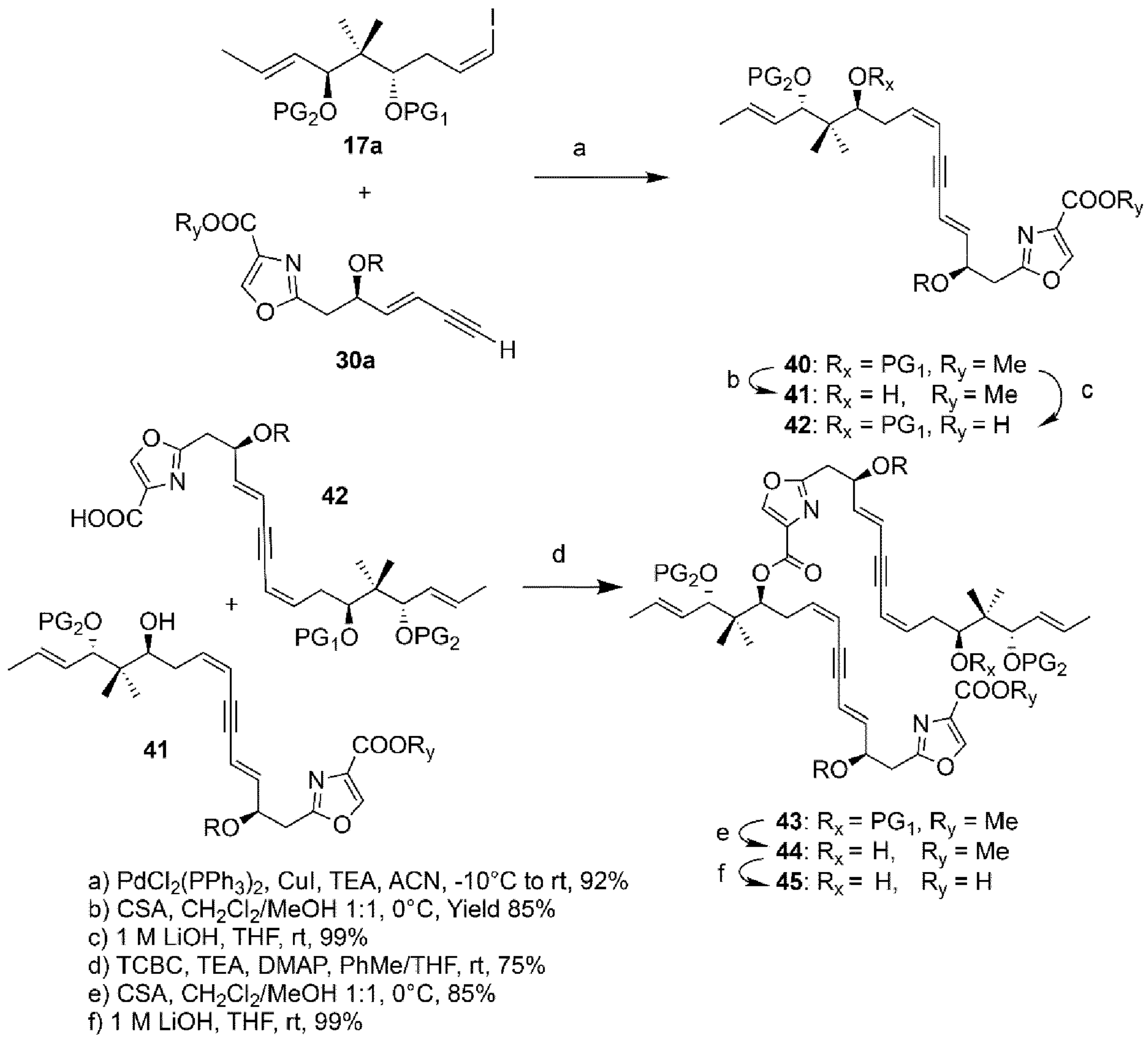


For a person skilled in the art it is clear that all possible diastereomers of disorazoles according to general formula III of formula 37 can be obtained, when starting the reactions with the respective diastereomeric precursors of formula I and formula II, as described above.

In the presented Schemes 1 to 6 different protective groups are indicated. It is clear for a person skilled in the art that other protective groups may be used. Especially preferred are the following protective groups: MOM (methoxymethyl), MEM ((2-methoxyethoxy)phenyl), THP (tetrahydropyranyl), TMS (trimethylsilyl), TES (triethylsilyl), TIPS(triisopropylsilyl), TBS (tert-butyldimethylsilyl), TBDPS (tert-butyldiphenylsilyl), OAc (O-Acyl).

Furthermore, in the Schemes 1 to 6 name reactions are mentioned. It is also clear for persons skilled in the art that other reactions or name reactions may be used according to the invention.

Details of the performed reactions are disclosed in the examples as presented herein. Complete chemical names of the compounds of the given formulas are also disclosed in the examples as given herein.

It has to be pointed out that the above described synthetic pathways can be modified by persons skilled in the art to produce all disorazoles and their analogues according to the present invention. The most important feature of the present invention is the use of two precursors as described as compounds of formula I and II. These compounds are each used twice in different steps.

## Examples

The following examples disclose the preferred embodiments of the present invention. It should be appreciated by those of skill in the art that the techniques disclosed in the examples which follow represent techniques discovered by the inventor to function well in the practice of the disclosure, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the disclosure.

## Synthesis of compounds of the invention

**General:** Solvents were dried by standard procedures and redistilled under $N_2$ atmosphere prior to use. All organometallic reactions were run under nitrogen. The products were purified by flash chromatography on Merck silica gel 60 (40-63 μm). Mass spectra were recorded on Finnigan MAT 95 and Waters Xevo G2-TOF spectrometers. NMR spectra were recorded on Brucker AVIII 400 and Brucker AVI 600 spectrometers. Optical rotations were recorded with a Perkin-Elmer 341 polarimeter.

Example 1:

(S)-(+)-Mandelic acid methyl ester (1)

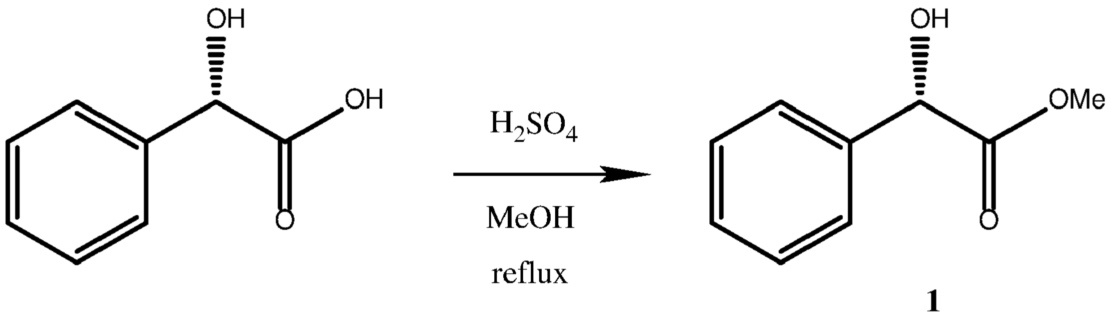


1

To a solution of (S)-(+)-Mandelic acid (57.13 g, 376 mmol, 1 eq) in MeOH (300 mL), concentrated sulfuric acid (600 μL, 11.3 mmol, 0.03 eq) was added and the mixture was refluxed for 4 h. The reaction was quenched with $K_2CO_3$ (1.04 g, 7.52 mmol, 0.02 eq) in 1.2 mL of water and the MeOH was evaporated in vacuo. Then $Et_2O$ (300 mL) was added and the solid was filtered off; the mixture was concentrated and crystallized from hexane (75 mL) to furnish ester **1** (54.9 g, 330.72 mmol, 88%) as a white solid.

**General Data:** $C_9H_{10}O_3$; FW: 166.06; Mp: 56-58°C; TLC: Rf= 0.35 (Pentane/$Et_2O$ 1:1); UV (+); Vanillin: yellow.

**$^1$H-NMR** (600 MHz, $CDCl_3$): δ (ppm): 7.36-7.34 (d, 2H); 7.31-7.30 (d, 2H); 7.27-7.26 (dd, 1H); 5.11 (s, 1H), 3.69 (s, 3H).

**$^{13}$C-NMR** (151 MHz, $CDCl_3$): δ (ppm): 174.17; 138.22; 128.65; 128.55; 126.65; 126.61; 72.89 (C-2); 53.09.

Example 2:

**(S)-(-)-1,1,2-Triphenyl-1,2-ethandiol (2)**

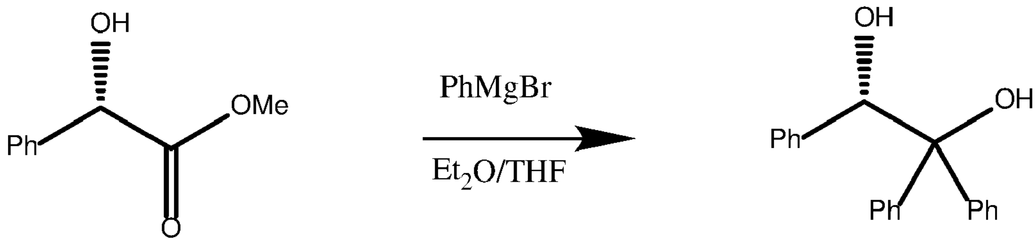


**1** **2**

To a solution of Phenyl magnesium bromide 3 M in $Et_2O$ (200 mL, 600 mmol, 5.0 eq), ester **1** (20 g, 120.36 mmol, 1 eq) in $Et_2O$ (120 mL) and THF (12 mL) was added dropwise at 0°C at such a rate that the temperature does not rise above 10°C (90 min required). The mixture was allowed to slowly warm to room temperature overnight and then refluxed for 1 h. After cooling to room temperature, the mixture was carefully poured into ice (200 g) and HCl 2 M was added dropwise to adjust the pH value to 4. The mixture was stirred for 1 h at room temperature and then the organic layer was separated. The aqueous phase was extracted with $CH_2Cl_2$ (3x200 mL) and the combined organic extracts were washed with $NaHCO_3$, dried over $MgSO_4$ and concentrated in vacuo. Crystallization of the residue from methanol (70 mL) afforded diol **2** (24.1 g, 83 mmol, 69%) as a white needle-shaped solid.

**General Data:** $C_{20}H_{18}O_2$; FW: 290.13; Mp: 123-127°C; $[\alpha]_D^{20}$ = -125.5 (*c* = 1.0, $CHCl_3$); TLC: Rf= 0.25 (Pentane/$Et_2O$ 2:1); UV (+); Vanillin: yellow.

**$^1$H-NMR** (600 MHz, $CDCl_3$): δ (ppm): 7.65-7.61 (m, 2H); 7.40-7.00 (m, 13H); 5.55 (s, 1H).

**$^{13}$C-NMR** (151 MHz, $CDCl_3$): δ (ppm): 145.12; 143.36; 138.81; 128.47; 128.09; 127.72; 127.64; 127.63; 127.48; 127.40; 127.30; 127.12; 127.02; 126.74; 126.19; 80.77; 50.89.

Example 3:

**(S)-(-)-2-Hydroxy-1,2,2-triphenylethylacetate [(S)-HYTRA] (3)**

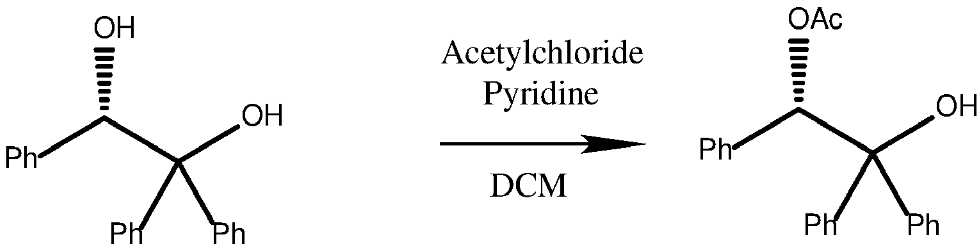


**2** **3**

Acetyl chloride (7.85 mL, 110.39, 1.33 eq) in $CH_2Cl_2$ (20 mL) was added dropwise at 0°C to a solution of diol 2 (24.1 g, 83 mmol, 1 eq) in $CH_2Cl_2$ (220 mL) and Pyridine (10.74 mL, 132.8 mmol, 1.6 eq), at such a rate that the temperature does not rise above 5°C. The mixture was stirred for 16 h at room temperature. Then water (100 mL) was added and the mixture was stirred for 10 min. The mixture was concentrated in vacuo until second phase $CH_2Cl_2$ was no longer observed. The white precipitate was filtered by suction, washed with water and HCl and dried by suction for 2-3 h. Then the solid was dissolved in hot toluene and the azeotrope was removed by atmospheric distillation. The mixture was allowed to cool to room temperature over several hours and then cooled to 0°C for 1 h. The precipitate was collected on a filter and recrystallized from acetone to furnish (S)-HYTRA **3** (23.2 g, 69.8 mmol, 84 %) as a white solid.

**General Data:** $C_{22}H_{20}O_3$; FW: 332.14; Mp: 249-251°C; $[\alpha]_D^{20}$ = -215.5 (*c* = 1.0, Pyridine); TLC: Rf= 0.50 (Pentane/$Et_2O$ 2:1); UV (+).

**$^1$H-NMR** (600 MHz, $CDCl_3$): δ (ppm): 7.50-7.48 (m, 2H); 7.31-6.98 (m, 13H); 6.61 (s, 1H); 2.76 (s, 1H); 1.92 (s, 3H).
**$^{13}$C-NMR** (151 MHz, $CDCl_3$): δ (ppm): 169.73; 144.82; 142.64; 135.82; 128.44; 128.38; 127.93; 127.79; 127.47; 127.35; 127.01; 126.41; 126.30; 126.17; 126.12; 125.03; 80.30; 78.50; 21.14.

Example 4:

**Ethyl 3-hydroxy-2,2-dimethylpentanoate (4)**

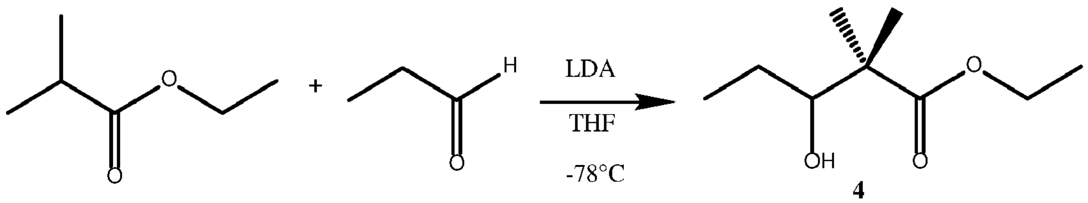


4

*n*-BuLi (133 mL, 332.38 mmol, 1.1 eq, 2.5 M solution in hexane) was added dropwise at -78°C to a solution of diisopropylamine (46.5 mL, 332.37 mmol, 1.1 eq) in THF (300 mL). This LDA solution was stirred for 30 min at 0°C and then cooled to -78°C. Ethyl isobutyrate (40.6 mL, 302.16 mmol, 1 eq) dissolved in THF (58 mL) was added dropwise and the mixture was stirred for 1 h at -78°C. Propionaldehyde (23.8 mL, 332.38 mmol, 1.1 eq) was added dropwise at -78°C and then the bath was removed and the mixture was stirred for 30 min between -50°C and -10°C. The reaction was quenched by dropwise addition of saturated aqueous $NH_4Cl$ solution (300 mL) and the organic layer was separated and the aqueous phase was extracted with $Et_2O$ (3x200 mL). The combined organic extracts were dried over $MgSO_4$ and concentrated in vacuo. Purification of the residue by vacuum distillation through a short Vigreux column afforded β-hydroxy ester **4** (48.97 g, 281.25 mmol, 93%) as a colorless liquid.
**General Data:** $C_9H_{18}O_3$; FW: 174.13; Bp: 105°C (5 mbar); TLC: $R_f$ = 0.35 (Pentane/$Et_2O$ 2:1); UV (-); Vanillin: light blue.
**$^1$H-NMR** (600 MHz, $CDCl_3$): δ (ppm): 4.16 (q, *J*= 7.27 Hz, J= 21.46 Hz, 2H); 3.45 (dd, *J* = 10.68, *J* = 2.02, 1H); 1.54-1.48 (m, 2H); 1.25 (t, *J* = 7.34 Hz, 3H); 1.17 (s, 3H,); 1.15 (s, 3H); 0.95 (t, *J* = 7.77 Hz, 3H).
**$^{13}$C-NMR** (151 MHz, $CDCl_3$): δ (ppm): 177.80; 78.39; 60.62; 47.02; 24.58; 20.42; 14.15; 11.29.
**MS** (EI): *m/z* (%): 175.07 (100) $[M+H]^+$, 173.06 (68), 170.05 (26), 169.04 (22).
**HR-MS:** calculated: 174.1329, found: 175.1329 $[M+H]^+$, 197.1152 $[M+Na^+]^+$.

Example 5:

**Ethyl (E)-2,2-dimethyl-3-pentenoate (5)**

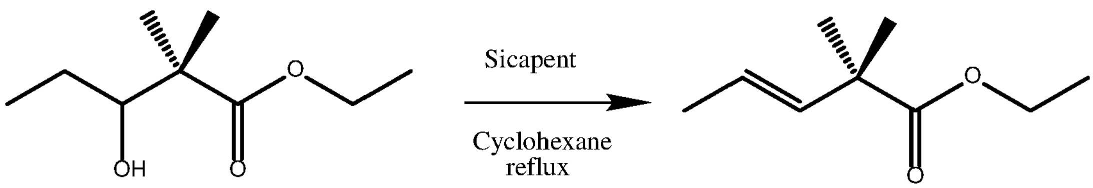


4 5

Hydroxy ester 4 (48.97 g, 281.25 mmol) was refluxed with Sicapent (70 g) in Cyclohexane (250 mL) for 30 min. The solvent was removed by atmospheric distillation and vacuum distillation of the residue afforded ester 5 (29.85 g, 191.25 mmol, 68%) as a colorless liquid.
**General Data:** $C_9H_{16}O_2$; FW: 156.12; Bp: 55-60°C (10 mbar); TLC: Rf= 0.75 (Pentane/$Et_2O$ 2:1); UV (-); Vanillin: blue.
**$^1$H-NMR** (600 MHz, $CDCl_3$): δ (ppm): 5.64 (dq, *J* = 15.72 Hz, *J* = 1.6 Hz 1H); 5.53-5.47 (m, 1H); 4.14 (qd, *J* = 7.09 Hz, *J* = 1.18 Hz, 2H); 1.68 (dd, J = 6.24 Hz, *J* = 1.58 Hz 3H), 1.27 (s, 6H); 1.25 (t, *J* = 7.15 Hz, 3H).
**$^{13}$C-NMR** (151 MHz, $CDCl_3$): δ (ppm): 176.83; 135.61; 123.33; 60.54; 44.02; 25.13; 18.03; 14.15.
**MS** (EI): *m/z* (%): 156.07 (100) $[M]^+$, 141.05 (77), 116.03 (54), 110.02 (21). **HR-MS:** calculated: 156.1145, found:

156.1145.

Example 6:

**(E)- 2,2-dimethyl-3-penten-1-ol (6)**

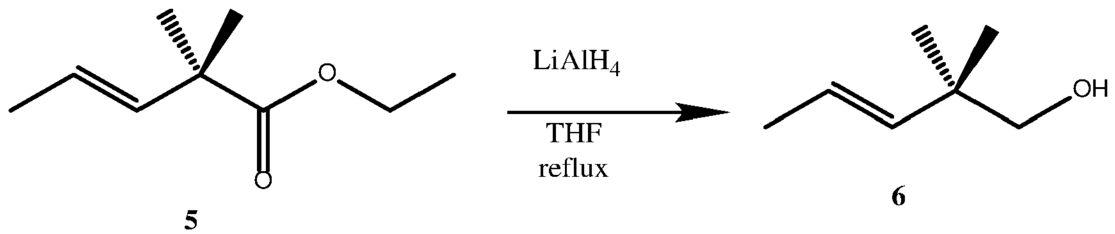


5

6

To a solution of ester **5** (16.37 g, 104.94 mmol, 1 eq) in THF (150 mL), $LiAlH_4$ (7.98 g, 209.87 mmol, 2 eq) was added and the mixture was refluxed for 2 h. After cooling to 0°C, $Et_2O$ (100 mL) was added and the reaction was quenched by dropwise addition of water (10 mL). The mixture was stirred for 30 min at room temperature until a white precipitate was formed, which was filtered off by suction through Celite and washed with $Et_2O$ (3x200 mL). The filtrate and the washings were combined and concentrated in vacuo to furnish crude alcohol **6** (8.97 g, 78.7 mmol, 75%) as a colorless liquid, which was used for the preparation of aldehyde **7** without further purification.
**General Data:** $C_7H_{14}O$; FW: 114.10; Bp: 152-160°C; TLC: Rf= 0.35 (Pentane/$Et_2O$ 2:1); UV (-); dark blue.
**$^1$H-NMR** (600 MHz, $CDCl_3$): δ (ppm): 5.50-5.44 (m, 1H); 5.35 (dq, *J* = 15.68 Hz, *J* = 1.29 Hz, 1H); 3.28 (s, 2H); 1.69 (d, *J* = 6.22 Hz, 3H); 1.65 (s, 1H); 0.977 (s, 6H).
**$^{13}$C-NMR** (151 MHz, $CDCl_3$): δ (ppm): 137.76; 124.14; 71.6; 38.33; 46.21; 23.92; 18.23.
**HR-MS:** calculated: 114.10, found: 115.11 [*M*+H]$^+$.

Example 7:

**(E)-2,2-dimethyl-3-pentenal (7)**

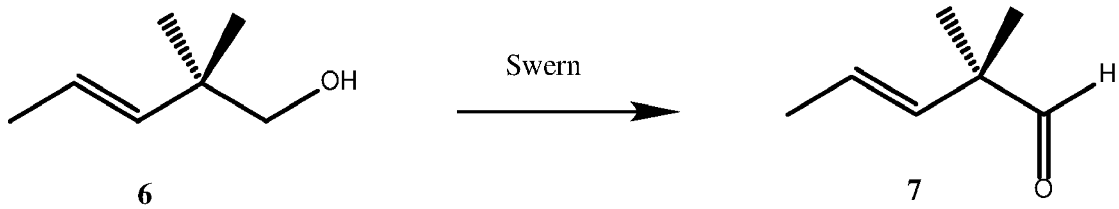


6

7

DMSO (11.2 mL, 157.4 mmol, 2.0 eq) in $CH_2Cl_2$ (45 mL) was added dropwise at -78°C to a stirred solution of $(COCl)_2$ (8.1 mL, 94.44 mmol, 1.2 eq) in $CH_2Cl_2$ (210 mL). The mixture was stirred for 10 min at -78°C. The crude (E)-2,2-dimethyl-3-penten-1-ol 6 (8.97 g, 78.7 mmol, 1 eq) dissolved in $CH_2Cl_2$ (60 mL) was added dropwise at -78°C and the mixture was stirred for 1 h at - 78°C. The reaction was quenched by dropwise addition of $NEt_3$ (54.6 mL, 393.5 mmol, 5.0 eq) and the mixture was warmed to room temperature over 45 min. Water was added (250 mL) and the mixture was stirred for 10 min. The organic layer was separated and the aqueous phase was extracted with $CH_2Cl_2$ (3x150 mL). The combined organic extracts were dried over $MgSO_4$ and concentrated in vacuo. Vacuum distillation of the residue afforded aldehyde 7 (6.6 g, 79.02 mmol, 75%) as a colorless liquid.
**General Data:** $C_7H_{14}O$; FW: 112.09; Bp: 127-128°C; TLC: Rf= 0.80 (Pentane/$Et_2O$ 5:1); UV (-); Vanillin: dark blue.
**$^1$H-NMR** (600 MHz, $CDCl_3$): δ (ppm): 9.32 (s, 1 H); 5.58-5.48 (m, 1H); 5.36 (dq, *J* = 15.76 Hz, *J* = 1.69 Hz, 1H); 1.71 (dd, *J* = 6.41 Hz, *J* = 1.61 Hz, 3H); 1.14 (s, 6H).
**$^{13}$C-NMR** (151 MHz, $CDCl_3$): δ (ppm): 202.92; 132.46; 127.06; 46.21; 21.49; 18.3.

Example 8:

**(1S)-2-Hydoxy-1,2,2-triphenylethl (3S,5E)-3hydoxy-4,4-dimethyl-5-heptenoate (8)**

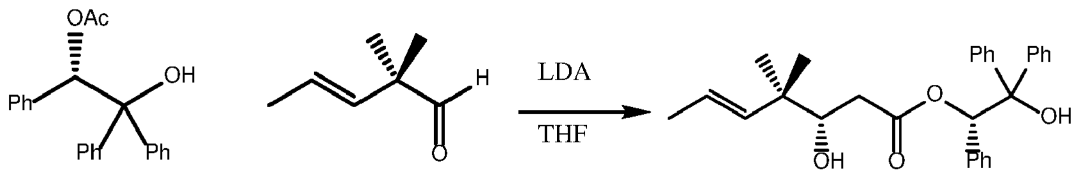


3 7 8

n-BuLi (22.5 mL, 56.32 mmol, 2.2 eq, 2.5 M solution in hexane) was added dropwise at -78°C to a solution of diisopropylamine (7.9 mL, 56.32 mmol, 2.2 eq) in THF (80 mL). This LDA solution was stirred for 30 min at 0°C and then added dropwise to a solution of (S)-HYTRA 3 (8.5 g, 25.6 mmol, 1 eq) in THF (150 mL) at -78°C. The mixture was stirred for 1 h at 0°C. The resulting orange-red solution was cooled to -78°C and a solution of aldehyde 7 (3.44 mg, 30.7 mmol, 1.2 eq) in THF (7 mL) was added dropwise. The mixture was stirred for 2h30min at -78°C. The reaction was quenched by dropwise addition of saturated aqueous $NH_4Cl$ solution (150 mL) and the mixture was allowed to warm to room temperature over 30 min. The organic layer was separated and the aqueous phase was extracted with $CH_2Cl_2$ (3x100 mL). The combined organic extracts were dried over $MgSO_4$ and concentrated in vacuo. Purification of the residue by flash chromatography (pentane/$Et_2O$ 3:1) afforded β-hydroxy ester **8** (8.75 g, 19.71 mmol, 77%) as a colorless solid.

**General Data:** $C_{29}H_{32}O_4$; FW: 444.23; Melting point: 120 - 126°C; $[\alpha]_D^{20}$ = - 167.8 (*c* = 1.0, $CHCl_3$); TLC: Rf= 0.45 (Pentane/$Et_2O$ 2:1); UV (+); Vanillin: green.

**$^1$H-NMR** (600 MHz, $CDCl_3$): δ (ppm): 7.61-7.53 (m, 2H); 7.39-7.33 (m, 2H); 7.31-7.26 (m, 1H); 7.22-7.03 (m, 10H); 6.71 (s, 1H); 5.43-5.45 (m, 1H); 5.26 (dd, *J* = 15.72, *J* = 1.31 Hz, 1H); 3.51 (dd, *J* = 10.38 Hz, *J* = 2.18 Hz, 1H); 2.79 (s, 1H); 2.36 (dd, *J* = 15.81, Hz, *J* = 2.07 Hz, 1H); 2.24 (dd, *J* = 16.29 Hz, *J* = 10.35 Hz, 1H); 1.98 (s, 1H); 1.65 (dd, *J* = 6.08 Hz, *J* = 1.28 Hz, 3H); 0.92 (s, 3H); 0.91 (s, 3H).

**$^{13}$C-NMR** (151 MHz, $CDCl_3$): δ (ppm): 172.23; 144.61; 142.58; 137.02; 135.42; 128.41; 128.35; 127.99; 127.83; 127.54; 127.36; 127.11; 127.28; 127.20; 123.86; 80.33; 78.96; 74.86; 40.03; 37.35; 23.62; 22.68; 18.26.

**MS** (EI): *m/z* (%): 911.45 (40) [2*M*+Na$^+$]$^+$, 467.22 (97) [*M*+Na$^+$]$^+$, (77), 444.23 (<0.4), 273.13 (100), 255.12 (18), 195.08 (31).

**HR-MS:** calculated: 444.2298, found: 467.2198 [*M*+Na$^+$]$^+$.

Example 9:

**(3S,5E)-4,4-dimethyl-5-heptene-1,3-diol (9)**

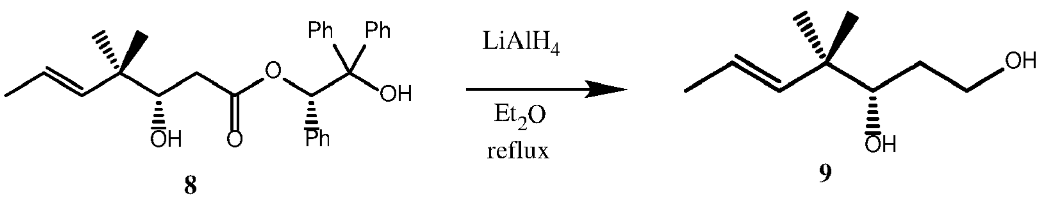


8 9

$LiAlH_4$ (5.2 g, 137.97 mmol, 7.0 eq) was added portionwise to a refluxing solution of β-hydroxy ester **13** (8.75 g, 19.71 mmol, 1 eq) in $Et_2O$ (210 mL), during a period of 2 h. Reflux was continued for 30 min. After cooling to 0°C, the reaction was quenched by dropwise addition of water (10 mL). Then $Et_2O$ (150 mL) and water (0.95 mL) was and the mixture was stirred for 30 min at room temperature until a white precipitate was formed. The precipitate was filtered off by suction through Celite and washed with $Et_2O$ (4x100 mL). The filtrate and the washings were combined and concentrated in vacuo. Purification of the residue by flash chromatography (pentane/$Et_2O$ 2:1 to pure $Et_2O$) afforded diol **9** (2.5 g, 15.77 mmol, 80%) as a colorless oil and (S)-(-)-1,1,2-triphenyl-1,2-ethandiol **2** (5.1 g, 17.74 mmol, 90 %)

**General Data:** $C_9H_{18}O_2$; FW: 158.13; $[\alpha]_D^{20}$ = -7.28 (*c* = 0.7, $CHCl_3$); TLC: Rf= 0.30 ($Et_2O$); UV (-); Vanillin: dark blue.
**$^1$H-NMR** (600 MHz, $CDCl_3$): δ (ppm): 5.52-5.43 (m, 1 H); 5.35 (dq, J= 15.69 Hz, *J* = 1.29 Hz, 1H); 3.86-3.76 (m, 2H); 3.48 (dd, *J* = 10.49 Hz, *J* = 2.16 Hz, 1H); 2.39 (s, 1H); 1.68 (dd, *J* = 5.96 Hz, *J* = 1.23 Hz, 3H); 1.74-1.66 (m, 1H); 1.74-1.66 (m, 1H); 1.62-1.52 (m, 1H); 0.98 (s, 6H).
**$^{13}$C-NMR** (151 MHz, $CDCl_3$): δ (ppm): 137.55; 124.51; 78.88; 62.56; 40.71; 32.62; 23.67; 22.16; 18.30.
**MS** (EI): *m/z* (%): 181.1207 (100), 158.13 (<0.4), 123.12 (42).
**HR-MS:** calculated: 158.1307, found: 181.1207 $[M+Na^+]^+$.

Example 10:

**(S,E)-3,3,9,9-tetraethyl-5-(2-methylpent-3-en-2-yl)-4,8-dioxa-3,9-disilaundecane (10)**

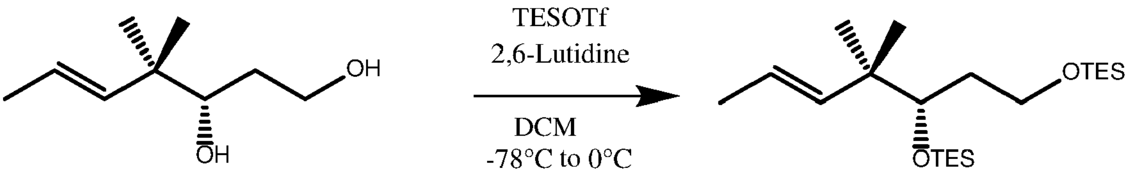


9 10

To a solution of diol **9** (1.96 g, 12.4 mmol, 1 eq) in $CH_2Cl_2$ (120 mL), 2,6-Lutidine (5.8 mL, 49.6 mmol, 4 eq) and TESOTf (8.4 mL, 37.18 mmol, 3 eq) were sequentially added dropwise at -78°C. The mixture was stirred for 30 min at -78°C and for 1 h at 0°C. Saturated aqueous $NaHCO_3$ solution (100 mL) was added and the layers were separated. The aqueous phase was extracted with $CH_2Cl_2$ (3x80 mL) and the combined organic extracts were dried over $MgSO_4$ and concentrated in vacuo. Purification of the residue by flash chromatography (pure pentane) furnished compound **9** (4.74 g, 12.28 mmol, 99%) as a colorless liquid.

**General Data:** $C_{21}H_{46}O_2Si_2$; FW: 386.3; $[\alpha]_D^{20}$ = -7.25 (*c* = 0.4, $CHCl_3$); TLC: Rf= 0.20 (pentane); UV (-); Vanillin: black.
**$^1$H-NMR** (600 MHz, $CDCl_3$): δ (ppm): 5.43 (dq, *J* = 15.67 Hz, *J* = 1.22 Hz, 1H); 5.38-5.29 (m, 1H); 3.71-3.63 (m, 1H); 3.61-3.53 (m, 1H); 3.46 (dd, , *J* = 8.17 Hz, *J* = 2.7 Hz); 1.65 (dd, *J* = 5.93 Hz, *J* = 1.19, 3H); 1.77-1.68 (m, 1H); 1.53-1.43 (m, 1H); 1.00-0.89 (m, 18H); 0.96 (s, 3H); 0.92 (s, 3H); 0.66-0.47 (m, 12H).
**$^{13}$C-NMR** (151 MHz, $CDCl_3$): δ (ppm): 139.29; 121.46; 76.87; 60.87; 41.12; 36.71; 24.63; 22.90; 18.30; 7.14; 6.81; 6.79; 6.41; 5.47; 4.46.

Example 11:

**(S)-2,2-dimethyl-3,5-bis((triethylsilyl)oxy)pentanal (11)**

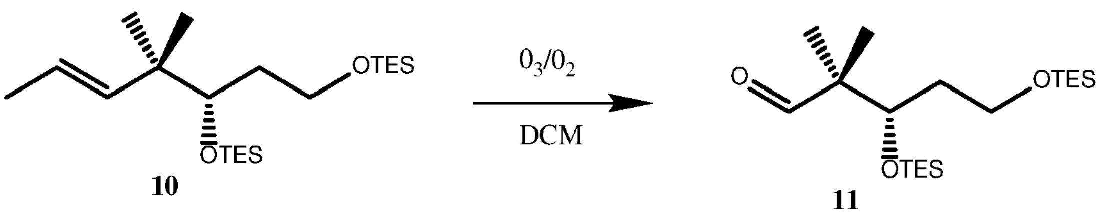


10 11

A stream of ozone in oxygen was bubbled through a solution of compound **10** (4.74 g, 12.28 mmol, 1 eq) in $CH_2Cl_2$ (400

mL) at -78°C until the blue color of the solution persisted. Then $PPh_3$ (3.86 g, 14.73 mmol, 1.2 eq) was added at -78°C and the mixture was allowed to slowly warm to room temperature over 4 h. The crude was concentrated in vacuo and purified by flash chromatography (pentane/$Et_2O$ 200:1 to 50:1) to furnish aldehyde **11** (2.75 g, 7.36 mmol, 60 %) as a colorless liquid.

**General Data:** $C_{19}H_{42}O_3Si_2$; FW: 374.27; $[\alpha]_D^{20} = +10.11$ (*c* = 0.9, $CHCl_3$); TLC: Rf= 0.35 (Pentane/$Et_2O$ 50:1); UV (-); Vanillin: violet.

**$^1$H-NMR** (600 MHz, $CDCl_3$): δ (ppm): 9.57 (s, 1H); 3.99 (dd, *J* = 8.13 Hz, *J* = 3.02 Hz, 1H); 3.71-3.58 (m, 2H); 1.74-1.64 (m, 1H); 1.62-1.52 (m, 1H); 1.04 (s, 3H); 1.00 (s, 3H); 0.98-0.908 (m, 18H); 0.65-0.54 (m, 12H).

**$^{13}$C-NMR** (151 MHz, $CDCl_3$): δ (ppm): 206.6; 73.21; 59.53; 51.09; 36.33; 18.88; 17.34; 6.97; 6.78; 5.35; 4.43.

Example 12:

**(4S,6S)-5,5-dimethyl-6,8-bis((triethylsilyl)oxy)oct-1-en-4-ol (12)**

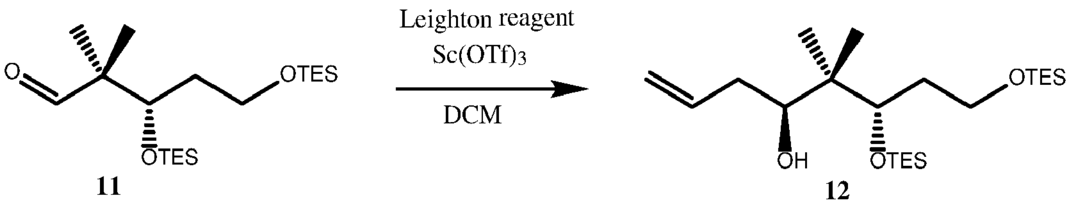


11 12

A solution of Leighton reagent (4.74 g, 8.58 mmol, 1.2 eq) in $CH_2Cl_2$ (20 mL) was added to a solution of aldehyde **11** (2.67 g, 7.15 mmol, 1 eq) in $CH_2Cl_2$ (50 mL). Then $Sc(OTf)_3$ (175 mg, 0.375 mmol, 0.05 eq) was added and the mixture was stirred for 24 h at room temperature. TBAF trihydrate (2.26 g, 7.15 mmol, 1 eq) was added and the mixture was stirred for 30 min at room temperature. The solvent was evaporated and the residue was purified by flash chromatography (pentane/$Et_2O$ 50:1, then pentane/EtOAc/$NEt_3$ 1:1:0.1) to furnish the allylic alcohol (2.36 g, 5.65 mmol, 80%) as a colorless liquid and the recovered diamine (3.64 g, 8.08 mmol, 87 %) as a yellow paste.

**General Data:** $C_{22}H_{48}O_3Si_2$; FW: 416.31; $[\alpha]_D^{20} = -4.2$ (*c* = 0.5, $CHCl_3$); TLC: Rf= 0.2 (Pentane/$Et_2O$ 50:1); UV (-); Vanillin: dark blue.

**$^1$H-NMR** (600 MHz, $CDCl_3$): δ (ppm): 5.94-5.86 (m, 1H); 5.13-5.05 (m, 2H); 3.79 (dd, *J* = 6.76 Hz, *J* = 2.79 Hz, 1H); 3.74 (m, 1H); 3.65 (m, 1H); 3.51 (d, *J* = 10.49 Hz, 1H); 2.73 (s, 1H); 2.31-2.25 (m, 1H); 2.1-2.02 (m, 1H); 2.01-1.93 (m, 1H); 1.54-1.48 (m, 1H); 0.993-0.923 (m, 18H); 0.897 (s, 3H); 0.781 (s, 3H); 0.65-0.54 (m, 12H).

**$^{13}$C-NMR** (151 MHz, $CDCl_3$): δ (ppm): 137.22; 116.51; 75.64; 75.41; 61.24; 42.44; 36.46; 36.37; 18.72; 18.49; 7.08; 6.72; 5.51; 4.28.

Example 13:

**(5S,7S)-5-allyl-11,11-diethyl-6,6-dimethyl-7-((triethylsilyl)oxy)-2,4,10-trioxa-11-silatridecane (13)**

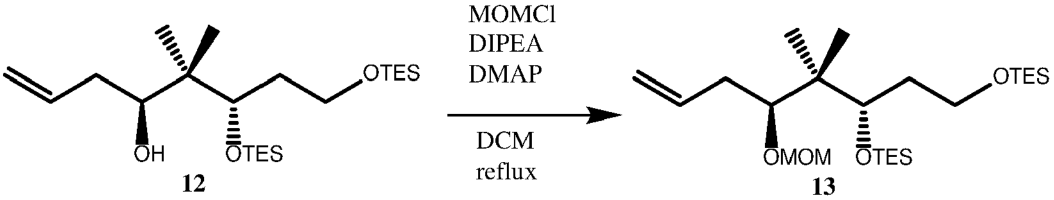


12 13

MOMCl (1.3 mL, 17 mmol, 3 eq) was added dropwise at 0°C to a solution of allylic alcohol **12** (2.36 g, 5.67

mmol, 1 eq), DIPEA (3 mL, 17 mmol, 3 eq) and DMAP (207 mg, 1.7 mmol, 0.3 eq) in $CH_2Cl_2$ (60 mL). The mixture was stirred overnight at 45°C. Evaporation of the solvent and purification of the residue by flash chromatography (Pen/$Et_2O$ 60:1) afforded the protected alcohol (2.4 g, 5.21 mmol, 92 %) as a colorless liquid.

**General Data:** $C_{24}H_{52}O_4Si_2$; FW: 460.34; $[\alpha]_D^{20}$ = -10.67 (*c* = 0.75, $CHCl_3$); TLC: Rf= 0.5 (Pentane/$Et_2O$ 50:1); UV (-); Vanillin: dark blue.
**$^1$H-NMR** (600 MHz, $CDCl_3$): δ (ppm): 5.95-5.87 (m, 1H); 5.09-4.98 (m, 2H); 4.61 (q, *J* = 6.61 Hz, 2H); 3.74-3.68 (m, 2H); 3.63.3.57 (m, 1H); 3.48 (dd, *J* = 8.59 Hz, *J* = 2.85, Hz 1H); 2.48-2.41 (m, 1H); 2.23-2.14 (m, 1H); 1.91-1.83 (m, 1H); 1.55-1.48 (m, 1H); 1.00-0.923 (m, 18H); 0.917 (s, 3H); 0.811 (s, 3H); 0.655-0.565 (m, 12H).
**$^{13}$C-NMR** (151 MHz, $CDCl_3$): δ (ppm): 137.22; 115.95; 98.09; 83.46; 74.54; 60.86; 56.11; 43.39; 36.17; 20.78; 19.26; 7.14; 6.79; 5.63; 4.43.

Example 14:

**(3S,5S,E)-5-(methoxymethoxy)-4,4-dimethyl-3-((triethylsilyl)oxy)non-7-en-1-ol (15)**

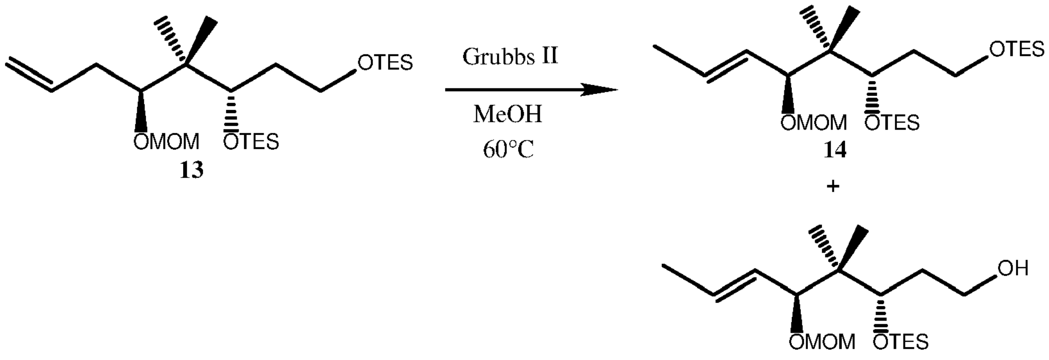


13

14

+

15

A solution of protected triol **13** (2.3 g, 5 mmol, 1 eq) and Grubbs II (212 mg, 0.250 mmol, 0.05 eq) in MeOH was stirred overnight at 60°C. The mixture was then concentrated in vacuo and the residue was filtered on a pad of silica gel (Pen/$Et_2O$ 1:1). The filtrate was concentrated in vacuo affording a mixture of **15** (80%) and **14** (15%) as a colorless liquid, which was used in the next step without further purification.

**General Data (14):** $C_{25}H_{52}O_4Si_2$; FW: 460.34; $[\alpha]_D^{20}$ (*c* = 0.2, $CHCl_3$); TLC: Rf= 0.5 (Pentane/$Et_2O$ 50:1); UV (-); Vanillin: dark blue.
**$^1$H-NMR (14)** (600 MHz, $CDCl_3$): δ (ppm): 5.64-5.54 (m, 1H); 5.31 (qd, *J* = 7.79 Hz, *J* = 1.69 Hz, 1H); 4.66 (d, *J* = 6.58 Hz, 1H); 4.43 (d, *J* = 6.58 Hz, 1H); 3.86 (d, *J* = 8.85 Hz, 1H); 3.75 (dd, *J* = 8.62 Hz, *J* = 2.38 Hz, 1H); 3.73.3.55 (m, 2H); 3.35 (s, 3H); 1.94-1.82 (m, 1H); 1.71 (dd, *J* = 6.37 Hz, *J*= 1.54 Hz, 3H) 1.63-1.53 (m, 1H); 1.01-0.878 (m, 18H); 0.948 (s, 3H); 0.939 (s, 3H); 0.668-0.540 (m, 12H).
**$^{13}$C-NMR (14)** (151 MHz, $CDCl_3$): δ (ppm): 130.63; 127.94; 93.43; 81.11; 74.3; 61.09; 55.74; 42.18; 35.74; 20.10; 19.35; 17.79; 7.14; 6.77; 5.63; 4.37.

**General Data (15):** $C_{18}H_{38}O_4Si$; FW: 346.25; $[\alpha]_D^{20}$ = -69.0 (*c* = 0.8, $CHCl_3$); TLC: Rf= 0.3 (Pentane/$Et_2O$ 3:1); UV (-); Vanillin: dark blue.
**$^1$H-NMR (15)** (600 MHz, $CDCl_3$): δ (ppm): 5.64-5.54 (m, 1H); 5.31 (qd, *J* = 6.70 Hz, *J* = 1.64 Hz, 1H); 4.62 (d, *J* = 6.46 Hz, 1H); 4.43 (d, *J* = 6.46 Hz, 1H); 3.87 (dd, *J* = 8.68 Hz, *J* = 2.59 Hz, 2H); 3.81 (d, *J* = 8.50 Hz, 1H); 3.79-3.72 (m, 1H); 3.71-3.62 (m, 1H); 3.33 (s, 3H); 1.95-1.84 (m, 1H); 1.71 (dd, *J* = 6.48 Hz, *J* = 1.53 Hz, 3H); 1.67-1.57 (m, 1H); 1.00-0.932 (m, 9H); 0.923 (s, 3H); 0.736 (s, 3H); 0.678-0.583 (m, 6H).
**$^{13}$C-NMR (15)** (151 MHz, $CDCl_3$): δ (ppm): 130.84; 127.77; 93.64; 81.75; 74.46; 60.85; 55.67; 42.06; 35.34; 20.10; 19.10; 17.82; 7.11; 5.60.

Example 15:

**(3S,5S,E)-5-(methoxymethoxy)-4,4-dimethyl-3-((triethylsilyl)oxy)oct-6-enal (16)**

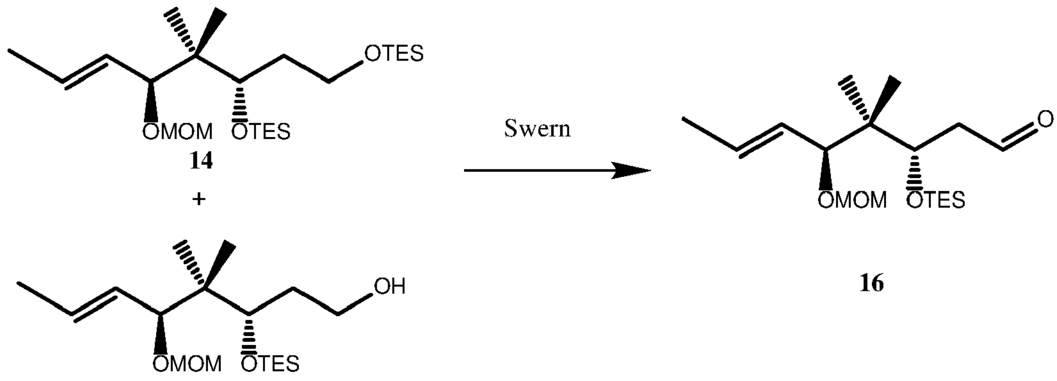


14 + 15

16

DMSO (0.780 mL, 11 mmol, 2 eq) in $CH_2Cl_2$ (5 mL) was added dropwise to a solution of oxalyl chloride (0.644 mL, 7.5 mmol, 1.2 eq) in $CH_2Cl_2$ (20 mL) at - 78°C. The mixture was stirred for 10 min at -78°C and then the crude **14** + **15** dissolved in $CH_2Cl_2$ (5 mL) was added dropwise. The reaction was stirred for 1 h at -78°C, quenched by dropwise addition of $NEt_3$ (3.5 mL, 25 mmol, 5 eq) and then warmed to room temperature over 45 min. $H_2O$ (30 mL) was added and the layers were separated. The aqueous phase was extracted with $CH_2Cl_2$ (3x20 mL) and the combined organic extracts were dried over $Na_2SO_4$, filtered and concentrated in vacuo. Purification of the residue by flash chromatography (Pen/$Et_2O$ 10:1) afforded aldehyde **16** (1.33 g, 3.86 mmol, 77%) as a colorless liquid.

**General Data:** $C_{18}H_{36}O_4Si_2$; FW: 344.24; $[\alpha]_D^{20}$ = -54.87 (*c* = 0.8, $CHCl_3$); TLC: Rf= 0.3 (Pentane/$Et_2O$ 10:1); UV (-); Vanillin: grey.

**$^1$H-NMR** (600 MHz, $CDCl_3$): δ (ppm): 9.83 (dd, *J* = 3.00 Hz, *J* = 1.21 Hz, 1H); 5.66-5.56 (m, 1H); 5.29 (qd, *J* = 7.60 Hz, *J* = 1.66 Hz, 1H); 4.64 (d, *J* = 6.71 Hz, 1H); 4.41 (d, *J* = 6.71 Hz, 1H); 4.31 (dd, *J* = 7.05 Hz, *J* = 3.59 Hz, 1H); 3.78 (d, *J* = 8.70 Hz, 1H); 3.33 (s, 3H); 2.75 (qd, *J* = 16.71, Hz, *J* = 1.33 Hz, 1H); 2.58 (qd, *J* = 16.23, Hz, *J* = 3.01 Hz, 1H); 1.72 (dd, *J* = 6.48 Hz, *J* = 1.67 Hz, 3H); 0.965 (s, 3H); 0.957-0.907 (m, 9H); 0.767 (s, 3H); 0.640-0.545 (m, 6H).

**$^{13}$C-NMR** (151 MHz, $CDCl_3$): δ (ppm): 202.75; 131.42; 127.20; 93.26; 81.37; 72.01; 55.87; 47.82; 42.06; 20.05; 19.74; 6.99; 5.35.

Example 16:

**(5S,7S)-9,9-diethyl-7-((Z)-3-iodoallyl)-6,6-dimethyl-5-((E)-prop-1-en-1-yl)-2,4,8-trioxa-9-silaundecane (17)**

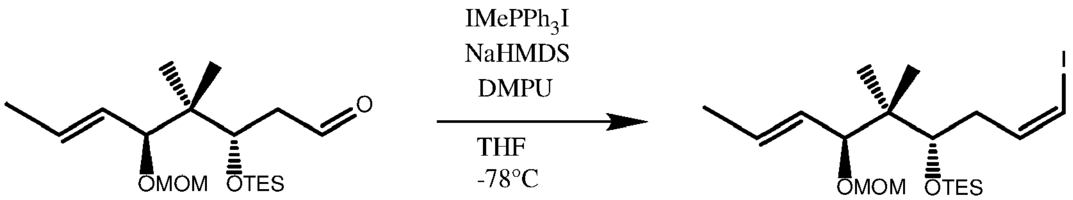


16

17

NaHMDS (1 M in THF, 4.2 mL, 4.2 mmol, 1.5 eq) was added dropwise at 0°C to a solution of $IMePPh_3I$ (2.22 g, 4.2 mmol, 1.5 eq) in THF (30 mL). The red solution was stirred for 10 min at room temperature and then cooled to

-78°C. DMPU (2.5 mL, 20.93 mmol, 7.5 eq) was added dropwise, followed by aldehyde **16** (960 mg, 2.79 mmol, 1 eq) in THF (7 mL). The mixture was stirred for 1 h at -78°C and 30 min at room temperature. Saturated $NH_4Cl$ solution was added and the aqueous phase was extracted with $Et_2O$ (3x25 mL). The combined organic layers were dried over $Na_2SO_4$, filtered and concentrated in vacuo. The residue was purified by flash chromatography (Pen/$Et_2O$ 100:1) giving the Z Iodide **17** (875 mg, 1.87 mmol, 67%) as a slightly yellow liquid.

**General Data:** $C_{19}H_{37}IO_3Si$; FW: 468.16; $[\alpha]_D^{20}$ = -30.133 (*c* = 0.75, $CHCl_3$); TLC: Rf= 0.25 (Pentane/$Et_2O$ 100:1); UV (-); Vanillin: black.

**$^1$H-NMR** (600 MHz, $CDCl_3$): δ (ppm): 6.36 (q, *J* = 7.43 Hz, 1H); 6.21 (dt, *J* = 7.36 Hz, *J* = 1.67 Hz, 1H); 5.69-5.56 (m, 1H); 5.33 (qd, *J* = 6.69 Hz, *J* = 1.62 Hz, 1H); 4.68 (d, *J* = 6.60 Hz, 1H); 4.45 (d, *J* = 6.60 Hz, 1H); 3.88 (t, *J* = 5.03 Hz, 1H); 3.86 (d, *J* = 3.80 Hz, 1H); 3.36 (s, 3H); 2.44-2.38 (m, 2H); 1.73 (dd, *J* = 6.52 Hz, *J* = 1.57 Hz, 3H); 1.02-0.922 (m, 9H); 0.941 (s, 3H); 0.769 (s, 3H); 0.658-0.559 (m, 6H).

**$^{13}$C-NMR** (151 MHz, $CDCl_3$): δ (ppm): 140.20; 131.02; 127.77; 93.49; 82.57; 81.41; 75.63; 55.90; 42.65; 38.61; 20.16; 19.60; 17.85; 7.12; 5.57.

Example 17:

**(1Z,4S,6S,7E)-1-iodo-6-(methoxymethoxy)-5,5-dimethylnona-1,7-dien-4-ol (18)**

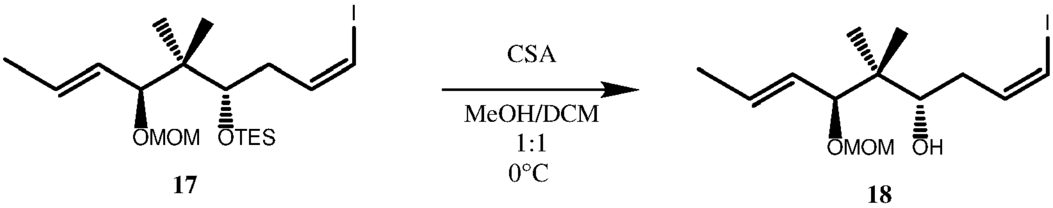


17 18

CSA (93 mg, 0.4 mmol, 0.2 eq) was added at 0°C to a solution of Z Iodide **17** (940 mg, 2 mmol, 1 eq) in $CH_2Cl_2$ (50 mL) and MeOH (50 mL). The mixture was stirred for 1 h at 0°C without $N_2$. Saturated $NaHCO_3$ solution (100 mL) was added and the layers were separated. The aqueous phase was extracted with $CH_2Cl_2$ (3x100 mL) and the combined organic extracts were dried over $Na_2SO_4$, filtered and concentrated in vacuo. The residue was purified by flash chromatography (Pen/$Et_2O$ 3:1) giving deprotected Iodide **18** (601 mg, 1.7 mmol, 85%) as a slightly yellow liquid.

**General Data:** $C_{13}H_{23}IO_3$; FW: 354.07; $[\alpha]_D^{20}$ = -52.7 (*c* = 1.00, $CHCl_3$); TLC: Rf= 0.3 (Pentane/$Et_2O$ 3:1); UV (-); Vanillin: dark blue.

**$^1$H-NMR** (600 MHz, $CDCl_3$): δ (ppm): 6.39 (q, *J* = 6.68 Hz, 1H); 6.28 (dt, *J* = 7.24 Hz, *J* = 1.18 Hz, 1H); 5.74-5.60 (m, 1H); 5.32 (qd, *J* = 6.34 Hz, *J* = 1.49 Hz, 1H); 4.72 (d, *J* = 6.65 Hz, 1H); 4.46 (d, *J* = 6.65 Hz, 1H); 3.89 (d, *J* = 9.12 Hz, 1H); 3.66 (dd, *J* = 10.22 Hz, *J* = 2.74 Hz, 1H); 3.38 (s, 3H); 2.42-2.32 (m, 1H); 2.30-2.16 (m, 1H); 1.74 (dd, *J* = 6.46 Hz, *J* = 1.42 Hz, 3H); 0.980 (s, 3H); 0.826 (s, 3H).

**$^{13}$C-NMR** (151 MHz, $CDCl_3$): δ (ppm): 139.48; 132.59; 126.83; 93.01; 84.89; 83.50; 77.43; 56.03; 40.94; 37.31; 21.62; 17.87; 15.85.

Example 18:

***tert*-Butyl (R)-3-hydroxypent-4-enoate (19)**

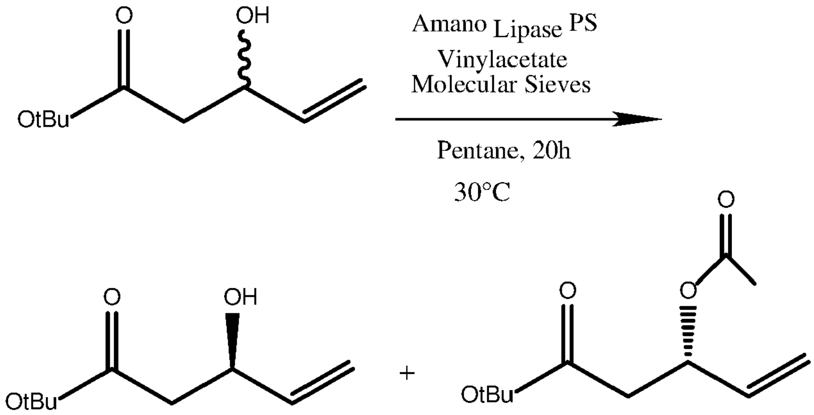


**(R)-19** (99:1 er) 47% **(S)-20** 48%

Vinyl acetate (16 mL, 174.18 mmol, 3 eq) was added to a solution of racemic *tert*-Butyl 3-hydroxypent-4-enoate (10 g, 58.06 mmol, 1 eq) in pentane (200 mL). Then Amano Lipase PS (6 g) and 4Â molecular sieves (9.5 g) were added and the mixture was stirred for 24 h at 30°C. The solids were filtered on paper and washed with $Et_2O$; the filtrate was concentrated in vacuo and the residue purified by flash chromatography (Pen/$Et_2O$ 6:1 to 2:1) to afford **(R)-19** (4.71 g, 27.35 mmol, 47%) as a colorless liquid and (S)-Acetate (6.04 g, 28.22 mmol, 48%).

**General Data:** $C_9H_{16}O_3$; FW: 172.11; $[\alpha]_D^{20} = +8.9$ ($c$ = 0.55, $CHCl_3$); TLC: Rf= 0.35 (Pentane/$Et_2O$ 2:1); UV (-); Vanillin: light blue.

**$^1$H-NMR** (600 MHz, $CDCl_3$): δ (ppm): 5.89-5.80 (m, 1H); 5.27 (dt, $J$ = 17.19 Hz, $J$ = 1.43 Hz, 1H); 5.11 (dt, $J$ = 10.53 Hz, $J$ = 1.29 Hz, 1H); 4.49-4.43 (m, 1H); 2.47 (dd, $J$ = 16.14 Hz, $J$ = 4.1 Hz, 1H); 2.47 (dd, $J$ = 16.02 Hz, $J$ = 8.35 Hz, 1H); 1.43 (s, 9H).

**$^{13}$C-NMR** (151 MHz, $CDCl_3$): δ (ppm): 171.64; 138.94; 115.05; 81.34; 68.98; 42.11; 28.05.

Example 19:

***tert*-Butyl (*R*)-3-Methoxypent-4-enoate (21)**

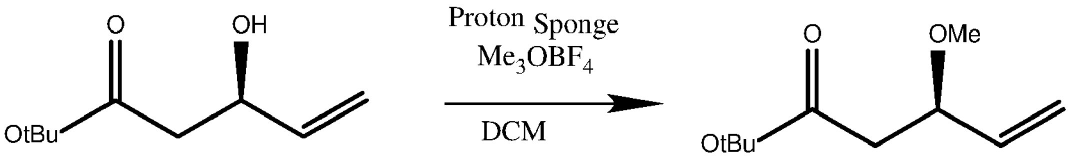


**19** **21**

Proton Sponge (17.5 g, 81.86 mmol, 3 eq) and trimethyl oxonium tetrafluoroborate (8.08 g, 54.58 mmol, 2 eq) were added to a solution of **(R)-19** (4.7 g, 27.29 mmol, 1 eq) in $CH_2Cl_2$ (115 mL) and the mixture was stirred for 3 h at room temperature. The reaction mixture was then filtered through a pad of Celite and the filtrate was washed with saturated aqueous solution of $NaHSO_4$ to remove Proton Sponge. The aqueous phase was extracted with $CH_2Cl_2$ and the organic extracts were dried over $Na_2SO_4$, filtered and concentrated in vacuo. The residue was purified by silica gel chromatography (Pen/$Et_2O$ 20:1, then 2:1) to give **21** (3.91 g, 21.03 mmol, 77%) as a colorless liquid and unreacted alcohol (517 mg, 3.00 mmol, 11%).

**General Data:** $C_{10}H_{18}O_3$; FW: 186.13; $[\alpha]_D^{20} = +1.1$ ($c$ = 0.55, $CHCl_3$); TLC: Rf= 0.30 (Pentane/$Et_2O$ 20:1); UV (-); Vanillin: dark blue.

**$^1$H-NMR** (600 MHz, $CDCl_3$): δ (ppm): 5.72-5.65 (m, 1H); 5.27 (dt, $J$ = 17.17 Hz, $J$ = 1.43 Hz, 1H); 5.21 (dt, $J$ = 10.53 Hz, $J$ = 1.29 Hz, 1H); 4.00-3.93 (m, 1H); 3.28 (s, 3H); 2.51 (dd, $J$ = 14.85 Hz, $J$ = 8.09 Hz, 1H); 2.36 (dd, $J$ = 14.75 Hz, $J$ =

5.68 Hz, 1H); 1.44 (s, 9H).
**$^{13}$C-NMR** (151 MHz, $CDCl_3$): δ (ppm): 170.16; 137.22; 117.65; 80.60; 79.40; 56.49; 42.06; 28.09.

Example 20:

***tert*-butyl (R)-3-methoxy-4-oxobutanoate (22)**

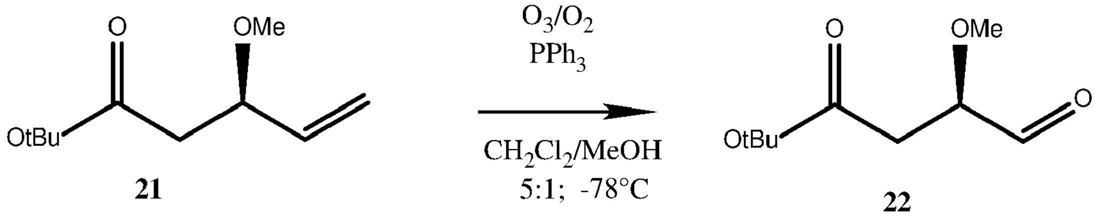


21 22

A stream of $O_3$ in $O_2$ was bubbled through a solution of **(R)-21** (3.23 g, 17.37 mmol, 1 eq) in $CH_2Cl_2$ (75 mL) and MeOH (15 mL) at -78°C until the blue color of the solution persisted. Then $O_2$ was bubbled for 10 min and $PPh_3$ (5.47 g, 20.84 mmol, 1.2 eq) was added. The mixture was warmed to room temperature and stirred for 2 h. The solvents were removed in vacuo and the residue was purified by flash chromatography (Pen/$Et_2O$ 2:1) to afford aldehyde **22** (2.91 g, 15.5 mmol, 89%) as a colorless liquid.

**General Data:** $C_9H_{16}O_4$; FW: 188.10; $[\alpha]_D^{20} = +30.08$ (*c* = 1.25, $CHCl_3$); TLC: Rf= 0.25 (Pentane/$Et_2O$ 2:1); UV (-); Vanillin: yellow.
**$^1$H-NMR** (600 MHz, $CDCl_3$): δ (ppm): 9.76 (s, 1H); 3.93-3.89 (m, 1H); 3.50 (s, 3H); 2.69 (dd, *J* = 16.06 Hz, *J* = 4.86 Hz, 1H); 2.60 (dd, *J* = 16.41 Hz, *J* = 6.68 Hz, 1H); 1.44 (s, 9H).
**$^{13}$C-NMR** (151 MHz, $CDCl_3$): δ (ppm): 202.38; 169.14; 82.06; 81.62; 58.65; 36.99; 28.01.

Example 21:

***tert*-Butyl (R,E)-3-methoxy-7-(triisopropylsilyl)hept-4-en-6-ynoate (24)**

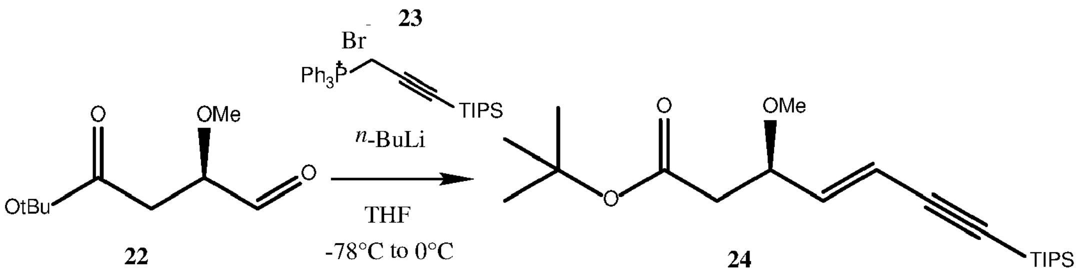


22 24

*n*BuLi 2.5 M in hexane (8 mL, 20.05 mmol, 1.3 eq) was added dropwise to a suspension of Phosphonium Bromide **23** (9.96 g, 18.5 mmol, 1.2 eq) in THF (100 mL) at -78°C. The red solution was stirred at 0°C for 30 min and then aldehyde **22** (2.9 g, 15.42 mmol, 1 eq) in THF (20 mL) was added dropwise. The mixture was warmed to room temperature and stirred for 30 min. The reaction was quenched by addition of saturated $NH_4Cl$ aqueous solution (100 mL) and the layers were separated. The aqueous phase was extracted with $Et_2O$ (3x80 mL) and the combined organic extracts were dried over $Na_2SO_4$, filtered and concentrated in vacuo. Purification of the residue by flash chromatography (Pen/$Et_2O$ 40:1 to 30:1) gave **E-24** (3.38 g, 9.25 mmol, 60%) and **Z-24** (1.25 g, 3.42 mmol, 22%) E/Z 2.7:1.

**General Data:** $C_{21}H_{38}O_3Si$; FW: 366.26; $[\alpha]_D^{20} = +8.2$ (*c* = 0.5, $CHCl_3$); TLC: Rf= 0.45 (Pentane/$Et_2O$ 20:1); UV (+); Vanillin: brown.

**$^{1}$H-NMR** (600 MHz, $CDCl_3$): δ (ppm): 6.01 (dd, *J* = 15.86 Hz, *J* = 7.29 Hz, 1H); 5.76 (dd, *J* = 15.87 Hz, *J* = 1.05 Hz, 1H); 4.04-3.98 (m, 1H); 3.30 (s, 3H); 2.50 (dd, *J* = 14.53 Hz, *J* = 7.94 Hz, 1H); 2.37 (dd, *J* = 14.98 Hz, *J* = 5.67 Hz, 1H); 1.44 (s, 9H); 1.07 (s, 20H).
**$^{13}$C-NMR** (151 MHz, $CDCl_3$): δ (ppm): 169.81; 142.32; 112.79; 104.44; 92.03; 80.86; 78.39; 56.98; 41.89; 28.06; 18.59; 11.24.

Example 22:

**(R,E)-3-methoxy-7-(triisopropylsilyl)hept-4-en-6-ynoic acid** (25)

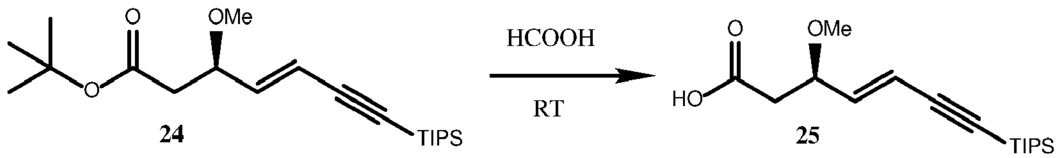


24 25

A solution of **24** (3.2 g, 8.74 mmol) in formic acid (13 mL) was stirred overnight at room temperature. The mixture was then concentrated in vacuo and azeotropically dried with toluene for 3 times to remove formic acid. Carbossilic acid **25** was obtained (2.68 g, 8.65 mmol, 99%) as a slightly yellow liquid.

**General Data:** $C_{17}H_{30}O_3Si$; FW: 310.20; $[\alpha]_D^{20} = +10.22$ (*c* = 0.45, $CHCl_3$); TLC: Rf= 0.3 (Pentane/$Et_2O$ 5:1); UV (+); Vanillin: brown.
**$^{1}$H-NMR** (600 MHz, $CDCl_3$): δ (ppm): 6.02 (dd, *J* = 15.86 Hz, *J* = 7.29 Hz, 1H); 5.76 (dd, *J* = 15.87 Hz, *J* = 1.05 Hz, 1H); 4.04-3.98 (m, 1H); 3.30 (s, 3H); 2.50 (dd, *J* = 14.53 Hz, *J* = 7.94 Hz, 1H); 2.37 (dd, *J* = 14.98 Hz, *J* = 5.67 Hz, 1H); 1.07 (s, 20H).
**$^{13}$C-NMR** (151 MHz, $CDCl_3$): δ (ppm): 175.91; 141.40; 113.50; 104.04; 92.69; 77.71; 57.03; 40.34; 18.60; 11.24.

Example 23:

**methyl ((R,E)-3-methoxy-7-(triisopropylsilyl)hept-4-en-6-ynoyl)-L-serinate (26)**

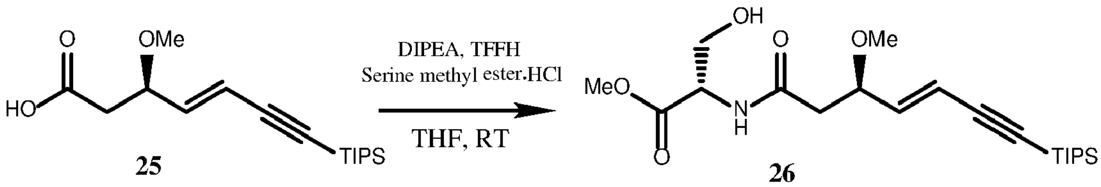


25 26

DIPEA (3.5 mL, 20.1 mmol, 2.3 eq) and TFFH (2.54 g, 9.61 mmol, 1.1 eq) were added to a solution of carboxylic acid **26** (2.7 g, 8.74 mmol, 1 eq) in THF (30 mL) and the mixture was stirred for 2 h at room temperature. L-serine methyl ester hydrochloride (1.63 g, 10.49 mmol, 1.2 eq) was added and the mixture was stirred for 3 h. $Et_2O$ (20mL) was added and the solution was washed with HCl 1 M (40 mL). The aqueous phase was extracted with $Et_2O$ (3x40 mL) and the organic layers were dried over $Na_2SO_4$, filtered and concentrated in vacuo. Purification of the residue by flash chromatography ($Et_2O$) afforded the serinate **26** (3.22 g, 7.82 mmol, 90%) as a yellow oil.

**General Data:** $C_{21}H_{37}NO_5Si$; FW: 411.24; $[\alpha]_D^{20} = +28.42$ (*c* = 1.2, $CHCl_3$); TLC: Rf= 0.25 ($Et_2O$); UV (+); Vanillin: brown.
**$^{1}$H-NMR** (600 MHz, $CDCl_3$): δ (ppm): 7.32-7.29 (br, 1H); 6.01 (dd, *J* = 15.79 Hz, *J* = 7.33 Hz, 1H); 5.79 (d, *J* = 16.01 Hz, 1H); 4.71-4.61 (m, 1H); 4.09-4.00 (m, 1H); 3.99-3.89 (m, 1H); 3.79 (s, 3H); 3.35 (s, 3H); 2.55 (dd, *J* = 15.45 Hz, *J* = 8.28 Hz, 1H); 2.46 (dd, *J* = 14.90 Hz, *J* = 3.31 Hz, 1H); 1.07 (s, 20H).
**$^{13}$C-NMR** (151 MHz, $CDCl_3$): δ (ppm): 170.79; 141.43; 113.26; 103.98; 92.85; 78.39; 63.24; 56.97; 54.87; 52.76; 41.98;

18.60; 11.23.

Example 24:

**Methyl 2-((R,E)-2-methoxy-6-(triisopropylsilyl)hex-3-en-5-yn-1-yl)-4,5-dihydrooxazole-4-carboxylate (27)**

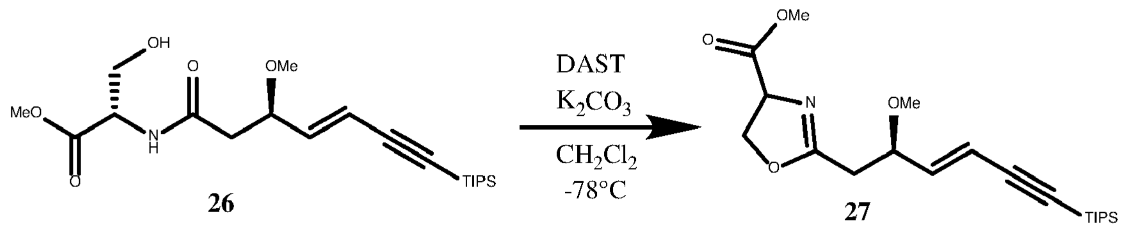


DAST (1.13 mL, 8.56 mmol, 1.1 eq) was added dropwise to a solution of serinate **26** (3.2 g, 7.78 mmol, 1 eq) in $CH_2Cl_2$ (60 mL) at -78°C and the mixture was stirred for 2 h at -78°C. $K_2CO_3$ (2.15 g, 15.56 mmol, 2 eq) was added and the mixture was warmed to room temperature and stirred for 1 h. A saturated solution of $NH_4Cl$ (50 mL) was carefully added and, after the gas evolution ceased, the layers were separated. The aqueous phase was extracted with $CH_2Cl_2$ (3x50 mL) and the combined organic extracts were washed with Brine, dried over $Na_2SO_4$, filtered and concentrated in vacuo to afford the crude product **27** as a yellow oil, which was used for the next step without further purification.

**General Data:** $C_{21}H_{35}NO_4Si$; FW: 393.23; $[\alpha]_D^{20} = +58.59$ (*c* = 0.85, $CHCl_3$); TLC: Rf= 0.4 ($Et_2O$); UV (+); Vanillin: brown.
**$^1$H-NMR** (600 MHz, $CDCl_3$): δ (ppm): 6.03 (dd, *J* = 15.83 Hz, *J* = 7.39 Hz, 1H); 5.77 (dd, *J* = 16.01 Hz, *J* = 0.965 Hz, 1H); 4.76-4.72 (m, 1H); 4.50 (dd, *J* = 9.26 Hz, *J* = 8.02 Hz, 1H); 4.38 (dd, *J* = 11.18 Hz, *J* = 8.69 Hz, 1H); 4.07-3.99 (m, 1H); 3.77 (s, 3H); 3.29 (s, 3H); 2.66 (dd, *J* = 14.63 Hz, *J* = 7.66 Hz, 1H); 2.49 (dd, *J* = 14.99 Hz, *J* = 5.87 Hz, 1H); 1.06 (s, 20H).
**$^{13}$C-NMR** (151 MHz, $CDCl_3$): δ (ppm): 171.49; 167.42; 141.96; 113.14; 104.28; 92.31; 78.41; 69.40; 68.10; 56.95; 52.64; 34.30; 18.60; 11.24.

Example 25:

**Methyl (R,E)-2-(2-methoxy-6-(triisopropylsilyl)hex-3-en-5-yn-1-yl)oxazole-4-carboxylate (28)**

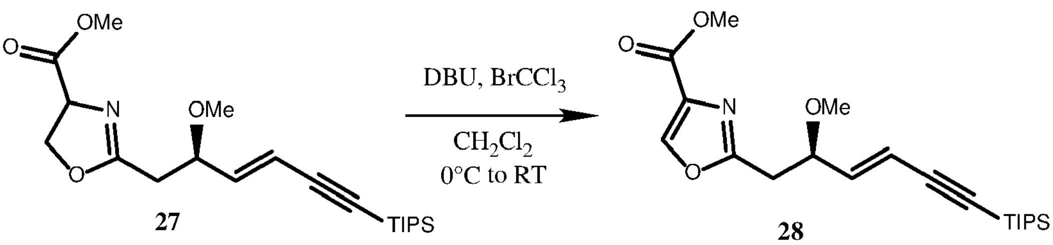


The crude material **27** was dissolved in $CH_2Cl_2$ (60 mL), cooled to 0°C and protected from light with aluminium foil. DBU (2.24 mL, 15.56 mmol, 2 eq) and $BrCCl_3$ (1.53 mL, 15.56 mmol, 2 eq) were sequentially added dropwise, then the bath was removed and the mixture was stirred at room temperature for 16 h. The reaction was quenched with saturated aqueous $NH_4Cl$ solution (100 mL) and the layers were separated. The aqueous phase was extracted with $CH_2Cl_2$ (3x100 mL) and the organic layers were dried over $Na_2SO_4$, filtered and concentrated in vacuo. Purification of the residue by flash chromatography (Pen/$Et_2O$ 2:1) afforded oxazole **28** (1.77 g, 4.51 mmol, 58% from **27**) as a yellow oil.

**General Data:** $C_{21}H_{33}NO_4Si$; FW: 391.22; $[\alpha]_D^{20} = -17.4$ (*c* = 0.5, $CHCl_3$); TLC: Rf= 0.35 (Pentane/$Et_2O$ 2:1); UV

(+); Vanillin: brown.

**$^1$H-NMR** (600 MHz, $CDCl_3$): δ (ppm): 8.16 (s, 1H); 6.04 (dd, *J* = 16.10 Hz, *J* = 7.51 Hz, 1H); 5.77 (dd, *J* = 15.93 Hz, *J* = 0.991 Hz, 1H); 4.18-4.11 (m, 1H); 3.90 (s, 3H); 3.26 (s, 3H); 3.08 (dd, *J* = 15.63 Hz, *J* = 8.13 Hz, 1H); 2.99 (dd, *J* = 15.05 Hz, *J* = 5.31 Hz, 1H); 1.07 (s, 20H).

**$^{13}$C-NMR** (151 MHz, $CDCl_3$): δ (ppm): 162.47; 161.68; 143.99; 141.58; 133.35; 113.72; 103.98; 92.81; 79.14; 56.97; 52.16; 34.51; 18.59; 11.22.

Example 26:

**Methyl (R,E)-2-(2-methoxyhex-3-en-5-yn-1-yl)oxazole-4-carboxylate (29)**

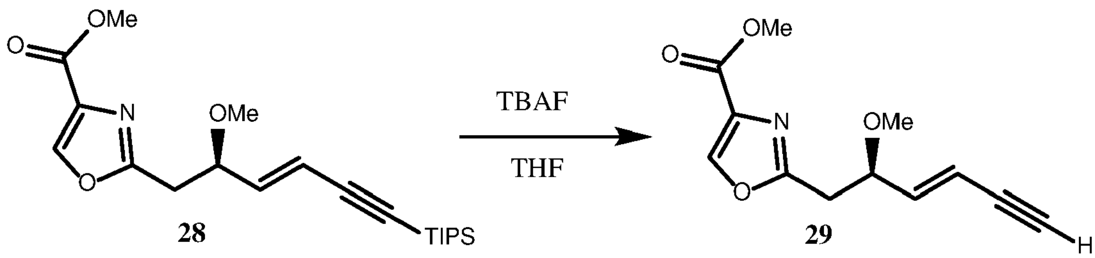


28 29

TBAF (1 M in THF, 4.6 mL, 4.6 mmol, 1.2 eq) was added dropwise at 0°C to a solution of TIPS oxazole **28** (1.5 g, 3.83 mmol, 1 eq) in THF (10 mL). The mixture was stirred for 1 h at room temperature and then quenched with water. The aqueous phase was extracted with $Et_2O$ and the organic layers were dried over $Na_2SO_4$, filtered and concentrated in vacuo. Purification of the residue by flash chromatography (Pen/$Et_2O$ 2:1 to 1:1) afforded oxazole 29 (675 mg, 2.87 mmol, 75%) as a yellow oil.

**General Data:** $C_{12}H_{13}NO_4$; FW: 235.08; $[\alpha]_D^{20}$ = -26.89 (*c* = 0.45, $CHCl_3$); TLC: Rf= 0.30 (Pentane/$Et_2O$ 1:1); UV (+); Vanillin: brown.

**$^1$H-NMR** (600 MHz, $CDCl_3$): δ (ppm): 8.16 (s, 1H); 6.10 (ddd, *J* = 16.19 Hz, *J* = 7.42 Hz, *J* = 0.434 Hz, 1H); 5.70 (ddd, *J* = 16.01 Hz, *J* = 2.15 Hz, *J* = 0.908 Hz, 1H); 4.19-4.12 (m, 1H); 3.89 (s, 3H); 3.26 (s, 3H); 3.08 (dd, J= 14.47 Hz, *J* = 7.89 Hz, 1H); 2.98 (dd, *J* = 14.74 Hz, *J* = 5.79 Hz, 1H); 2.92 (d, *J* = 2.30 Hz, 1H).

**$^{13}$C-NMR** (151 MHz, $CDCl_3$): δ (ppm): 162.24; 161.59; 144.00; 142.99; 133.34; 112.29; 80.80; 78.91; 78.89; 56.95; 52.13; 34.35.

Example 27:

**(R,E)-2-(2-methoxyhex-3-en-5-yn-1-yl)oxazole-4-carboxylic acid (30)**

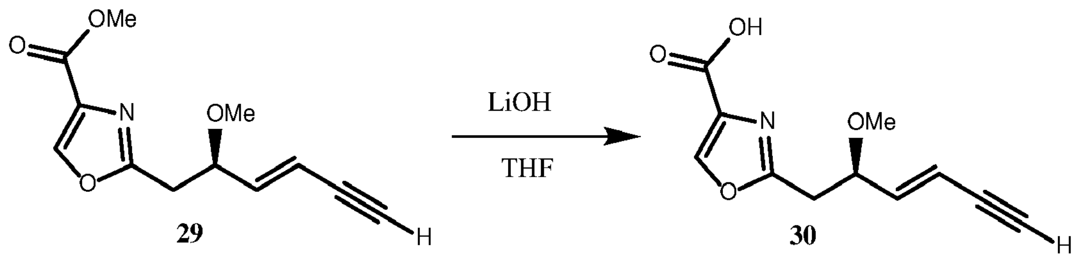


29 30

LiOH (1 M in $H_2O$, 3.9 mL, 3.9 mmol, 2.5 eq) was added to a solution of the oxazole **29** (367 mg, 1.56 mmol, 1 eq) in THF (1 mL) at room temperature. The mixture was stirred for 2 h and then quenched with 1 M HCl. The aqueous phase was extracted with $Et_2O$ and the organic layers were dried over $Na_2SO_4$, filtered and concentrated in vacuo to

afford the acid **30** (326 mg, 1.54 mmol, 99%) as a yellow solid.

**General Data:** $C_{11}H_{11}NO_4$; FW: 221.07; $[\alpha]_D^{20}$ = -26.4 (*c* = 0.5, $CHCl_3$); UV (+); Vanillin: brown.

**$^1$H-NMR** (600 MHz, $CDCl_3$): δ (ppm): 8.25 (s, 1H); 6.13 (ddd, *J* = 15.97 Hz, *J* = 7.36 Hz, *J* = 0.566 Hz, 1H); 5.73 (ddd, *J* = 16.02 Hz, *J* = 2.38 Hz, *J* = 0.977 Hz, 1H); 4.23-4.15 (m, 1H); 3.27 (s, 3H); 3.14 (dd, *J* = 15.14 Hz, *J* = 7.66 Hz, 1H); 3.05 (dd, *J* = 15.02 Hz, *J* = 5.57 Hz, 1H); 2.93 (d, *J* = 3.31 Hz, 1H).

**$^{13}$C-NMR** (151 MHz, $CDCl_3$): δ (ppm): 164.80; 162.73; 145.01; 142.88; 132.94; 112.43; 80.81; 78.96; 78.90; 56.98; 52.13; 34.22.

Example 28:

**Methyl 2-((2R,3E,7Z,10S,12S,13E)-10-hydroxy-2-methoxy-12-(methoxymethoxy)-11,11-dimethylpentadeca-3,7,13-trien-5-yn-1-yl)oxazole-4-carboxylate (30)**

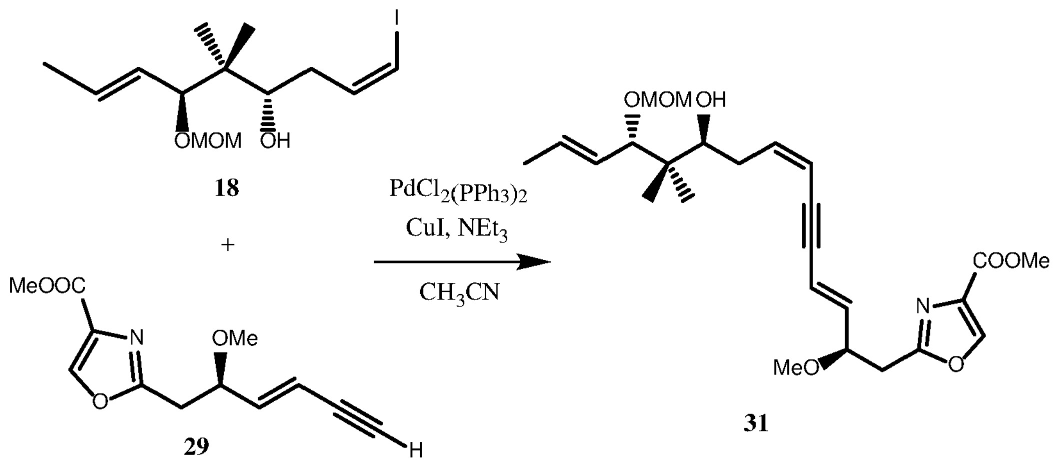


18 + 29 → 31

The vinyl iodide **18** (300 mg, 0.847 mmol, 1 eq) was dissolved in degassed $CH_3CN$ (5 mL) and CuI (39 mg, 0.254 mmol, 0.3 eq) and $PdCl_2(PPh_3)_2$ (60 mg, 0.0847 mmol, 0.1 eq) were added. The mixture was degassed by freeze-pump-thaw (2 cycles) and then cooled to -20°C. $NEt_3$ (0.706 mL, 5.08 mmol, 6 eq) was added, followed by a slow addition of the enyne **29** (240 mg, 1.02 mmol, 1.2 eq) in degassed $CH_3CN$ (3 mL). The solution became red and after 15 min the bath was removed. The mixture was stirred for 1 h at room temperature and quenched with $NH_4Cl$ solution. The aqueous phase was extracted with $Et_2O$ and the combined organic extracts were dried over $Na_2SO_4$, filtered and concentrated in vacuo. The residue was purified by flash chromatography ($Et_2O$/Pen 2:1) to give the monomer **31** (360 mg, 0.781 mmol, 92 %) as a yellow oil.

**General Data:** $C_{25}H_{35}NO_7$; FW: 461.24; TLC: Rf= 0.25 ($Et_2O$/Pentane 2:1); UV (+); Vanillin: black.

**$^1$H-NMR** (600 MHz, $CDCl_3$) δ (ppm): 8.16 (s, 1H); 6.19-6.08 (m, 1H); 5.96 (dd, *J* = 8.56 Hz, *J* = 7.30 Hz, 1H); 5.82 (dd, *J* = 15.9 Hz, *J* = 2.07 Hz, 1H); 5.73-5.61 (m, 2H); 5.33 (qd, *J* = 6.42 Hz, *J* = 1.71 Hz, 1H); 4.71 (d, *J* = 6.67 Hz, 1H); 4.46 (d, *J* = 6.67 Hz, 1H); 4.16 (q, *J* = 7.48 Hz, 1H); 3.90 (s, 3H); 3.89 (app dd, *J* = 8.46 Hz, *J* = 4.55 Hz, 1H); 3.62 (dd, *J* = 10.16, *J* = 2.80, 1H); 3.37 (s, 3H); 3.26 (s, 3H); 3.10 (dd, *J* = 7.75 Hz, *J* = 6.97 Hz, 1H); 2.99 (dd, *J* = 9.30 Hz, *J* = 5.62 Hz, 1H); 2.61-2.50 (m, 1H); 1.65 (s, 1H); 2.48-2.32 (m, 1H); 1.74 (dd, *J* = 6.50 Hz, *J* = 1.70 Hz, 3H); 0.976 (s, 3H); 0.809 (s, 3H). **$^{13}$C-NMR** (151 MHz, $CDCl_3$): δ (ppm): 162.41; 161.63; 143.99; 142.54; 140.27; 133.33; 132.38; 126.93; 113.78; 110.11; 93.04; 91.10; 88.02; 84.69; 79.22; 77.87; 56.83; 55.99; 52.15; 41.08; 34.55; 32.88; 21.39; 17.87; 16.02.

Example 29:

**(2E,4S,6S,8Z,12E,14R)-14-methoxy-15-(4-(methoxycarbonyl)oxazol-2-yl)-4-(methoxymethoxy)-5,5-dimethylpentadeca-2,8,12-trien-10-yn-6-yl 2-((R,E)-2-methoxyhex-3-en-5-yn-1-yl)oxazole-4-carboxylate (32)**

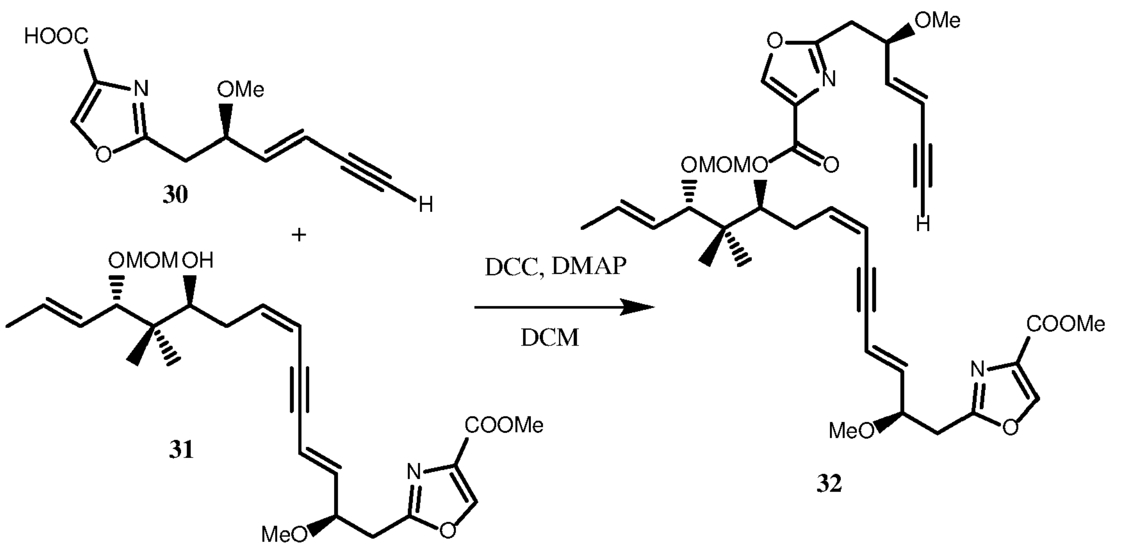


30 + 31 → 32

The monomer **31** (320 mg, 0.694 mmol, 1 eq) and the acid **30** (291 mg, 1.32 mmol, 1.5 eq) and DMAP (85 mg, 0.694 mmol, 1 eq) were dissolved in $CH_2Cl_2$ (10 mL) and cooled to 0°C. DCC (716 mg, 3.47 mmol, 5 eq) was added and the mixture was stirred overnight at room temperature. The solution was directly chromatographed ($CH_2Cl_2$/MeOH 99:1) to afford ester 32 (460 mg, 0.692 mmol, 99%) as a yellow wax, containing DCU impurity.

**General Data:** $C_{36}H_{44}N_2O_{10}$; FW: 664.30; $[\alpha]_D^{20} = +23.16$ (*c* = 0.6, $CHCl_3$); TLC: Rf= 0.25 ($CH_2Cl_2$/MeOH 99:1); UV (+); Vanillin: black.

**$^1$H-NMR** (600 MHz, $CDCl_3$): δ (ppm): 8.17 (s, 1H); 8.06 (s, 1H); 6.13 (dd, *J* = 16.38 Hz, *J* = 7.39 Hz, 1H); 5.98 (dd, *J* = 15.5 Hz, *J* = 7.39 Hz, 1H); 5.98-5.92 (m, 1H); 5.87 (dd, *J* = 15.92 Hz, *J* = 2.03 Hz, 1H); 5.73 (ddd, *J* = 16.25 Hz, *J* = 2.33 Hz, *J* = 1.09 Hz, 1H); 5.63-5.53 (m, 2H); 5.34 (app dd, *J* = 9.86 Hz, *J* = 3.28 Hz, 1H); 5.31 (app qd, *J* = 7.63 Hz, *J* = 1.74 Hz, 1H); 4.68 (d, *J* = 6.78 Hz, 1H); 4.45 (d, *J* = 6.68 Hz, 1H); 4.18 (q, *J* = 6.06 Hz, 2H); 3.90 (s, 3H); 3.82 (d, *J* = 8.69 Hz, 1H); 3.62 (dd, *J* = 10.16 Hz, *J* = 2.80 Hz, 1H); 3.37 (s, 3H); 3.28 (s, 6H); 3.14-3.05 (m, 2H); 3.03-2.96 (m, 2H); 2.92 (d, *J* = 2.15 Hz, 1H); 2.79-2.64 (m, 2H); 1.72 (dd, *J* = 6.59 Hz, *J* = 1.46 Hz, 3H); 1.02 (s, 3H); 0.948 (s, 3H).

**$^{13}$C-NMR** (151 MHz, $CDCl_3$): δ (ppm): 162.37; 162.25; 161.63; 160.62; 144.03; 143.53; 143.13; 140.49; 140.40; 133.45; 133.38; 132.19; 126.76; 113.61; 112.20; 111.11; 93.35; 91.26; 87.96; 81.37; 80.91; 79.20; 78.87; 78.83; 56.98; 55.75; 52.14; 41.61; 34.55; 34.37; 31.08; 25.45; 19.93; 19.46; 17.92.

Example 30:

**(2E,4S,6S,8Z,12E,14R)-14-methoxy-15-(4-(methoxycarbonyl)oxazol-2-yl)-4-(methoxymethoxy)-5,5-dimethylpentadeca-2,8,12-trien-10-yn-6-yl 2-((2R,3E,7Z,10S,12S,13E)-10-hydroxy-2-methoxy-12-(methoxymethoxy)-11,11-dimethylpentadeca-3,7,13-trien-5-yn-1-yl)oxazole-4-carboxylate (33)**

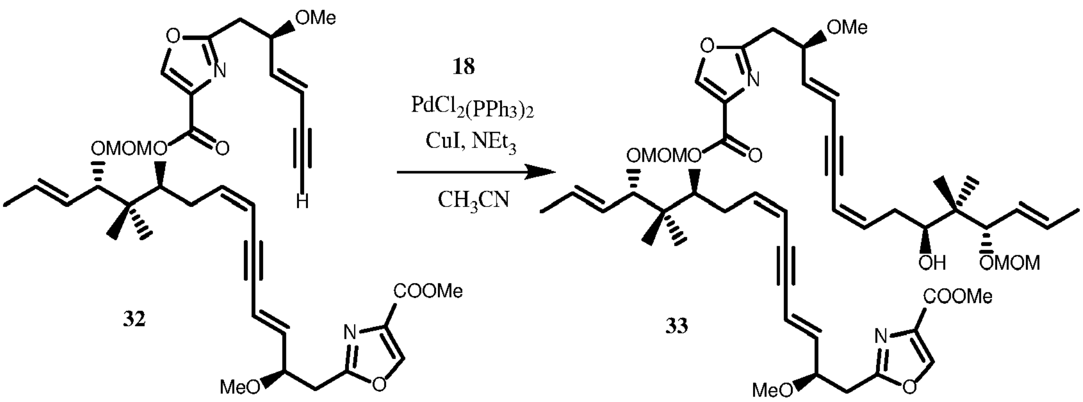


32

33

The vinyl iodide **18** (120 mg, 0.339 mmol, 1 eq) was dissolved in degassed $CH_3CN$ (5 mL) and CuI (15 mg, 0.102 mmol, 0.3 eq) and $PdCl_2(PPh_3)_2$ (24 mg, 0.0339 mmol, 0.1 eq) were added. The mixture was degassed by freeze-pump-thaw (2 cycles) and then cooled to -20°C. $NEt_3$ (0.283 mL, 2.03 mmol, 6 eq) was added, followed by a slow addition of the enyne **32** (300 mg, 0.452 mmol, 1.33 eq) in degassed $CH_3CN$ (3 mL). The solution became red and after 30 min the bath was removed. The mixture was stirred for 1 h at room temperature and quenched with $NH_4Cl$ solution. The aqueous phase was extracted with $Et_2O$ and the combined organic extracts were dried over $Na_2SO_4$, filtered and concentrated in vacuo. The residue was purified by flash chromatography ($CH_2Cl_2$/MeOH 99:1) to give **33** (246 mg, 0.276 mmol, 82%) as a yellow oil.

**General Data:** $C_{49}H_{66}N_2O_{13}$; FW: 890.46; $[\alpha]_D^{20}$ = -22.83 (*c* = 0.6, $CHCl_3$); TLC: Rf= 0.20 ($CH_2Cl_2$/MeOH 99:1); UV (+); Vanillin: black.

**$^1$H-NMR** (600 MHz, $CDCl_3$): δ (ppm): 8.17 (s, 1H); 8.05 (s, 1H); 6.19-6.09 (m, 1H); 6.03-5.83 (m, 5H); 5.70-5.51 (m, 4H); 5.37-5.25 (m, 3H); 4.70 (dd, *J* = 15.62 Hz, *J* = 6.57 Hz, 1H); 4.45 (t, *J* = 6.86 Hz, 1H); 4.22-4.13 (m, 2H); 3.90 (s, 3H); 3.89 (d, *J* = 9.06 Hz, 1H); 3.82 (d, *J* = 8.55 Hz, 1H); 3.56 (dt, *J* = 10.20 Hz, *J* = 3.26 Hz, 1H); 3.37 (s, 6H); 3.28 (s, 3H); 3.27 (s, 3H); 3.15-2.96 (m, 4H); 2.78-2.64 (m, 2H); 2.61-2.49 (m, 1H); 2.47-2.32 (m, 1H); 1.74 (dd, *J* = 6.43 Hz, *J* = 1.58 Hz, 3H); 1.72 (dd, *J* = 6.49 Hz, *J* = 1.40 Hz, 3H); 1.02 (s, 3H); 0.977 (s, 3H); 0.944 (s, 3H); 0.813 (s, 3H).

**$^{13}$C-NMR** (151 MHz, $CDCl_3$): δ (ppm): 162.45; 162.37; 161.62; 160.63; 144.03; 143.51; 142.52; 140.49; 140.42; 133.41; 133.36; 132.39; 132.19; 126.93; 126.76; 125.51; 113.67; 113.61; 111.09; 110.13; 93.34; 93.05; 91.27; 91.16; 87.99; 87.96; 84.71; 81.36; 79.20; 77.85; 77.23; 56.87; 56.85; 56.01; 52.15; 41.63; 41.07; 34.50; 34.39; 32.87; 31.13; 21.41; 19.88; 19.36; 17.93; 17.86; 16.02.

Example 31:

**2-((2R,3E,7Z,10S,12S,13E)-10-((2-((2R,3E,7Z,10S,12S,13E)-10-hydroxy-2-methoxy-12-(methoxymethoxy)-11,11-dimethylpentadeca-3,7,13-trien-5-yn-1-yl)oxazole-4-carbonyl)oxy)-2-methoxy-12-(methoxymethoxy)-11,11-dimethylpentadeca-3,7,13-trien-5-yn-1-yl)oxazole-4-carboxylic acid (34)**

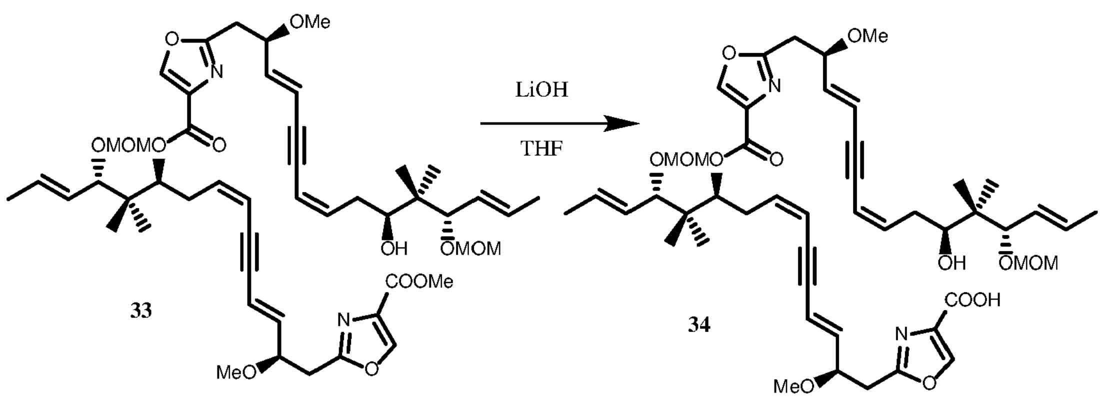


33

34

**33** (48 mg, 0.0539 mmol, 1 eq) was dissolved in THF (1 mL) and treated at room temperature with LiOH (1 M in $H_2O$, 0.108 mL, 0.108 mmol, 2 eq). The mixture was stirred overnight at room temperature and neutralized with 1 M HCl. The aqueous phase was extracted with $Et_2O$ and the organic extracts were dried over $Na_2SO_4$, filtered and concentrated in vacuo to give *seco*-acid **34** (44 mg, 0.0502 mmol, 93%) as a yellow wax, which was used without further purification.

**General Data:** $C_{48}H_{64}N_2O_{13}$; FW: 876.44; $[\alpha]_D^{20}$ = -26.4 (*c* = 0.5, $CHCl_3$); UV (+); Vanillin: grey.
**$^1$H-NMR** (600 MHz, $CDCl_3$): δ (ppm): 8.20 (s, 1H); 8.10 (s, 1H); 6.18-6.09 (m, 1H); 6.04-5.80 (m, 5H); 5.73-5.52 (m, 4H); 5.38-5.26 (m, 3H); 4.71 (dd, *J* = 13.08 Hz, *J* = 6.64 Hz, 1H); 4.46 (dd, *J* = 7.04 Hz, *J* = 4.02 Hz, 1H); 4.24-4.13 (m, 2H); 3.90 (d, *J* = 9.19 Hz, 1H); 3.83 (d, *J* = 8.68 Hz, 1H); 3.64 (dd, *J* = 10.12 Hz, *J* = 2.74 Hz, 1H); 3.39 (s, 3H); 3.38 (s, 3H); 3.30 (s, 3H); 3.28 (s, 3H); 3.18-2.94 (m, 4H); 2.82-2.51 (m, 3H); 2.47-2.33 (m, 1H); 1.74 (dd, *J* = 6.48 Hz, *J* = 1.52 Hz, 3H); 1.71 (dd, *J* = 6.38 Hz, *J* = 1.16 Hz, 3H); 1.03 (s, 3H); 0.983 (s, 3H); 0.950 (s, 3H); 0.815 (s, 3H).
**$^{13}$C-NMR** (151 MHz, $CDCl_3$): δ (ppm): 162.42; 162.36; 162.10; 160.61; 144.36; 143.74; 142.44; 140.44; 140.35; 133.46; 133.34; 132.47; 132.22; 126.86; 126.70; 125.51; 113.72; 113.64; 111.25; 110.20; 93.39; 93.03; 91.24; 91.20; 88.00; 87.92; 84.77; 81.54; 79.14; 77.98; 77.22; 56.85; 56.02; 55.99; 49.54; 41.63; 41.07; 34.59; 34.54; 33.94; 31.06; 21.39; 19.92; 19.45; 17.93; 17.86; 16.02.

Example 32:

**(16,16')-Bis(methoxymethyl)-(9,10,9',10')-tetradehydridodisorazole C1 (35)**

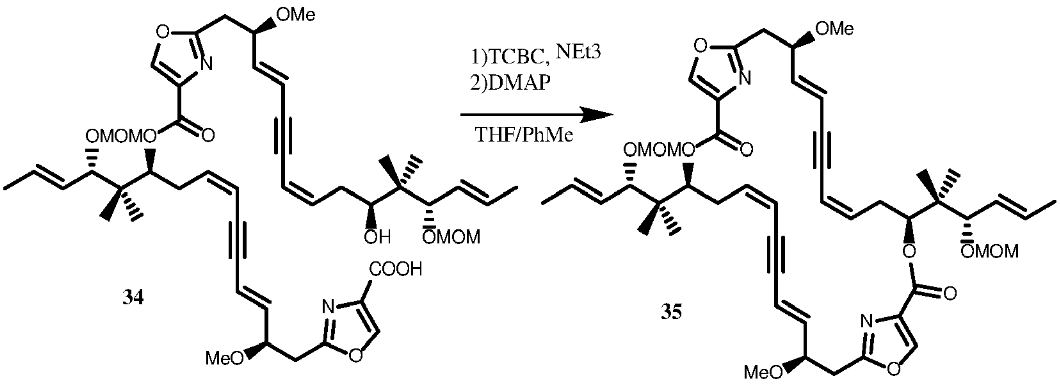


The crude *seco*-acid **34** (28 mg, 0.0319 mmol, 1 eq) was dissolved in THF (2.5 mL) and $NEt_3$ (89 μL, 0.638 mmol, 20 eq) and TCBC (50 μL, 0.319 mmol, 10 eq) were added at room temperature. The turbid solution was stirred for 2 h at room temperature and then diluted with toluene (1.5 mL) and added dropwise to a solution of DMAP (156 mg, 1.28 mmol, 40 eq) in toluene (45 mL). The mixture was stirred overnight at room temperature and then quenched with $NH_4Cl$ solution (10 mL) and water (10 mL) and the aqueous phase was extracted with EtOAc. The organic layers were dried over $Na_2SO_4$, filtered and concentrated in vacuo. The residue was purified by flash chromatography ($CH_2Cl_2$/MeOH 99:1) to afford the macrocycle 35 (22 mg, 0.0255, 80%) as a colorless wax.
**General Data:** $C_{48}H_{62}N_2O_{12}$; FW: 858.43; UV (+); TLC: Rf= 0.55 ($CH_2Cl_2$/MeOH 50:1 Vanillin: black.
**$^1$H-NMR** (600 MHz, $CDCl_3$): δ (ppm): 8.04 (s, 2H); 6.03-5.88 (m, 4H); 5.70-5.47 (m, 6H); 5.44-5.23 (m, 4H); 4.69 (d, *J* = 7.18 Hz, 1H); 4.45 (d, *J* = 6.68 Hz, 1H); 4.17-3.99 (m, 2H); 3.81 (d, *J* = 9.36 Hz, 2H); 3.38 (s, 6H); 3.36 (s, 6H); 3.32-3.25 (m, 2H); 3.07-2.83 (m, 4H); 2.53-2.43 (m, 2H); 1.73 (dd, *J* = 6.48 Hz, *J* = 1.52 Hz, 6H); 1.71 (dd, *J* = 6.36 Hz, *J* = 1.27 Hz, 3H); 1.03 (s, 6H); 0.962 (s, 6H).
**$^{13}$C-NMR** (151 MHz, $CDCl_3$): δ (ppm): 161.64; 160.52; 144.36; 143.33; 141.14; 140.31; 133.60; 132.38; 126.65; 125.51; 113.51; 112.08; 93.37; 90.80; 87.75; 81.47; 79.56; 77.22; 56.85; 56.07; 41.43; 33.96; 25.59; 19.84; 19.29; 17.94.

Example 33:

**(16,16')-Bis(methoxymethyl)-disorazole C1 (36)**

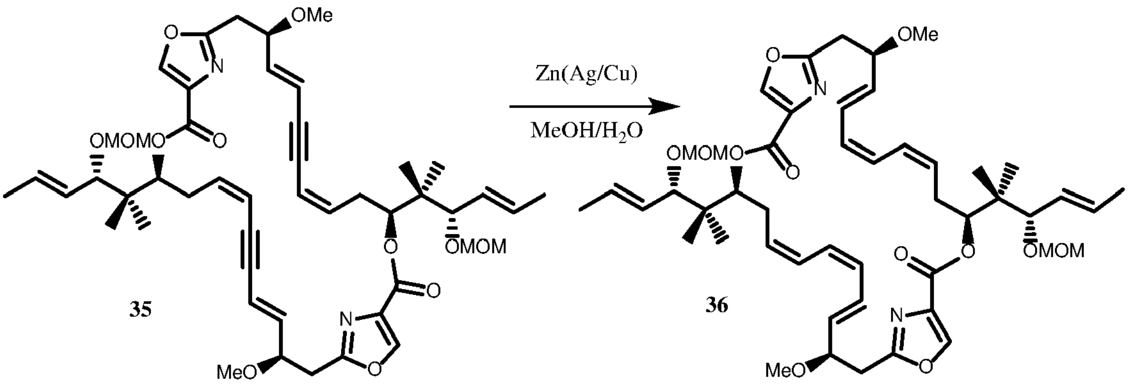


35 36

Nitrogen is bubbled for 15 min through a suspension of Zinc (3 g, 45.88 mmol) in $H_2O$ (18 mL) and then $Cu(OAc)_2 \cdot H_2O$ (300 mg, 1.50 mmol) was added at room temperature and after 15 min $AgNO_3$ (300 mg, 1.77 mmol) was added (exothermic reaction). The mixture was stirred for 30 min at room temperature, filtered by suction and washed with $H_2O$ (30 mL), MeOH (20 mL), acetone (20 mL) and $Et_2O$ (20 mL). This activated zinc solids were added to a solution of **35** (20 mg, 0.0233 mmol) in MeOH/$H_2O$ 1:1 (5 mL). The mixture was stirred overnight at 50°C and then filtered on a pad of silica and washed with MeOH. The filtrate was concentrated in vacuo and the residue was purified by flash chromatography ($CH_2Cl_2$/MeOH 60:1) to afford **36** (12 mg, 0.0139, 60%) as a colorless oil.
**General Data:** $C_{48}H_{66}N_2O_{12}$; FW: 862.46; UV (+); TLC: Rf= 0.40 ($CH_2Cl_2$/MeOH 50:1) Vanillin: dark blue.
**$^1$H-NMR** (600 MHz, $CDCl_3$): δ (ppm): 7.89 (s, 2H); 6.44 (dd, *J* = 14.93 Hz, *J* = 11.38 Hz, 2H); 6.34 (t, *J* = 11.38 Hz, 2H); 6.21 (t, J= 11.64 Hz, 2H); 5.90 (t, *J* = 11.04 Hz, 2H); 5.63-5.58 (m, 2H); 5.56-5.51 (m, 2H); 5.38 (dd, *J* = 10.91 Hz, *J* = 2.48 Hz, 1H); 5.34-5.27 (m, 4H); 4.68 (d, *J* = 6.67 Hz, 1H); 4.43 (d, *J* = 6.67 Hz, 1H); 4.15-4.10 (m, 2H); 3.81 (d, *J* = 8.73 Hz, 2H); 3.36 (s, 6H); 3.26 (s, 6H); 3.12 (dd, *J* = 14.66 Hz, *J* = 6.03 Hz, 2H); 2.97-2.86 (m, 2H); 2.78 (dd, *J* = 14.83 Hz, *J* = 7.27 Hz, 2H); 2.63-2.50 (m, 2H); 1.73 (dd, *J* = 6.48 Hz, *J* = 1.52 Hz, 6H); 1.73 (dd, *J* = 6.59 Hz, *J* = 1.22 Hz, 3H); 0.984 (s, 6H); 0.919 (s, 6H).
**$^{13}$C-NMR** (151 MHz, $CDCl_3$): δ (ppm): 162.23; 160.59; 143.20; 133.34; 133.03; 132.28; 130.13; 128.98; 127.95; 126.73; 125.59; 125.55; 93.26; 81.30; 79.79; 56.55; 56.13; 41.44; 35.06; 29.70; 19.95; 19.45; 17.94.

Example 34:

**Disorazole C1 (37)**

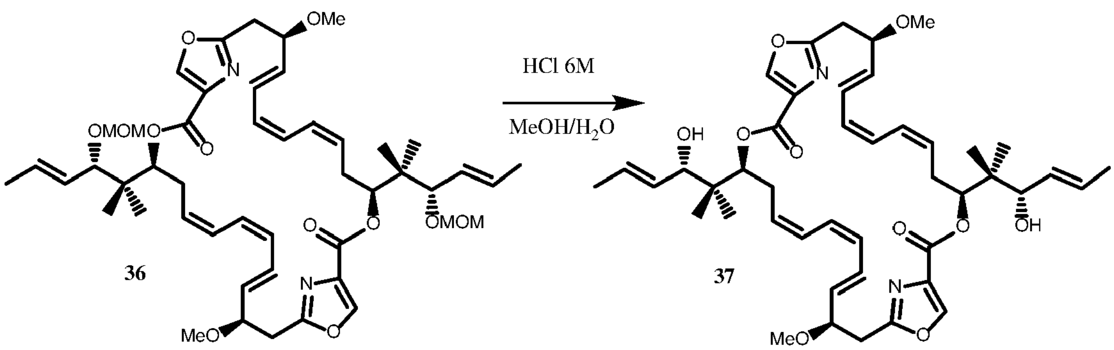


36 37

MOM protected disorazole $C_1$ **36** (1 mg, 1.16 μmol) was dissolved in MeOH (0.3 mL) and cooled to 0°C. HCl 6 M (0.6 mL) was added dropwise and then the mixture was stirred for 4 days at 0°C. The reaction was quenched with

saturated aqueous $NaHCO_3$ solution and the aqueous phase was extracted with $Et_2O$. The organic extracts were dried over $MgSO_4$, filtered and concentrated in vacuo. The residue was purified by flash chromatography ($CH_2Cl_2$/MeOH 50:1) to give Disorazole $C_1$ **37(0.6** mg, 0.7 μmol, 60%) as a colorless wax.

**General Data:** $C_{44}H_{58}N_2O_{10}$; FW: 774.41; UV (+); TLC: Rf= 0.20 ($CH_2Cl_2$/MeOH 50:1) Vanillin: dark blue.

**$^1$H-NMR** (600 MHz, $CD_3OD$): δ (ppm): δ 8.23 (s, 2 H); 6.50 (dd, *J* = 15.2 Hz, *J* = 11.5 Hz, 2H); 6.40 (app t, *J* = 11.2 Hz, 2 H); 6.28 (dd, 2 H, *J*= 11.4 Hz, *J* = 11.1 Hz, 2 H); 5.91 (dd, *J* = 11.2 Hz, *J* = 10.9 Hz, 2 H); 5.66 (dq, *J* = 15.2 Hz, *J* = 6.6 Hz, 2 H); 5.57 (ddd, *J* = 15.2 Hz, *J* = 7.8 Hz, *J* = 1.4 Hz, 2H); 5.54 (dd, *J* = 15.0 Hz, *J* = 8.3 Hz, 2 H); 5.48 (app dt, *J* = 10.0 Hz, *J* = 6.7 Hz, 2 H); 5.25 (dd, *J* = 11.3 Hz, *J* = 2.2 Hz, 2 H); 4.13 (ddd, *J* = 7.8 Hz, 7.2 Hz, 5.5 Hz, 2 H); 3.84 (d, 2 H, *J* = 7.8 Hz); 3.21 (s, 6 H), 2.99 (dd, 2 H, *J* = 15.5, *J* = 7.4 Hz); 2.76 (dd, *J* = 15.5 Hz, *J* = 5.4 Hz, 2 H); 2.69 (ddd, *J* = 13.8 Hz, *J* = 10.9 Hz, *J* = 10.2 Hz, 2 H); 2.38 (dd, *J* = 13.8 Hz, *J* = 6.1 Hz, 2 H); 1.69 (dd, *J* = 6.4 Hz, *J* = 1.3 Hz, 6 H); 1.00 (s, 6 H); 0.95 (s, 6 H).

**$^{13}$C-NMR** (151 MHz, $CD_3OD$) δ 164.12; 162.26; 145.83; 134.15; 134.09; 131.68; 130.88; 129.96; 129.63; 129.30; 127.36; 126.79; 80.57; 78.75; 77.84; 56.83; 42.70; 35.97; 29.24; 19.41; 19.32; 18.03;

## Claims

1. Compound of the formula III

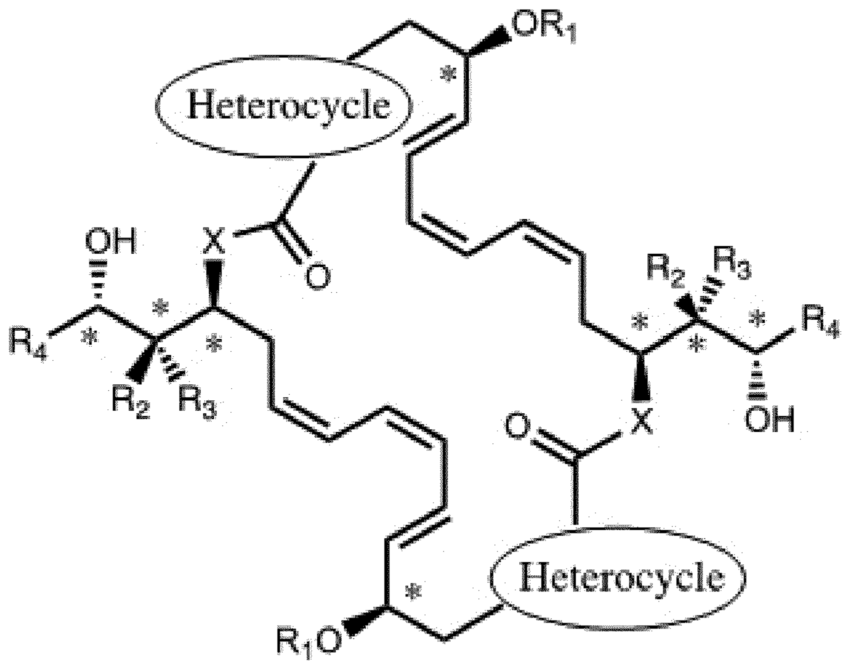

III

wherein:

X is O, S, or NR;
R is H, or $alkyl_{(C\leq8)}$;
$R_1$ is H, $alkyl_{(C\leq8)}$, or $cycloalkyl_{(C\leq8)}$;
$R_2$ and $R_3$ are each independently H, $alkyl_{(C\leq8)}$, $(CH_2)_n$ (n = 1-5);
R4 is independently H, $alkyl_{(C\leq8)}$, $cycloalkyl_{(C\leq8)}$, $alkenyl_{(C\leq8)}$, $alkinyl_{(C\leq8)}$, allyl, propargyl, phenyl, or benzyl;
Heterocycle is independently oxazole, thiazol, imidazole, furyl, pyrrolyl, thiophenyl, pyridyl, or phenyl;
* indicate the stereoisomeric centers of the compound;

as well as the individual stereoisomers of this compound and/or a pharmaceutically acceptable salt thereof.

2. Method for the production of a compound of formula III according to claim 1

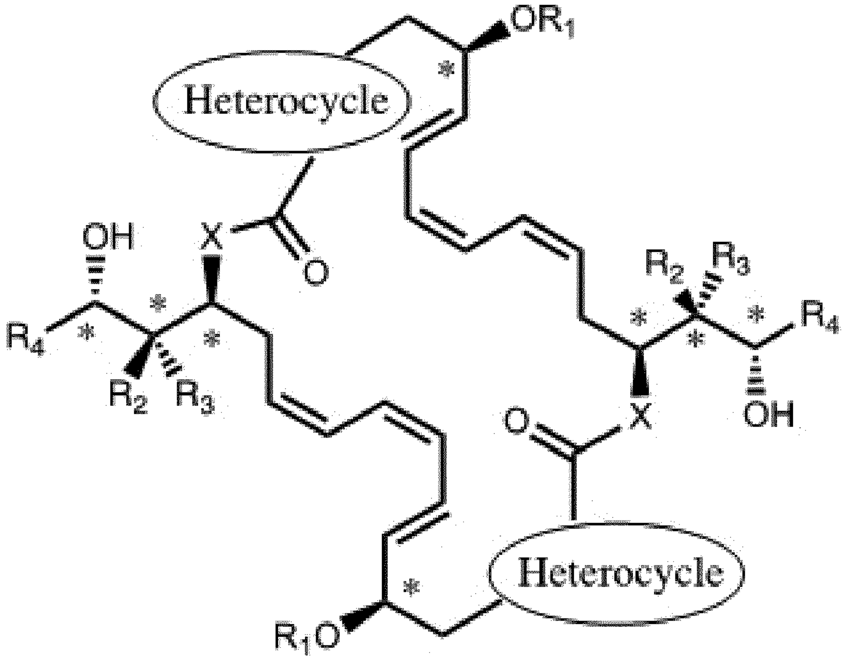

(III)

wherein:

X is O, S, or NR;
R is H, or alkyl$_{(C\leq 8)}$;
$R_1$ is H, alkyl$_{(C\leq 8)}$, or cycloalkyl$_{(C\leq 8)}$;
$R_2$ and $R_3$ are each independently H, alkyl$_{(C\leq 8)}$, $(CH_2)_n$ (n = 1-5);
$R_4$ is independently H, alkyl$_{(C\leq 8)}$, cycloalkyl$_{(C\leq 8)}$, alkenyl$_{(C\leq 8)}$, alkinyl$_{(C\leq 8)}$, allyl, propargyl, phenyl, or benzyl;
Heterocycle is independently oxazole, thiazol, imidazole, furyl, pyrrolyl, thiophenyl, pyridyl, or phenyl;
* indicate the stereoisomeric centers of the compound;

comprising the following steps:

(a) reacting a compound of formula I

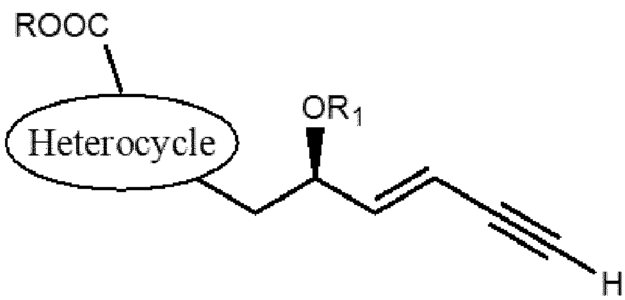

(I)

wherein R and $R_1$ is as defined above;
with a compound of formula II

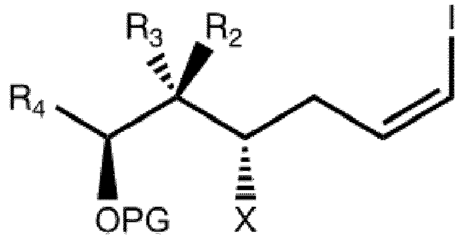

(II)

wherein X is O, S, or NR as defined above and wherein O, S, or NR comprise further a protective group PG; wherein $R_2$, $R_3$, and $R_4$ are defined as above; and
wherein PG is independently H, or alkyl$_{(C\leq8)}$; or a protective group selected independently from MOM, MEM, THP, TMS, TES, TIPS, TBS, TBDPS,

(b) reacting the product obtained in step (a) with a compound of formula I, wherein the coupling of the obtained product and compound of formula I is performed via the carboxyl ester of compound I and the X group of the obtained product;
(c) reacting the product obtained in step (b) with a compound of formula II in the same way as in step (a);
(d) cyclizing the product obtained from step (c) obtaining the product of formula III.

**3.** Method for the production of a compound of formula III according to claim 1

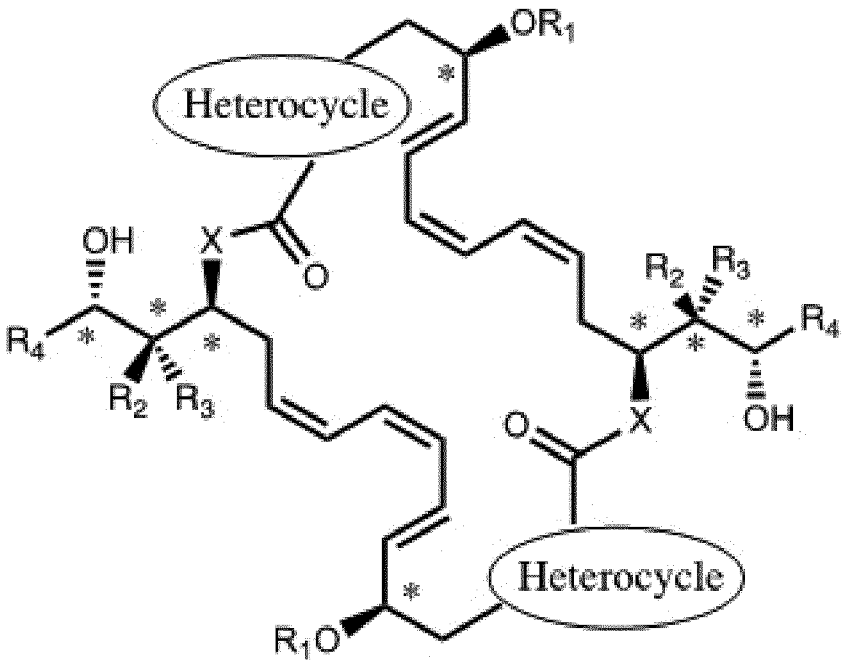

(III)

wherein:

X is O, S, or NR;
R is H, or alkyl$_{(C\leq8)}$;
$R_1$ is H, alkyl$_{(C\leq8)}$, or cycloalkyl$_{(C\leq8)}$;
$R_2$ and $R_3$ are each independently H, alkyl$_{(C\leq8)}$, $(CH_2)_n$ (n = 1-5);
$R_4$ is independently H, alkyl$_{(C\leq8)}$, cycloalkyl$_{(C\leq8)}$, alkenyl$_{(C\leq8)}$, alkinyl$_{(C\leq8)}$, allyl, propargyl, phenyl, or benzyl;
Heterocycle is independently oxazole, thiazol, imidazole, furyl, pyrrolyl, thiophenyl, pyridyl, or phenyl;
* indicate the stereoisomeric centers of the compound;

comprising the following steps:

(a) reacting a compound of formula I

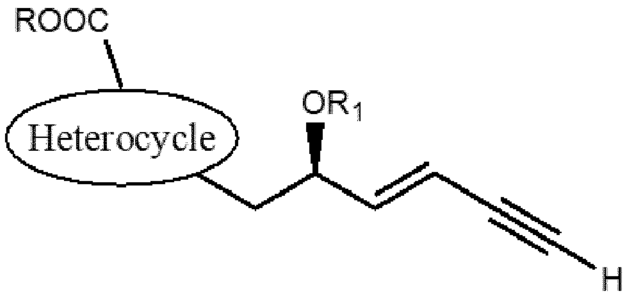

(I)

wherein R and $R_1$ is as defined above;
with a compound of formula II

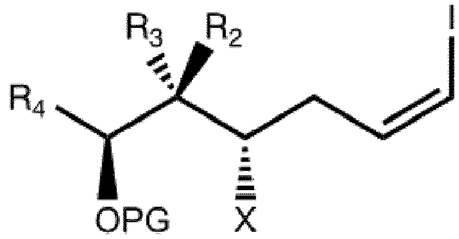

(II)

wherein X is O, S, or NR as defined above and wherein O, S, or NR comprise further a protective group PG;
wherein $R_2$, $R_3$, and $R_4$ are defined as above; and
wherein PG is independently H, or alkyl$_{(C\leq 8)}$; or a protective group selected independently from MOM, MEM, THP, TMS, TES, TIPS, TBS, TBDPS,

(b) reacting the product of step (a) in two different steps, namely step (b1) and step (b2),
wherein in step (b1) the compound obtained in step (a) is modified, in a way that PG from X is removed to obtain a compound wherein X is OH, SH, or $NH_2$,
and wherein in step (b2) the compound obtained in step (a) is modified, in a way that residue R of the carboxy group is removed and replaced with H;
(c) reacting the product obtained in step (b1) with the product obtained in step (b2);
(d) cyclizing the product obtained from step (c) obtaining the product of formula III.

**4.** Method for the production of disorazole-C1 of formula 37

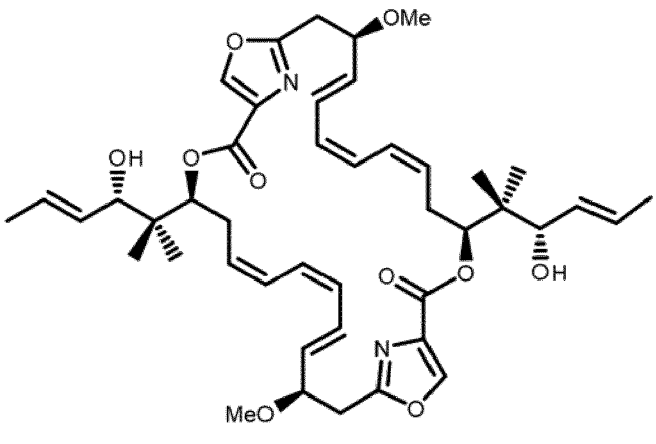

37

comprising the following steps:

(a) reacting a compound of formula 30a

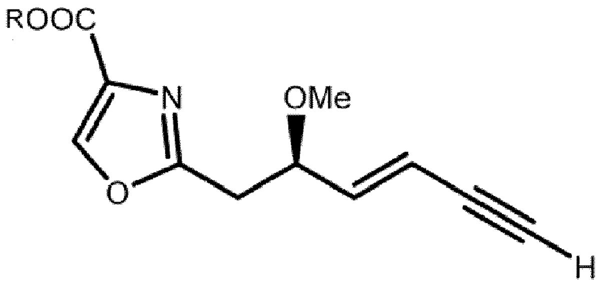

30a

wherein R is methyl or hydrogen
with a compound of formula 18

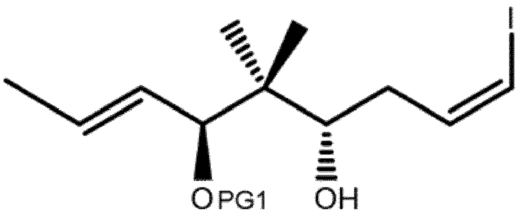

18

wherein PG1 is MOM
obtaining a compound of formula 31

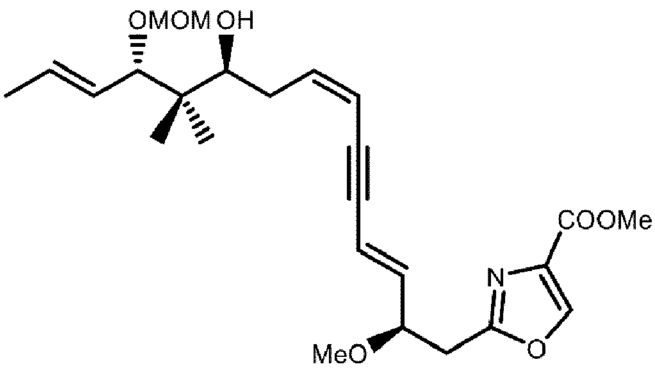

31;

(b) reacting the compound of formula 31 with a compound of formula 30a, obtaining a compound of formula 32

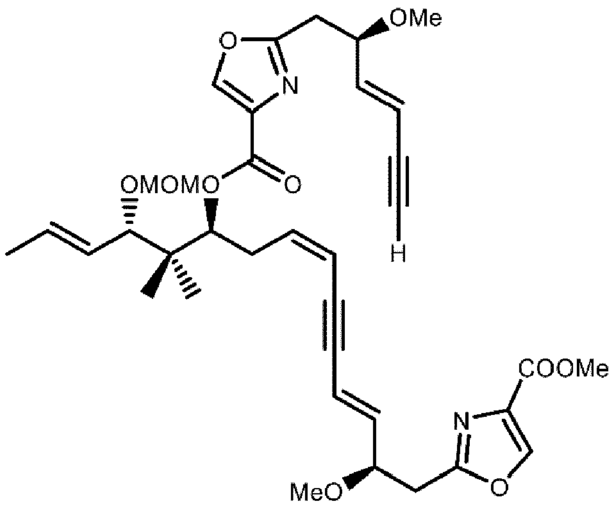

32

(c) reacting the compound of formula 32 with a compound of formula 18, obtaining a compound of formula 33

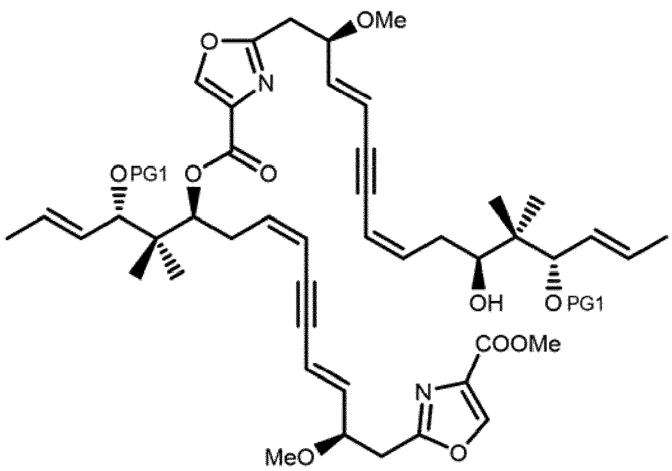

33

(d) reacting compound of formula 33 into disorazole-C1 of formula 37.

**5.** Method, according to claim 4, wherein step (a) is a coupling reaction combining compounds of formula 18 and of formula 30a, wherein R is methyl.

**6.** Method, according to claim 4 or 5, wherein step (b) is an esterification.

**7.** Method, according to at least one of preceding claims 4 to 6, wherein step (c) is a coupling reaction combining compounds of formula 31 and of formula 30a, wherein R is hydrogen.

**8.** Method, according to at least one of preceding claims 4 to 7, wherein step (d) comprises the following steps:

(d1) saponification of methyl ester from compound of formula 33 to a compound of formula 34

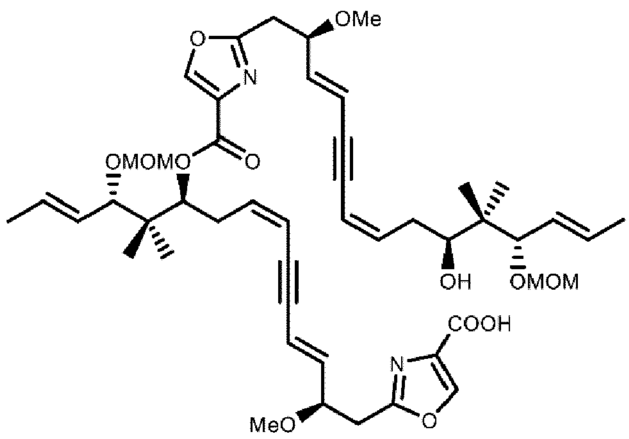

34;

(d2) macrolactonisation of open-chained compound 34 into compound of formula 35

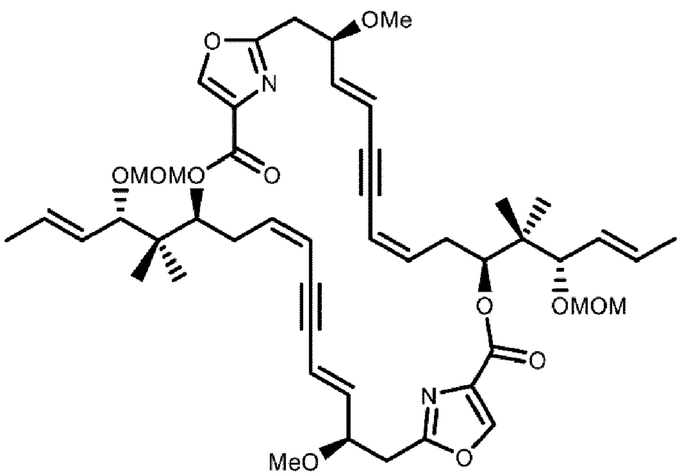

35;

(d3) reduction of the triple bonds of compound of formula 35 forming compound of formula 36

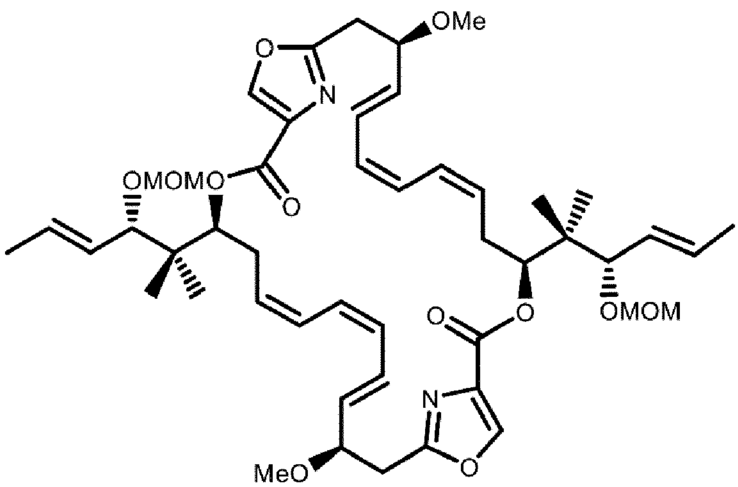

36;

and

(d4) removing the protection groups to obtain compound of formula 37.

**9.** Method for the production of disorazole-C1 of formula 37

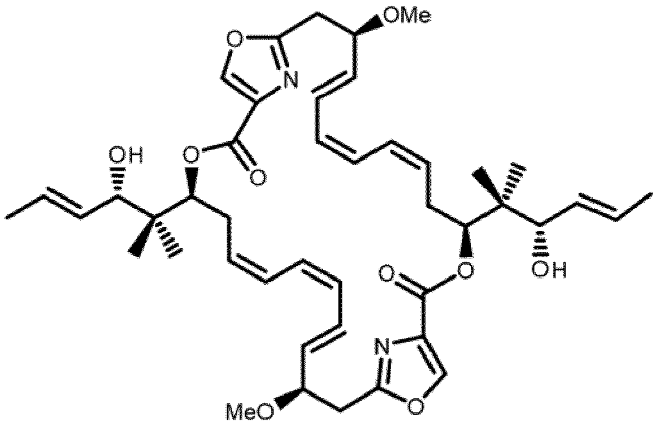

37

comprising the following steps:

(a) reacting a compound of formula 30a

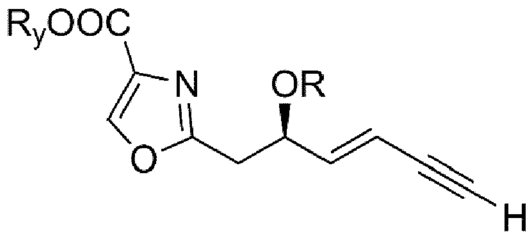

30a

wherein R and $R_y$ are methyl;
with a compound of formula 17a

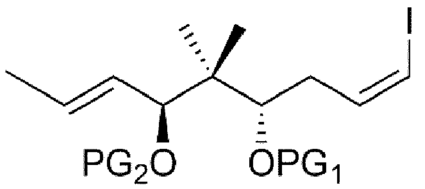

17a

wherein $PG_1$ is methyl and $PG_2$ is MOM;
obtaining a compound of formula 40

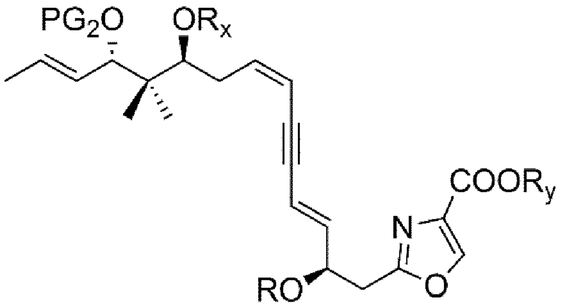

40

wherein $R_x$ is $PG_1$ and $R_y$ is methyl as defined above;

(b) reacting the compound of formula 40 partially in a step (b1) into a compound of formula 41

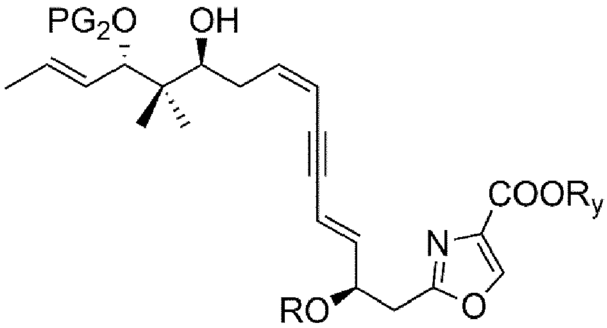

41

and partially in a step (b2) into a compound of formula 42

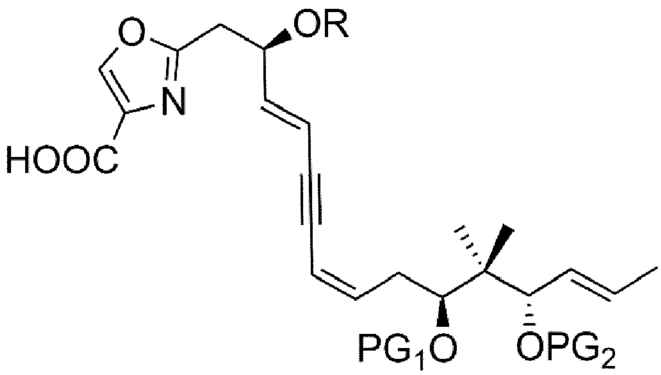

42

(c) reacting the compound of formula 41 with the compound of formula 42, obtaining a compound of formula 43

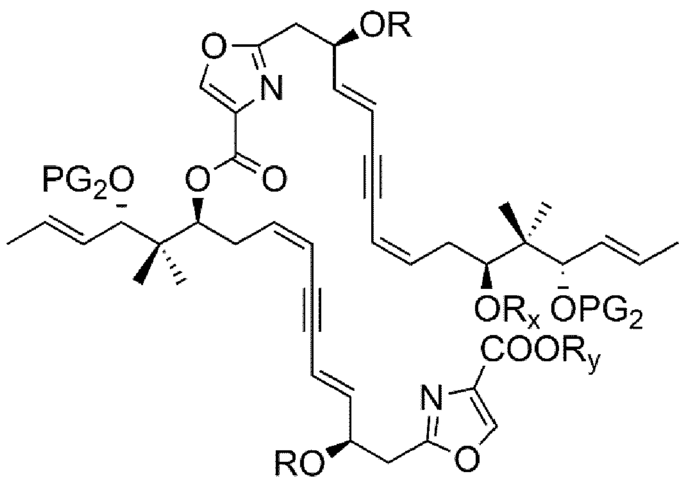

43

(d) removing $R_x$ and $R_y$ and replacing with hydrogen, obtaining a compound of formula 44

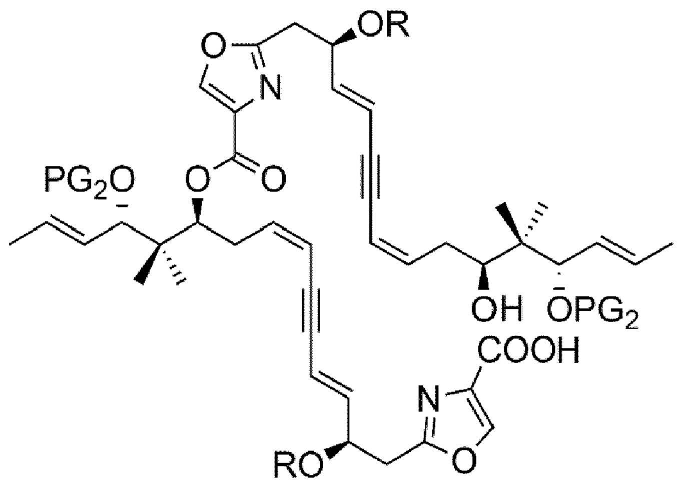

44

(e) reacting compound of formula 44 into disorazole-C1 of formula 37 as described in claim 8, wherein the starting compound is the compound of formula 44 instead of formula 34.

**10.** Intermediate product, for the production according to at least one of the claims 2 to 9, according to formula 30a

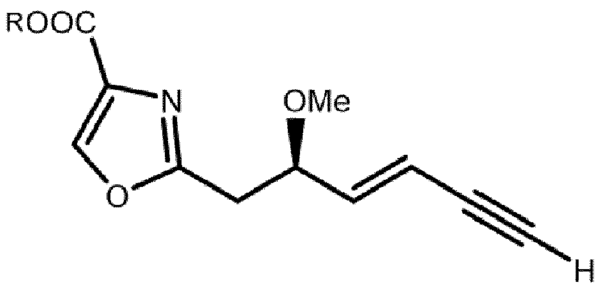

30a

wherein R is methyl or hydrogen.

**11.** Intermediate product, for the production according to at least one of the claims 2 to 9, according to formula 18

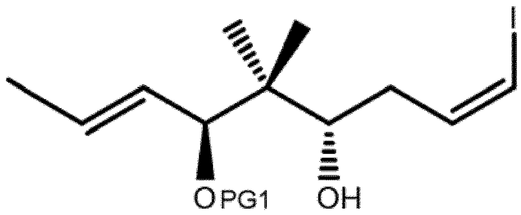

18

wherein PG1 is MOM.

**12.** Intermediate product, for the production according to at least one of the claims 2 to 9, according to formula 33

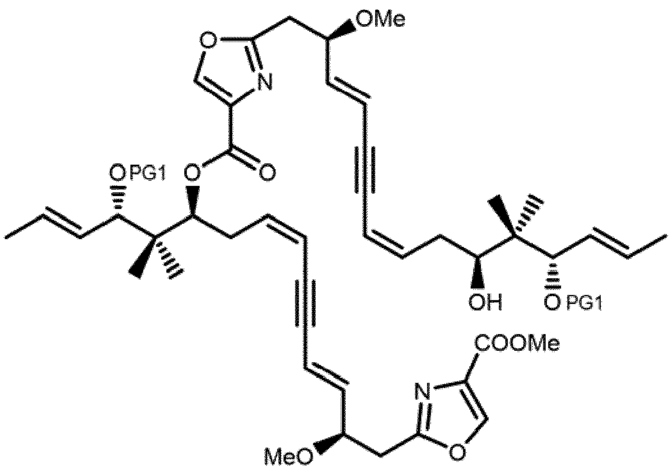

33

wherein PG1 is MOM.

**13.** Intermediate product, for the production according to at least one of the claims 2 to 9, according to formula I

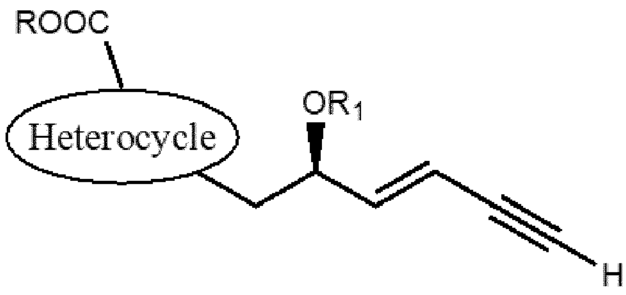

(I)

wherein R and $R_1$ is as defined above.

**14.** Intermediate product, for the production according to at least one of the claims 2 to 9, according to formula II

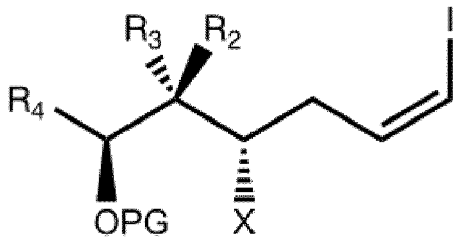

(II)

wherein X is O, S, or NR as defined above and wherein O, S, or NR comprise further a protective group PG; wherein $R_2$, $R_3$, and $R_4$ are defined as above; and
wherein PG is independently H, or alkyl$_{(C\leq8)}$; or a protective group selected independently from MOM, MEM, THP, TMS, TES, TIPS, TBS, TBDPS.

**15.** Pharmaceutical preparation for the treatment of cancer diseases, comprising at least one compound according to claim 1 and other acceptable excipients, adjuvants and /or additives.

Europäisches Patentamt
European Patent Office
Office européen des brevets

# EUROPEAN SEARCH REPORT

Application Number
EP 20 20 7769

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HARTUNG I V ET AL: "Toward the Total Synthesis of Disorazole A1 and C1: Asymmetric Synthesis of a Masked Southern Segment", ORGANIC LETTERS, AMERICAN CHEMICAL SOCIETY, US, vol. 4, no. 19, 1 January 2002 (2002-01-01), pages 3239-3242, XP002265585, ISSN: 1523-7060, DOI: 10.1021/OL026468J * the whole document * ----- | 1-15 | INV. A61P35/00 C07C21/17 C07D263/32 C07D498/22 A61K31/424 |
| X | WIPF PETER ET AL: "Total Synthesis of (-)-Disorazole C 1", JOURNAL OF THE AMERICAN CHEMICAL SOCIETY, vol. 126, no. 47, 1 December 2004 (2004-12-01), pages 15346-15347, XP055795096, US ISSN: 0002-7863, DOI: 10.1021/ja0443068 * scheme 3 * ----- | 1-15 | |
| | | | TECHNICAL FIELDS SEARCHED (IPC) |
| X | NIESS BARBARA ET AL: "Synthesis of a Tetradehydro-Disorazole C1", EUROPEAN JOURNAL OF ORGANIC CHEMISTRY, vol. 2006, no. 5, 1 March 2006 (2006-03-01), pages 1132-1143, XP055795209, DE ISSN: 1434-193X, DOI: 10.1002/ejoc.200500675 * scheme 9 * ----- -/-- | 1-15 | A61P C07C C07D |

1 The present search report has been drawn up for all claims

| Place of search | Date of completion of the search | Examiner |
|---|---|---|
| Munich | 14 April 2021 | Gavriliu, Daniela |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 20 20 7769

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HILLIER M C ET AL: "The synthesis of the monomeric moiety of disorazole C"1", TETRAHEDRON LETTERS, ELSEVIER, AMSTERDAM , NL, vol. 41, no. 16, 1 April 2000 (2000-04-01) , pages 2821-2824, XP004195679, ISSN: 0040-4039, DOI: 10.1016/S0040-4039(00)00271-9 * compound 18-page 2823 * ----- | 1-15 | |

TECHNICAL FIELDS SEARCHED (IPC)

The present search report has been drawn up for all claims

| Place of search | Date of completion of the search | Examiner |
|---|---|---|
| Munich | 14 April 2021 | Gavriliu, Daniela |

1

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018237178 A1


### Non-patent literature cited in the description


- **WIPF P. et al.** *J. Am. Chem. Soc.,* 2004, vol. 126 (47), 15346-15347